# EUROPEAN PATENT SPECIFICATION

(11) **EP 4 583 874 B1**
(45) Date of publication and mention of the grant of the patent: **07.10.2026**
(21) Application number: 22843109.4
(22) Date of filing: 21.12.2022
(51) Int. Cl.: A61K 31/47, A61K 31/498, A61P 1/16, A61P 3/04, A61P 3/06, A61P 3/08, A61P 9/10, C07D 215/00, C07D 241/00

(54) **MULTITARGET NUCLEAR RECEPTOR LIGANDS BASED ON 2-(4-(QUINOLIN-2-YLOXY)PHENOXY)PROPANOIC ACID AND 2-(4-(QUINOXALIN-2-YLOXY)PHENOXY)PROPANOIC ACID FOR THE TREATMENT OF METABOLIC AND LIVER DISEASES**
MULTITARGET-KERNREZEPTORLIGANDEN AUF BASIS VON 2-(4-(CHINOLIN-2-YLOXY)PHENOXY)PROPANSÄURE UND 2-(4-(CHINOXALIN-2-YLOXY)PHENOXY)PROPANSÄURE ZUR BEHANDLUNG VON STOFFWECHSEL- UND LEBERERKRANKUNGEN
LIGANDS DE RÉCEPTEURS NUCLÉAIRES MULTICIBLES À BASE D'ACIDE 2-(4-(QUINOLIN-2-YLOXY)PHÉNOXY)PROPANOÏQUE ET D'ACIDE 2-(4-(QUINOXALIN-2-YLOXY)PHÉNOXY)PROPANOÏQUE POUR LE TRAITEMENT DE MALADIES MÉTABOLIQUES ET HÉPATIQUES

(43) Date of publication of application: 16.07.2025
(73) Proprietor: CHARLES UNIVERSITY, FACULTY OF PHARMACY IN HRADEC KRALOVE, 500 05 Hradec Kralove (CZ)
(72) Inventor: PAVEK, Petr, 50002 Hradec Kralove (CZ); DUSEK, Jan, Brehy 53501 (CZ); DRASTIK, Martin, 50011 Hradec Kralove (CZ); STEFELA, Alzbeta, 50012 Hradec Kralove (CZ); ROH, Jaroslav, 50012 Hradec Kralove (CZ); KARABANOVICH, Galina, 50012 Hradec Kralove (CZ); MELNIKOVA, Julia, 50009 Hradec Kralove (CZ); SMUTNY, Tomas, 53009 Pardubice (CZ); MICUDA, Stanislav, 50801 Rasin (CZ); KUCERA, Otto, 50009 Hradec Kralove (CZ); LASTUVKOVA, Hana, 50012 Hradec Kralove (CZ)
(74) Representative: Jirotkova, Ivana
(86) International application number: PCT/CZ2022/050136
(87) International publication number: WO 2024/132001

(56) References cited:
- EP-A1- 1 266 888
- EP-A2- 0 023 785
- EP-A2- 0 098 751
- EP-A2- 0 206 751
- WO-A1-03/011832
- WO-A1-2010/054229
- WO-A2-2016/127019
- US-A- 5 696 119
- CARIELLO MARICA ET AL: "REVIEW Transcriptional Regulation of Metabolic Pathways via Lipid-Sensing Nuclear Receptors PPARs, FXR, and LXR in NASH", CELL MOL GASTROENTEROL HEPATOL., 2 February 2021 (2021-02-02), pages 1519 - 1539, XP093057926, Retrieved from the Internet <URL:https://www.ncbi.nlm.nih.gov/pmc/articles/PMC8042405/pdf/main.pdf> [retrieved on 20230626]

## Description

### Field of the Invention

We disclosed herein compounds of formula (I) and pharmaceutically acceptable salts thereof as common dual or multitarget modulators of several liver nuclear receptors including Farnesoid X receptor (FXR), Peroxisome proliferator-activated receptors (PPARs), and Liver X receptor α (LXRα) which are useful as therapeutic compounds, particularly in the treatment of metabolic or liver disorders including hyperlipidemia, hypercholesterolemia, dyslipidemia, steatosis, steatohepatitis, and other lipid disorders, cholestasis and non-alcoholic fatty liver disease (NAFLD), as well diseases associated with these diseases, including atherosclerosis and type 2 diabetes mellitus.

### Background Art

The invention features novel multitarget nuclear receptor ligands based on 2-(4-(quinolin-2-yloxy)phenoxy)propanoic acid and 2-(4-(quinoxalin-2-yloxy)phenoxy)propanoic acid, salts, and compositions containing them, and methods of using them

Quinoline or quinoxaline compounds are already disclosed in EP 0 206 751 A2, EP 0 098 751 A2, EP 0 023 785 A2, WO 03/011832 A1, US 5 696 119 A for use as leukotrienes antagonists, herbicides or as anti-tumor agents.

Nuclear receptors, Farnesoid X receptor (FXR), Peroxisome proliferator-activated receptors (PPARs), and Liver X receptor α (LXRα), are ligand-activated transcription factors belonging to the nuclear receptor superfamily. These nuclear receptors are expressed in the liver where they significantly contribute to the regulating genes involved in fatty acids, cholesterol, bile acids, and glucose metabolism and homeostasis.

Nuclear receptors (NRs) are also key transcriptional regulators of energy expenditure as well as inflammation, fibrosis, and cellular proliferation in the liver. Dysregulation of these processes contributes to the pathogenesis and progression of metabolic diseases, metabolic syndrome, and nonalcoholic fatty liver disease (NAFLD) or nonalcoholic steatohepatitis (NASH).

Some NRs are already pharmacologically targeted in metabolic disorders such as hyperlipidemia (fibrates as ligands for PPARα), and diabetes (glitazones as ligands for PPARγ). Obeticholic acid, a steroidal ligand of FXR, is approved for the treatment of primary biliary cholangitis and tested in clinical trials for the treatment of NASH. There are no LXRα ligands approved for therapy so far.

Nonalcoholic fatty liver disease (NAFLD) often progresses from metabolic syndrome and steatosis to nonalcoholic steatohepatitis (NASH), which has the potential to progress to advanced liver damage or hepatocellular carcinoma. However, current treatment strategies focus only on lifestyle management of some risk factors and individual components of metabolic syndrome. No other therapeutic interventions are available at present (Dibba et al., 2018).

Currently, FXR agonists, PPARα/β agonists, PPARγ agonists, pan-PPARs agonists, LXR inverse agonists, as well as e.g. ACC, SCD1, CCR2/5, DGAT1, or ASK1 enzyme or kinase inhibitors, or FGF1 analogs are tested in clinical trials to treat NAFLD/NASH (Parlati et al., 2021; Sumida et al., 2020).

It is more and more evident, that treatment of NAFLD/NASH or other metabolic diseases need multitarget or combination therapies due to the inefficiency of drugs modulating one nuclear receptor/enzyme target or due to side effects (Cariello et al., 2021).

Peroxisome proliferator-activated receptors (PPARs) are members of families of nuclear receptors. PPARα is mainly expressed in metabolically active tissues, such as the liver, kidney, heart, and muscle. PPARγ is confirmed in white and brown adipocytes, and PPARδ is expressed ubiquitously (Protein Atlas database, www.proteinatlas.org).

Fibrates, such as fenofibrate, bezafibrate, or gemfibrosil were developed as PPARα agonists for the treatment of dyslipidemia. In clinics, the administration of fibrates could reduce the concentration of triglycerides (TG) and increase the concentration of high-density lipoprotein (HDL) in plasma. Fibrates, mainly fenofibrate, are currently used in hypertriglyceridemia and are used in combination with statins to treat refractory combined dyslipidemia in patients with a high risk of cardiovascular diseases (Jones, 2009).

The genes regulated by PPARα include enzymes involved in the beta-oxidation of fatty acids (such as CPT-1, CYP4A1), the liver lipid (such as ApoA1, an important component of high-density lipoproteins (HDL) or Apo-CIII) or fatty acid transport protein (such as FABP1, 4 or LPL). Selective, high-affinity PPARα agonists increase hepatic fatty acid oxidation, which in turn decreases circulating triglycerides and free fatty acids (Pawlak et al., 2015).

Many structural modifications of fibrates have been done in the past receiving selective (Selective Peroxisome Proliferator-Activated Receptor Alpha Modulators (SPPARMα)), dual, or pan-PPAR agonists intended for dyslipidemia or NAFLD/NASH treatment (Fruchart et al., 2021; Giampietro et al., 2019a). Several clinical trials are in progress with PPAR ligands such as pemafibrate, elafibranor, saroglitazar, lobeglitazone, lanifibranor in patients with NAFLD/NASH (Todisco et al., 2022). However, no FXR agonist with a structure similar to fibrates has been published so far.

The Liver X receptors (LXRα and LXRβ) belong to the superfamily of nuclear receptors and are expressed in various tissues. LXRα (NR1H3) is expressed in the liver, kidneys, intestines, breast, and adipose tissue. LXRs stimulate hepatic lipogenesis and regulate the transport and efflux of cholesterol favoring hepatic steatosis.

Although LXR agonists have anti-atherogenic properties due to the elevated cholesterol efflux from peripheral tissues, the activation of LXR increases the expression of hepatic regulators and enzymes involved in *de novo* lipogenesis, such as sterol regulatory element-binding protein (Srebp1), fatty acids synthase (Fasn) or stearoyl-CoA desaturase (Scd1) (Ni et al., 2019).

LXRα inverse agonists (such as SR-9238 or SR-9243, PXL-665) have been shown in experimental models (obese or NAFLD/NASH-induced mice) to suppress lipogenesis to alleviate NAFLD, decrease liver weight, hepatic inflammation, down-regulate proinflammatory genes such as IL6, IL1β, TGFβ, CD68, reduced hepatocellular damage and reduced fibrosis and collagen deposition (Bunay et al., 2021; Griffett et al., 2015; Ni et al., 2019).

Importantly, LXR expression correlates with the severity of NAFLD in patients (Cariello et al., 2021). In addition, the first clinical testing with 25-hydroxycholesterol-3-sulfate, an LXRα antagonist, is ongoing in patients with NASH (Parlati et al., 2021).

Farnesoid X receptor (FXR) from the family of nuclear receptors (NRs) is the main bile acid sensor in the liver and intestine, regulating key genes involved in bile acid synthesis and enterohepatic transport and in protecting the liver from their toxic overload (Bunay et al., 2021; Cariello et al., 2021; Dibba et al., 2018).

FXR activation reduces lipotoxicity by inhibiting *de novo* lipogenesis via SREBP-1c, increases mitochondrial beta-oxidation and increase in cholesterol excretion. Several preclinical studies with obeticholic acid in NAFLD/NASH rodent models demonstrate hepatic anti-inflammatory and anti-fibrotic effects, reduced insulin resistance, hepatic steatosis/steatohepatitis, and fat deposition (Cariello et al., 2021).

Obeticholic acid, the first steroidal FXR agonist, as well as many non-steroidal FXR agonists are studied in randomized clinical trials for the treatment of NAFLD/NASH, including Px-102, Px-104, LMB763, tropifexor, cilofexor, and nidufexor. The compounds are tested in monotherapy or combination with other drugs, e.g. fircostat, an acetyl-CoA carboxylase alpha (ACC) enzyme inhibitor (Cariello et al., 2021; Dibba et al., 2018; Parlati et al., 2021).

The G protein-coupled bile acid receptor 1 (GPBAR1) also known as Takeda G protein Receptor 5 (TGR5) is a member of the G protein-coupled receptor (GPCR). This protein functions as a cell surface receptor for bile acids. The highest expression of the receptor occurs, in the ileum and colon, in cholangiocytes, and gallbladder. It has many functions including controlling body metabolism and energy thermogenesis.

EP 1 266 888 A1 and WO 2010/054229 A1 disclose quinoline compounds for use, as PPAR alpha or gamma activators and liver X receptor modulators respectively, in the treatment of diabetes and lipid disorders.

However, no combined FXR agonist/LXRα antagonist or PPARα agonist/LXRα antagonist have been developed or tested for the treatment of metabolic diseases or NAFLD/NASH.

### Disclosure of the Invention

This invention discloses compounds of formula I, some of them are novel, and compositions comprising these compounds, as first-in-class class agonists or partial agonists or combined or dual agonists of FXR or PPARs receptors that are at the same time antagonists/inverse agonists of LXRα. In addition, some novel compounds of formula I, and compositions comprising these compounds, are agonists or partial agonists of GPBAR1 (TGR5) receptor. Thus, derivatives of formula I could be used for therapy of metabolic diseases or can be used for the further modification to tune multitarget synergistic activities against FXR, LXRα, PPARs and TGR5 receptors.

The present invention relates to a compound of general Formula I: 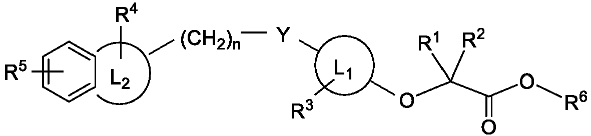 wherein
L₁ is either aryl or 6-membered heteroaryl, which optionally further includes a heteroatom selected from N, O, and S;
L₂ is aryl, 5 or 6-membered heteroaryl containing 1 or 2 heteroatoms selected from N, O and S; or C5-C6 cycloalkyl;
R¹ is H or independently selected from the group consisting of C1-C2 alkyl, C1-C2 haloalkyl, wherein each of these substituents may optionally be further substituted by one or more substituents selected from the group consisting of halogen, pseudohalogen, hydroxy, amino, or alkyloxy moieties.
R² is H or independently selected from the group consisting of C1-C4 alkyl, C1-C4 haloalkyl, wherein each of these substituents may optionally be further substituted by one or more substituents selected from the group consisting of halogen, pseudohalogen, hydroxy, amino, or alkyloxy moieties.
R³ is H or independently selected from the group consisting of halogen, pseudohalogen, C1-C2 alkyl, C1-C2 haloalkyl, hydroxy or amino moieties.
R⁴ is H or independently selected from the group consisting of halogen, pseudohalogen, C1-C2 alkyl, C1-C2 haloalkyl, methoxy C1-C4 alkyl, thio C1-C4 alkyl, halogen, pseudohalogen, wherein each of these substituents may optionally be further substituted by halogen, pseudohalogen, hydroxy, or amino moiety.
R⁵ is H or independently selected from the one or two groups consisting of C1-C4 alkyl, C1-C4 haloalkyl, methoxy C1-C4 alkyl, thio C1-C4 alkyl, halogen, pseudohalogen, wherein each of these substituents may optionally be further substituted by halogen, pseudohalogen, hydroxy, nitro, or amino moiety.
R⁶ is H or independently selected from C1-C6 alkyl, C1-C4 haloalkyl, methoxy C1-C4 alkyl, thio C1-C4 alkyl, wherein each of these substituents may optionally be further substituted by halogen, pseudohalogen, hydroxy, nitro, or amino moiety.
Y is O, N, or S
n is 0, 1 or 2

Compounds of general formula I is intended to include pharmaceutically acceptable derivatives and solvates.

Compounds of general formula I can be in the form of pharmaceutically acceptable salts or esters thereof, such as methyl, ethyl, isopropyl or propyl ester.

### Definitions:

- the term "aryl" refers to a group having 6 to 12 atoms and containing at least one aromatic ring. Aryl may contain one aromatic ring or two aromatic rings, preferably fused. Aryl is preferably selected from phenyl;
- the term "heteroaryl" refers to an aromatic cyclic group having 5 or 6 atoms and containing 1 to 4 heteroatoms (preferably 1 to 3 heteroatoms) selected from O, S, N, the remaining atoms being carbon atoms.
- the term "alkyl" refers to a linear or branched saturated radical, typically containing one to six carbons. Alkyl is preferably selected from methyl, ethyl, n-propyl, isopropyl, n-butyl, sec-butyl, tert-butyl, amyl, t-amyl, n-pentyl, n-hexyl;
- the term "haloalkyl" refers to alkyl as defined herein above, substituted by at least one halogen. Haloalkyl is preferably selected from perfluoro(C1-C6)alkyl, fluoromethyl, difluoromethyl, trifluoromethyl, fluoroethyl, difluoroethyl, trifluoroethyl, mono- or polyhalo substituted propyl, butyl, pentyl, hexyl;
- the term "cycloalkyl" refers to a saturated cyclic radical, typically containing three to six carbons. Cycloalkyl is preferably selected from cyclopentyl or cyclohexyl;
- the term "amino" represents an -NH₂ group;
- the term "nitro" refers to -NO₂;
- the term "hydroxy" refers to -OH;
- the term "halogen" or "halide" includes -F, -Cl, -Br, and -I;
- the term "pseudohalogen" includes cyanide, cyanate, thiocyanate, selenocyanate, and azide.

Preferably, at least one R¹ and/or R⁵ is present, wherein the said at least one R¹ and/or R⁵ is independently selected from methyl, ethyl, isopropyl, halogen, pseudohalogen and alkoxy. Further R¹ and R⁵ substituents can be present, selected from the group defined above.

Preferably, at least one heteroatom is present in L₂, wherein the said heteroatom is N, S, or O.

Preferred embodiments of the invention are the compounds of general formula (I), selected from the group comprising:
(*R*)-2-(4-((7-Chloroquinolin-2-yl)oxy)phenoxy)propanoic acid
(*R*)-2-(4-((6-Chloroquinolin-2-yl)oxy)phenoxy)propanoic acid
(*R*)-2-(4-((5-Chloroquinolin-2-yl)oxy)phenoxy)propanoic acid
(*R*)-2-(4-((5,7-Dichloroquinolin-2-yl)oxy)phenoxy)propanoic acid
(*R*)-2-(4-((7-Bromoquinolin-2-yl)oxy)phenoxy)propanoic acid
(*R*)-2-(4-((5-Bromoquinolin-2-yl)oxy)phenoxy)propanoic acid
(*R*)-2-(4-((7-Fluoroquinolin-2-yl)oxy)phenoxy)propanoic acid
(*R*)-2-(4-((7-(Trifluoromethyl)quinolin-2-yl)oxy)phenoxy)propanoic acid
(*R*)-2-(4-((5-(Trifluoromethyl)quinolin-2-yl)oxy)phenoxy)propanoic acid
(*R*)-2-(4-((7-Ethylquinolin-2-yl)oxy)phenoxy)propanoic acid
(*R*)-2-(4-((5,7-Dimethylquinolin-2-yl)oxy)phenoxy)propanoic acid
(*R*)-2-(4-((7-Methylquinolin-2-yl)oxy)phenoxy)propanoic acid
(*R*)-2-(4-((5-Methylquinolin-2-yl)oxy)phenoxy)propanoic acid
(*R*)-2-(4-((7-Iodoquinolin-2-yl)oxy)phenoxy)propanoic acid
(*R*)-2-(4-((5-Iodoquinolin-2-yl)oxy)phenoxy)propanoic acid
Ethyl 2-(4-((7-chloroquinolin-2-yl)oxy)phenoxy)-2-methylpropanoate
Ethyl 2-(4-((7-bromoquinolin-2-yl)oxy)phenoxy)-2-methylpropanoate
2-(4-((7-Chloroquinolin-2-yl)oxy)phenoxy)-2-methylpropanoic acid
2-(4-((7-Bromoquinolin-2-yl)oxy)phenoxy)-2-methylpropanoic acid
(*R*)-2-(4-(Quinoxalin-2-yl)oxy)phenoxy)propanoic acid
(*R*)-2-(4-((7-Chloroquinoxalin-2-yl)oxy)phenoxy)propanoic acid
(*R*)-2-(4-((6-Chloroquinoxalin-2-yl)oxy)phenoxy)propanoic acid
(*R*)-2-(4-((7-Bromoquinoxalin-2-yl)oxy)phenoxy)propanoic acid
(*R*)-2-(4-((7-Methylquinoxalin-2-yl)oxy)phenoxy)propanoic acid
(*R*)-2-(4-((6,7-Dimethoxyquinoxalin-2-yl)oxy)phenoxy)propanoic acid
(*R*)-2-(4-((7-Iodoquinoxalin-2-yl)oxy)phenoxy)propanoic acid
(*R*)-2-(4-((7-Methoxyquinoxalin-2-yl)oxy)phenoxy)propanoic acid
2-(4-(Quinoxalin-2-yloxy)phenoxy)acetic acid
2-(4-((7-Iodoquinoxalin-2-yl)oxy)phenoxy)acetic acid
Ethyl (R)-2-(4-((5-chloroquinolin-2-yl)oxy)phenoxy)propanoate
Ethyl 2-(4-((7-chloroquinoxalin-2-yl)amino)phenoxy)propanoate
Ethyl 2-(4-((6-chloroquinoxalin-2-yl)amino)phenoxy)propanoate
2-(4-((7-Chloroquinoxalin-2-yl)amino)phenoxy)propanoic acid
2-(4-((6-Chloroquinoxalin-2-yl)amino)phenoxy)propanoic acid
2-(4-(((5,7-Dichloroquinolin-2-yl)methyl)amino)phenoxy)propanoic acid
Ethyl 2-(4-(((5-chloroquinolin-2-yl)methyl)amino)phenoxy)propanoate

### Detailed description of the invention

- One aspect of the present invention is a compound of general formula I for use in prevention or treatment of a condition which is mediated by the action, or by loss of action, of FXR, PPARα, PPARγ, LXRα, or TGR5 receptors or their endogenous ligands, preferably in the liver or in other organs with sufficient expression such as in the adipose tissue, intestine, brain, thyroid, heart, ovary or prostate. More specifically, said compounds have combined direct agonistic, inverse agonistic, partial agonistic or antagonistic activity for human FXR, PPARα, γ, LXRα, or TGR5 receptors and interact with them to stimulate or repress their transcriptional activities.
- The present invention is a compound of the general formula I that regulates, normalize, or modify the expression of key genes encoding enzymes and regulators of lipid, glucose, cholesterol and amino acid metabolism and energy homeostasis.
- The present invention is directed to a compound of the general formula I for use in prevention or treatment of metabolic disorders such as defects in lipid, triglyceride, cholesterol, bile acids and glucose metabolism, including dyslipidemia, hypertriglyceridemia, liver steatosis, inflammatory liver diseases connected with metabolic diseases such as steatosis, steatohepatitis, NAFLD or NASH, impaired glucose tolerance and insulin sensitivity, metabolic syndrome, type II diabetes, gestational diabetes, obesity, obesity related atherosclerosis, cholestasis, inhered lipid metabolic disorders, and in protection against liver stress.

The medical uses of compounds of general formula I are relevant in particular for human subjects.

The compounds of formula I may be prepared in a crystalline or an amorphous form and may be optionally hydrated or solvated.

Pharmaceutically acceptable salts may be salts derived from non-toxic inorganic or organic acids or non-toxic inorganic or organic bases to render higher solubility in water or oil. A person skilled in the art will be able to determine which are pharmaceutically acceptable salts.

The compounds of general formula I according to this invention can also be used in the form of esters (e.g. methyl or ethyl esters), in the form of a precursor (prodrug) or in another suitable form which releases the active substance *in vivo.*

The compounds of formula I may be administered by any convenient method, for example by oral, parenteral (e.g. intravenous), buccal, sublingual, nasal, rectal, subcutaneous or transdermal administration and the pharmaceutical compositions adapted accordingly. The pharmaceutical composition can be for use in the treatment of any of the conditions described herein via these application routes.
- For use in human medicine, the compounds of the present invention are usually administered as a standard pharmaceutical composition. The present invention therefore provides a pharmaceutical composition comprising a compound of formula (I) or a pharmaceutically (i.e physiologically) acceptable salt or ester thereof and a pharmaceutically acceptable carrier or excipient to form appropriate formulation, microformulation or nanoformulation.

A composition in the form of a tablet can be prepared using any suitable pharmaceutical carrier(s) routinely used for preparing solid pharmaceutical formulations. Examples of such carriers include lactulose, lactose, sucrose, cellulose magnesium stearate or starch.

A composition in the form of a capsule can be prepared using routine encapsulation procedures. Alternatively, a dispersion or suspension can be prepared using any suitable pharmaceutical carrier(s) and then filled into a soft gelatin capsule.
- In the framework of the present invention, it was found that compounds of the invention may concurrently activate PPARα and γ receptors, FXR or TGR5 receptor, but inhibit or be inverse agonists of LXRα. The combined effects of these compounds of the invention are synergistic (e.g. FXR activation and LXRα inverse agonism) to promote antisteatotic effects via inhibition of lipidogenesis but activation fatty acid oxidation.

### Brief Description of the Drawings

To further clarify the invention and its features, a more detailed description of the invention will be rendered by reference to specific embodiments thereof which are illustrated in the appended drawings in which:

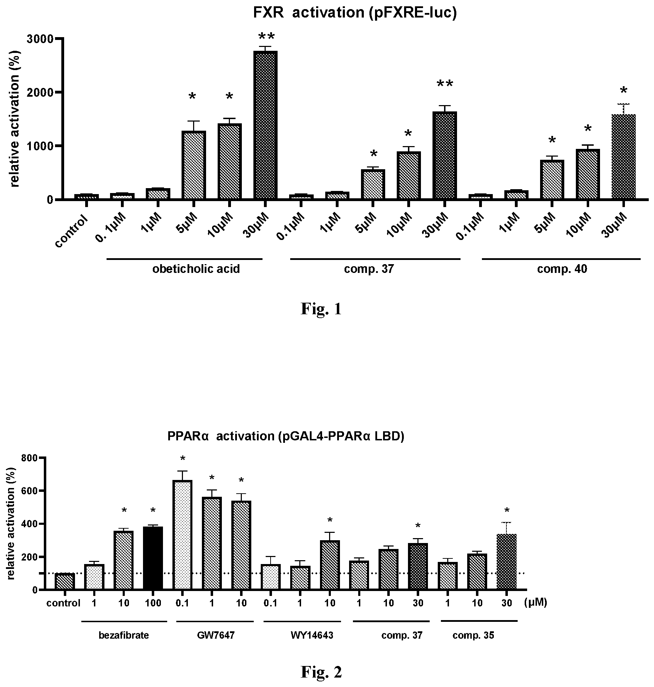

Fig. 1 shows activities for selected compounds **37** and **40** of the invention in a FXR responsive reporter gene luciferase assay performed in HepG2 cells. Data show relative activities of compounds (% of control) of the invention to activate human FXR with respect to increasing concentration tested. OCA stands for obeticholic acid, which is a high-affinity model FXR ligand. Cells have been treated with tested compounds for 24 hours after cotransfection with pFXRE-luc luciferase reporter and human FXR expression constructs. * or ** -statistically significant (p<0.05 or 0.001, respectively)
Fig. 2 shows activities for selected **compounds 35** and **37** of the invention in a PPARα responsive reporter gene luciferase assay performed in HepG2 cells. Data show relative activities of compounds (% of control) of the invention to activate human PPARα with respect to increasing concentration tested. Bezafibrate, GW7647 and WY14643 are known PPARα ligands. Cells have been treated with tested compounds for 24 hours after cotransfection with pGAL4-PPARα expression vector and a pGL5-UAS 9x luc luciferase reporter constructs. * - statistically significant (p<0.05)

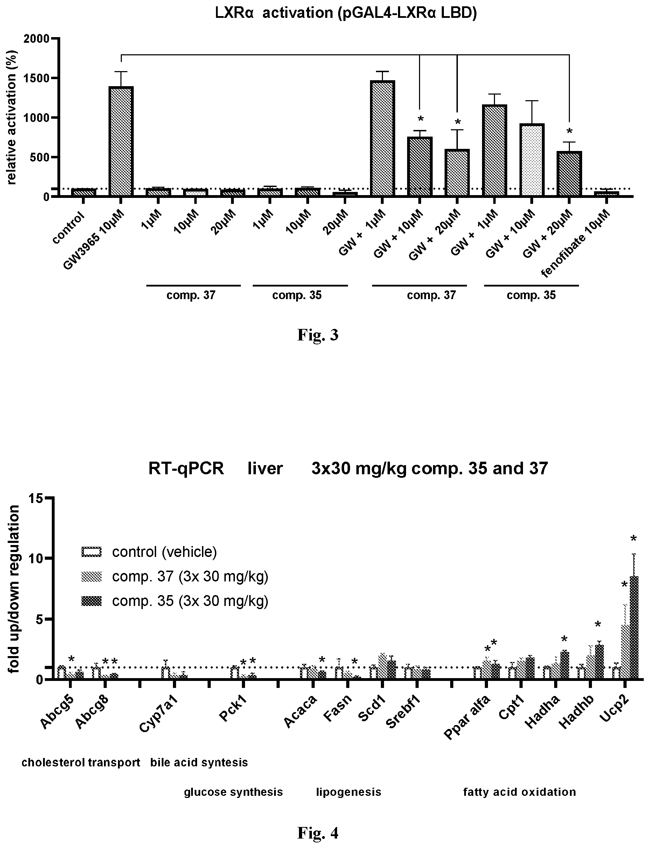

Fig. 3 shows the activities for selected compounds of EXAMPLES **35** and **37** of the invention in an LXRα responsive reporter gene luciferase assay performed in HepG2 cells. Data show relative activities of compounds (% of control) of the invention to inhibit/reverse human LXRα activation by GW3965 agonist (GW, 10 µM) with respect to increasing concentration of tested compounds. GW3965 is known high-affinity LXRα ligand. Cells have been treated with tested compounds alone or in combination with GW3965 for 24 hours after cotransfection with pGAL4-LXRα expression vector and a pGL5-UAS 9x-luc luciferase reporter construct. * -statistically significant (p<0.05) inhibition of the GW3965 effect.

Table 1 shows how selected compounds of the invention activate PPARα, PPARγ, FXR or TGR5 receptors, but inhibit LXRα activation at the 10 µM concentration.

| **compound** | **PPARα** | **PPARγ** | **FXR** | **TGR5** | **LXRα antagonism** |
|---|---|---|---|---|---|
| **35** | 117% | 4% | 4% | 5% | 63% |
| **36** | -5% | 23% | 19% | nd | 36% |
| **37** | 8% | 1% | 77% | 10% | 39% |
| **38** | 0% | 1% | 14% | 2% | 41% |
| **39** | 87% | 3% | 4% | 6% | 43% |
| **40** | 0% | 1% | 89% | 16% | 48% |
| **41** | 1% | 7% | 1% | nd | nd |
| **43** | -5% | -1% | 1% | nd | 48% |
| **44** | 0% | 4% | 15% | nd | 7% |
| **45** | 0% | 17% | 7% | nd | 43% |
| **50** | 87% | nd | 0% | nd | nd |
| **55** | 1% | -2% | 0% | -15% | nd |
| **62** | 0% | 2% | 7% | 43% | nd |
| **63** | 0% | 2% | -3% | 4% | nd |
| **64** | nd | nd | 1% | nd | 17% |
| **65** | 69% | 2% | -2% | -3% | nd |
| **66** | 1% | 2% | -3% | nd | nd |
| **68** | 49% | 9% | -3% | 11% | nd |
| **69** | 46% | 1% | 3% | nd | nd |
| **70** | 10% | 0% | -2% | nd | nd |
| **73** | 59% | 1% | 6% | 20% | nd |
| **76** | nd | nd | 25% | nd | 51% |
| **80** | 0% | nd | -1% | nd | 66% |
| **84** | 0% | 1% | -2% | nd | 5% |
| **85** | 1% | -6% | -1% | 30% | nd |
| | 100% | 100% | 100% | 100% | |
| | fenofibrate 10 µM | rosiglitazone 10 µM | OCA 10 µM | LCA 10 µM | |
| **TFCA 10 µM** | | | | | 47% |

Activities of tested compounds are presented as relative activation to a prototype PPARα fenofibrate), PPARγ (rosiglitazone), FXR (obeticholic acid, OCA), TGR5 (lithocholic acid, LCA) ligands. Inverse agonistic/antagonistic activities of tested compounds to LXRα receptor activated by the LXRα agonist GW3965(10 µM). More potent LXR antagonists/inverse agonists produces more extensive inhibition (e.g. over 50%); nd.- not determined, TFCA is a known LXRα inverse agonist/antagonist. Negative value indicates inverse agonism.

Fig. 4 demonstrates that the compounds of EXAMPLES **35** and **37** up-regulate some key protein involved in fatty acid (beta) oxidation, down-regulate Fasn and Pck1 genes expression involved fatty acid synthesis and gluconeogenesis, down-regulates Cyp7a1, a critical enzyme in bile acid synthesis from cholesterol, and down-regulates cholesterol efflux transporters Abcg5/8 in the mouse liver.

Compounds **35** and **37** have been applied three times after 24 h (3x 30 mg/kg) via *p.o.* gavage to wild-type C57BL/6J mice. After 24 h from the last application, mice were sacrificed, and live samples collected on dry ice for total RNA isolation. RT-qPCR experiments have been performed with TaqMan probes and expression has been quantified using a reference gene (beta-actin and Gapdh) and delta-delta method. Data represents fold up or downregulation in mRNA expression for tested genes related to the expression in vehicle-treated control animals. * - statistically significant (p<0.05) up- or down- regulation in animals treated with tested compounds (*n*=4).

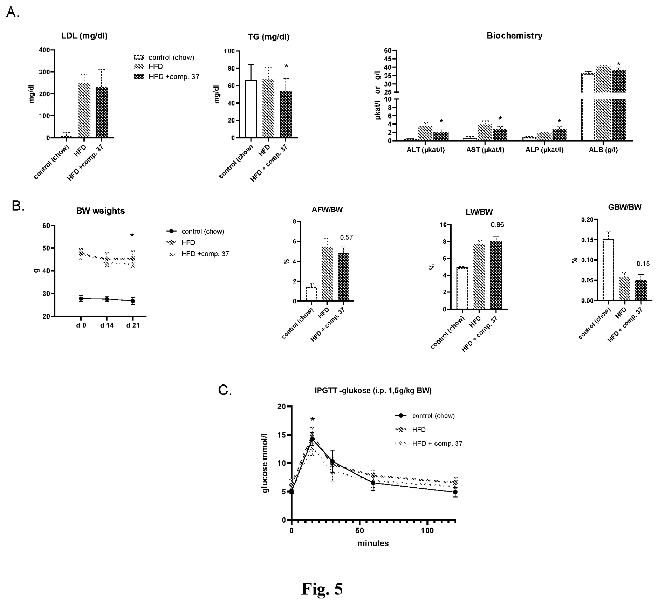

Fig. 5 demonstrates that (A) the compound of EXAMPLES **37** significantly decreases plasma triglyceride (TG) levels and improves ALT and AST plasma levels but increases ALP plasma levels in wild-type C57BL/6J mice fed with high fat diet (HFD, 20% fats) with fructose in water for 21 weeks and then were applied daily with *p.o*. gavage of **compound 37** (10 mg/kg) for further 3 weeks together with the HFD diet. Control group received a chow diet. In the same experiments, compound of EXAMPLE **37**significantly decreased body weight (BW) in HFD-treated mice after three weeks of treatment (B). The compound also decreased the abdominal fat weight (AFW) and gallbladder weight (GBW), but the effects were not statistically significant. (C) The compound of EXAMPLE **37** significantly decreased glucose plasma levels in *Intraperitoneal* Glucose Tolerance Test (IPGTT, 1.5 g/kg BW) in the study after 15 minutes, in other interval the decrease was not statistically significant.. * - statistically significant (p<0.05) vs HFD group (n ≥ 10).

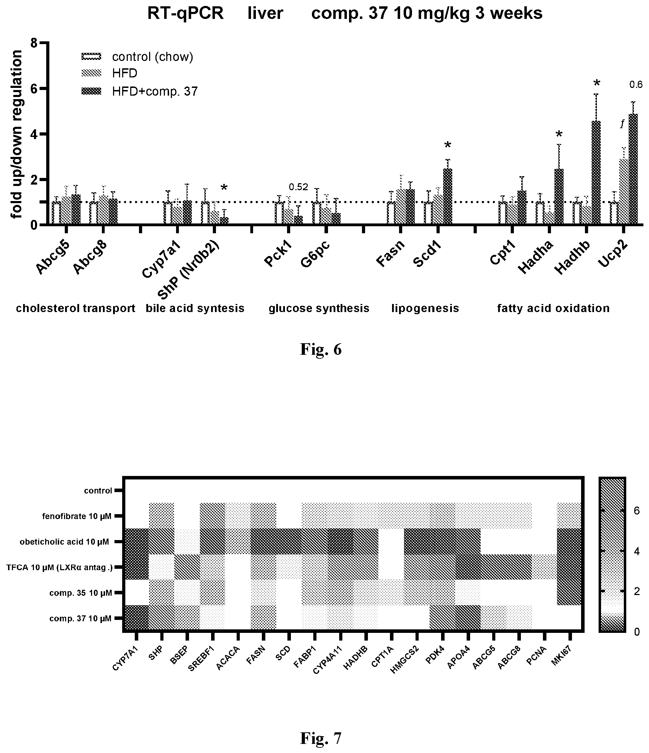

Fig. 6 demonstrates that the compounds of EXAMPLE **37** up-regulates key genes involved in fatty acid (beta) oxidation (Ucp2, Hadha and Hadha) in mouse livers.

The compound **37** has been applied daily (10 mg/kg) via *p.o.* gavage for three weeks to wild-type C57BL/6J mice fed with high-fat diet (HFD) for 24 weeks. After 24 h from the last application, mice were sacrificed, and live samples collected on dry ice for total RNA isolation. RT-qPCR experiments have been performed with TaqMan probes and expression has been quantified using a reference gene (Gapdh) and delta-delta method. Data represents fold up or downregulation in mRNA expression for tested genes related to the expression in vehicle-treated control animals. * - denotes statistically significant (p<0.05) up-regulation in animals treated with the compound of EXAMPLE **37** in comparison to animals fed with HFD only (n ≥ 10 in each groups). *^{f}* - statistically significant effect of HFD diet on a gene expression.

Fig. 7 shows the expression of genes involved in lipid, cholesterol, bile acid and glucose metabolism or in hepatocyte proliferation in 3D primary human hepatocyte spheroids (JEL donor) after treatment with compounds of EXAMPLES **35** and **37,** obeticholic acid, fenofibrate or TFCA, an LXRα/β inverse agonist/antagonist, for 72 hours. After the interval, spheroid have been subjected to RNA isolation and RT-qPCR was used to analyse mRNA expression using specific TaqMan probes. Expression has been quantified using a reference gene (TBP) and delta-delta method. Data represents fold up or downregulation in mRNA expression for tested genes (column) related to the expression in vehicle-treated controls (equal to 1, white color).

### Examples

### Abbreviations

- Ac: Acetyl
- DMSO: Dimethylsulfoxide
- Et: Ethyl
- EtOAc: Ethyl acetate
- eq.: Eqvivalent
- Fasn: mouse fatty acid synthase
- FXR: Farnesoid X receptor
- GAPDH: Glyceraldehyde 3-phosphate dehydrogenase gene
- HepG2: Human hepatocyte derived cell line
- IPGTT: Glucose tolerance test after intraperitoneal application of glucose solution
- IR: Infrared spectroscopy
- LBD: Ligand binding domain
- LXRα: Liver X receptor alpha
- Me: Methyl
- NMR: Nuclear magnetic resonance
- PPAR: Peroxisome proliferator-activated receptor
- PXR: Pregnane X receptor
- RP: Reverse-phase
- rt: Room temperature
- RT-qPCR: Reverse transcription quantitative Polymerase Chain Reaction
- Scd1: Stearoyl-Coenzyme A desaturase 1
- *t*-Bu: Tertial butyl
- TEA: Triethylamine
- THF: Tetrahydrofuran
- TLC: Thin-layer chromatography
- TMS: Trimethylsilyl
- UPLC: Ultra-high-pressure liquid chromatography
- mp: melting point

Examples 50, 51, 69, 73, 74 and 75, describing compounds not falling within formula (I), are given to illustrate preparation of similar compounds.

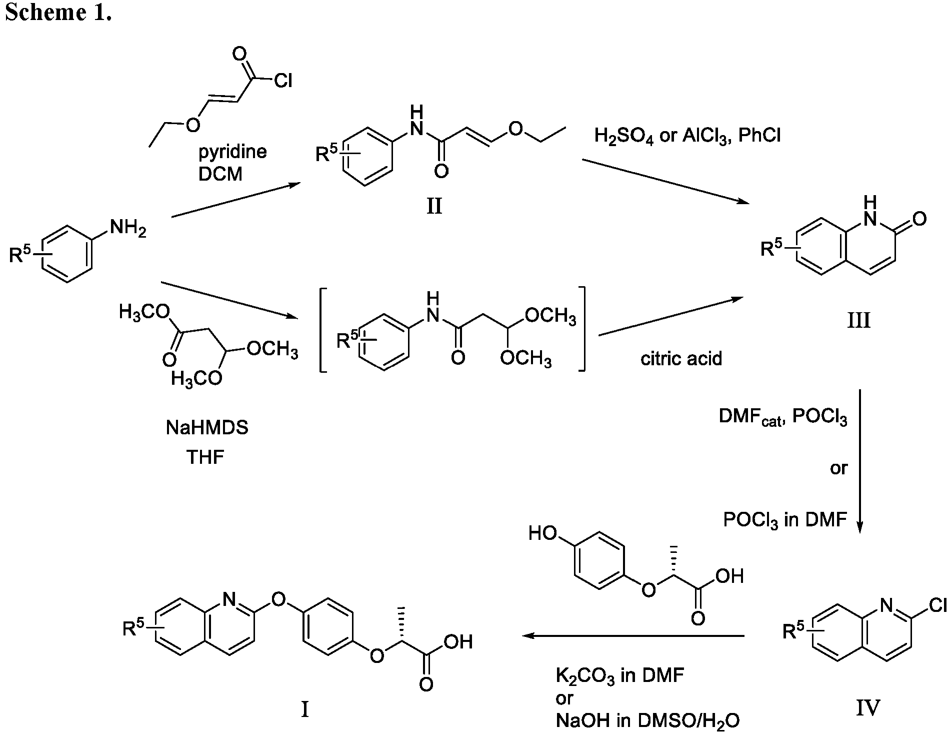

### General Procedure 1: Synthesis of acrylamides of general formula II

A solution of 3-ethoxyacryloyl chloride (1.48 g/1.37 mL, 11 mmol) in dry CH₂Cl₂ (10 mL) was added dropwise to a solution of substituted aniline (7.8 mmol) and pyridine (1.27 g/1.3 mL, 16 mmol) in dry CH₂Cl₂ (10 mL) under inert atmosphere and cooling in ice bath. The reaction mixture was stirred at rt for 24 hours and then washed with 1 % HCl (1 × 50 mL), water (1 × 30 mL) and brine (1 × 30 mL). The organic layer was dried over anhydrous sodium sulfate and concentrated under reduced pressure. The product was purified by column chromatography (mobile phase: hexane/EtOAc, 7:1).

### EXAMPLE 1

### N-(3-Chlorophenyl)-3-ethoxyacrylamide (IIa, intermediate compound)

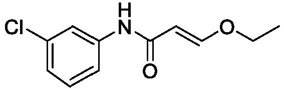

Title compound was prepared according to General Procedure 1 (Scheme 1). Yield: 82 % as a light yellow solid; mp 86-88 °C (lit (Effenberger, 1969) mp 98 °C). ¹H NMR (600 MHz, DMSO) δ 9.86 (s, 1H), 7.81 (t, *J* = 2.1 Hz, 1H), 7.47 (d, *J* = 12.3 Hz, 1H), 7.39-7.37 (m, 1H), 7.26 (t, *J =* 8.1 Hz, 1H), 7.02-7.01 (m, 1H), 5.46 (d, *J* = 12.3 Hz, 1H), 3.92 (q, *J* = 7.0 Hz, 2H), 1.23 (t, *J =* 7.0 Hz, 3H). ¹³C NMR (151 MHz, DMSO) δ 165.5, 160.6, 141.6, 133.6, 130.9, 122.9, 118.9, 117.8, 100.1, 67.2, 15.0. Elem. Anal. Calcd. for C₁₁H₁₂ClNO₂: C, 58.55; H, 5.36; N, 6.21. Found: C, 58.20; H 5.37, N 5.95.

### EXAMPLE 2

### N-(4-Chlorophenyl)-3-ethoxyacrylamide (IIb, intermediate compound)

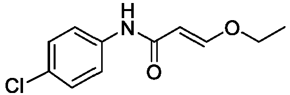

Title compound was prepared according to General Procedure I (Scheme 1). Yield: 74 % as a yellow solid; mp 168-170 °C (lit mp (Effenberger, 1969) 180 °C). ¹H NMR (600 MHz, DMSO) δ 9.81 (s, 1H), 7.60-7.58 (m, 2H), 7.45 (d, *J* = 12.3 Hz, 1H), 7.30-7.28 (m, 2H), 5.46 (d, *J* = 12.38 Hz, 1H), 3.91 (q, *J =* 7.0 Hz, 2H), 1.23 (t, *J =* 7.0 Hz, 3H). ¹³C NMR (126 MHz, DMSO) δ 165.26, 160.36, 139.15, 129.10, 126.75, 120.99, 100.16, 67.13, 15.01. Elem. Anal. Calcd. for C₁₁H₁₂ClNO₂: C, 58.55; H, 5.36; N, 6.21. Found: C,58.16; H, 5.26; N, 6.19.

### EXAMPLE 3

### N-(3,5-Dichlorophenyl)-3-ethoxyacrylamide (IIc, intermediate compound)

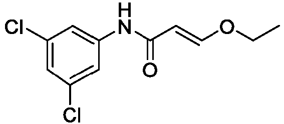

Title compound was prepared according to General Procedure I (Scheme 1). Yield: 39 % as a light yellow solid; mp 141-142 °C. ¹H NMR (500 MHz, DMSO) δ 10.13 (s, 1H), 7.68 (d, J = 1.9 Hz, 2H), 7.53 (d, J = 12.3 Hz, 1H), 7.21 (t, J = 1.9 Hz, 1H), 5.47 (d, J = 12.3 Hz, 1H), 3.96 (q, J = 7.0 Hz, 2H), 1.27 (t, J = 7.0 Hz, 3H). ¹³C NMR (126 MHz, DMSO) δ 165.63, 161.10, 142.47, 134.47, 122.27, 117.39, 99.68, 67.28, 14.94. Elem. Anal. Calcd. For C₁₁H₁₁Cl₂NO₂: C, 50.79; H, 4.26; N, 5.38. Found: C, 50.91; H, 4.33; N, 5.18.

### EXAMPLE 4

### N-(3-bromophenyl)-3-ethoxyacrylamide (IId, intermediate compound)

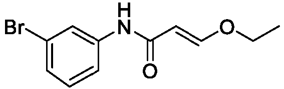

Title compound was prepared according to General Procedure I (Scheme 1). Yield: 76 % as a light yellow solid; mp 94-95 °C (lit mp (Alabaster et al., 1988) 98-100 °C). ¹H NMR (600 MHz, DMSO) δ 9.84 (s, 1H), 7.95 (t, *J* = 2.0 Hz, 1H), 7.46 (d, *J =* 12.3 Hz, 1H), 7.44-7.42 (m, 1H), 7.20 (t, *J =* 8.0 Hz, 1H), 7.16-7.14 (m, 1H), 5.45 (d, *J =* 12.3 Hz, 1H), 3.92 (q, *J =* 7.0 Hz, 2H), 1.23 (t, *J =* 7.0 Hz, 3H). ¹³C NMR (151 MHz, DMSO) δ 165.4, 160.6, 141.8, 131.2, 125.8, 122.1, 121.8, 118.2, 100.1, 67.2, 15.0. Elem. Anal. Calcd. for C₁₁H₁₂BrNO₂: C, 48.91; H, 4.48; N, 5.19. Found: C, 48.55; H, 4.37; N, 4.95.

### EXAMPLE 5

### 3-Ethoxy-N-(3-ethylphenyl)acrylamide (IIe, intermediate compound)

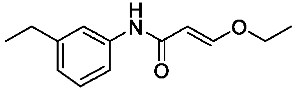

Title compound was prepared according to General Procedure I (Scheme 1). Yield: 78 % as a light yellow solid; mp 81-83 °C. ¹H NMR (600 MHz, DMSO) δ 9.60 (s, 1H), 7.43-7.37 (m, 3H), 7.13 (t, *J =* 7.8 Hz, 1H), 6.82-6.80 (m, 1H), 5.49 (d, *J =* 12.3 Hz, 1H), 3.90 (q, *J =* 7.1 Hz, 2H), 2.52 (q, *J =* 7.6 Hz, 2H), 1.23 (t, *J =* 7.0 Hz, 3H), 1.12 (t, *J =* 7.6 Hz, 3H). ¹³C NMR (151 MHz, DMSO) δ 165.09, 159.88, 144.69, 140.19, 129.09, 122.78, 118.87, 116.99, 100.48, 66.99, 28.81, 16.02, 15.01. Elem. Anal. Calcd. for C₁₃H₁₇NO₂: C, 71.21; H, 7.81; N, 6.39. Found: C, 71.24; H, 7.85; N, 6.38.

### EXAMPLE 6

### 3-Ethoxy-N-(3-methylphenyl)acrylamide (IIf, intermediate compound)

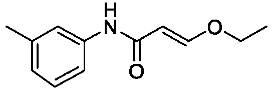

Title compound was prepared according to General Procedure I (Scheme 1). Yield: 68 % as a yellow solid; mp 75-76 °C. ¹H NMR (600 MHz, DMSO) δ 9.58 (s, 1H), 7.42 (d, *J* = 12.3 Hz, 1H), 7.39 (s, 1H), 7.35 (dd, *J =* 8.2, 2.0 Hz, 1H), 7.11 (t, *J* = 7.8 Hz, 1H), 6.81-6.76 (m, 1H), 5.48 (d, *J* = 12.2 Hz, 1H), 3.90 (q, *J =* 7.0 Hz, 2H), 2.22 (s, 3H), 1.23 (t, *J =* 7.0 Hz, 3H). ¹³C NMR (151 MHz, DMSO) δ 165.08, 159.89, 140.13, 138.30, 129.04, 123.95, 120.04, 116.72, 100.48, 66.99, 21.75, 15.02. Elem. Anal. Calcd. for C₁₂H₁₅NO₂: C, 70.22; H, 7.37; N, 6.82. Found: C, 69.91; H, 7.33; N, 6.74.

### General procedure 2: Cyclization to heterocycle of general formula III

*N*-(Substituted-phenyl)-3-ethoxyacrylamide II (3.5 mmol) was dissolved in sulfuric acid (20 mL) at cooling and then stirred at rt for 3 hours. The reaction mixture was poured into ice water (200 mL) and stirred at rt for 1 hour. The precipitated product was filtered off, washed with water to neutral pH and then with diethyl ether (25 mL) and dried over P₂O₅.

### General procedure 3: Cyclization to heterocycle of general formula III

Aluminum chloride (4.1 g, 30.5 mmol) was added portion wise to a solution of*N*-(substituted-phenyl)-3-ethoxyacrylamide II (6.1 mmol) in chlorobenzene (40 mL) at rt. The reaction mixture was heated to 90 °C for 4 hours. The reaction mixture was cooled to rt, poured into ice water (200 mL), stirred at rt for 30 min and then extracted with EtOAc (3 × 70 mL). The organic layer was washed with water (1 × 50 mL) and brine (1 × 40 mL), dried over anhydrous sodium sulfate, and concentrated under reduced pressure. The product was purified by column chromatography (mobile phase: hexane/EtOAc/CH₃COOH, 60:40:1).

### EXAMPLE 7

### The mixture of 7-chloroquinolin-2(1H)-one (IIIa) and 5-chloroquinolin-2(1H)-one and (IIIb), (intermediate compounds)

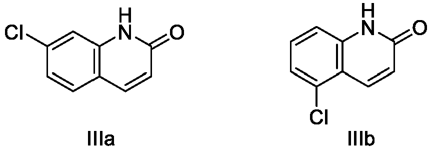

Title compounds were prepared according to General Procedure 2 (Scheme 1). The product was obtained as a mixture of two isomers 7-chloroquinolin-2(1*H*)-one and 5-chloroquinolin-2(1*H*)-one in a ratio 2:1. Total yield 88 % as a white solid. 7-Chloroquinolin-2(1*H*)-one (IIIa): ¹H NMR (600 MHz, DMSO) δ 7.87 (d, *J =* 9.6 Hz, 1H), 7.65 (d, *J* = 8.4 Hz, 1H), 7.29 (d, *J* = 2.0 Hz, 1H), 7.17 (dd, *J =* 2.1, 8.4 Hz, 1H), 6.47 (d, *J* = 9.5 Hz, 1H). ¹³C NMR (151 MHz, DMSO) δ 162.3, 140.3, 136.2, 131.7, 130.2, 122.7, 122.5, 114.9. 5-Chloroquinolin-2(1H)-one (IIIb): ¹H NMR (600 MHz, DMSO) δ 8.04 (dd, *J* = 0.7, 9.8 Hz, 1H), 7.45 (t, *J =* 8.0 Hz, 1H), 7.26-7.24 (m, 2H), 6.59 (d, *J* = 9.8 Hz, 1H). ¹³C NMR (151 MHz, DMSO) δ 162.1, 140.8, 140.3, 135.3, 131.6, 123.9, 122.8, 118.5, 115.3.

### EXAMPLE 8

### 6-Chloroquinolin-2(1H)-one (IIIc, intermediate compound)

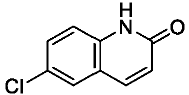

Title compound was prepared according to General Procedure 2 (Scheme 1). Yield: 84 % as a light beige solid; mp 212-214 °C. ¹H NMR (600 MHz, DMSO) δ 11.82 (s, 1H), 7.84 (d, *J =* 9.6 Hz, 1H), 7.74 (d, J = 2.4 Hz, 1H), 7.49 (dd, *J =* 2.4, 8.8 Hz, 1H), 7.27 (d, *J =* 8.8 Hz, 1H), 6.52 (d, *J =* 9.6 Hz, 1H). ¹³C NMR (151 MHz, DMSO) δ 162.28, 139.80, 138.10, 130.76, 127.42, 126.15, 123.67, 120.82, 117.56. Anal. Calcd. for C₉H₆ClNO: C, 60.19; H, 3.37; N, 7.80. Found: C, 60.52; H, 3.32; N, 7.89.

### EXAMPLE 9

### 5,7-Dichloroquinolin-2(1H)-one (IIId, intermediate compound)

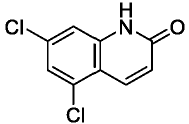

Title compound was prepared according to General Procedure 2 (Scheme 1). Yield: 42 % as a brown solid; mp 89-90 °C. ¹H NMR (600 MHz, DMSO) δ 12.04 (s, 1H), 8.00 (d, *J =* 9.8 Hz, 1H), 7.42 (d, *J =* 2.0 Hz, 1H), 7.28 (d, *J =* 2.0 Hz, 1H), 6.61 (d, *J =* 9.8 Hz, 1H). ¹³C NMR (151 MHz, DMSO) δ 161.91, 141.33, 135.78, 135.31, 132.80, 124.28, 122.54, 116.03, 114.61. Elem. Anal. Calcd. for C₉H₅Cl₂NO: C, 50.50; H, 2.35; N, 6.54. Found: C, 50.35; H, 2.68; N, 6.34.

### EXAMPLE 10

### The mixture of 7-bromoquinolin-2(1H)-one (IIIe) and 5-bromoquinolin-2(1H)-one (IIIf), (intermediate compounds)

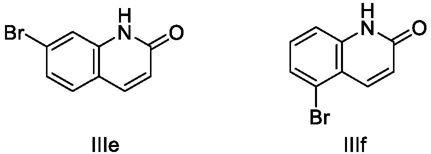

Title compounds were prepared according to General Procedure 2 (Scheme 1). The product was obtained as a mixture of two isomers 7-bromoquinolin-2(1*H*)-one and 5-bromoquinolin-2(1*H*)-one in a ratio 1.5:1. Total yield 78 % as a light yellow solid. 7-Bromoquinolin-2(1*H*)-one (IIIe): ¹H NMR (600 MHz, DMSO) δ 11.79 (s, 1H), 7.86 (dd, *J =* 0.6, 9.6 Hz, 1H), 7.57 (d, *J* = 8.4 Hz, 1H), 7.44 (d, *J* = 2.0 Hz, 1H), 7.30 (d, *J* = 2.0 Hz, 1H), 6.48 (d, *J* = 9.5 Hz, 1H). ¹³C NMR (151 MHz, DMSO) δ 162.3, 140.4, 138.7, 130.3, 125.2, 124.2, 122.9, 118.7, 117.9. 5-Bromoquinolin-2(1H)-one (IIIf): ¹H NMR (600 MHz, DMSO) δ 11.79 (s, 1H), 7.98 (dd, *J* = 0.7, 9.8 Hz, 1H), 7.43 (dd, *J* = 0.6, 9.6 Hz, 1H), 7.37 (t, *J* = 8.0 Hz, 1H), 7.28 (d, *J= 2.0* Hz, 1H), 6.59 (d, *J =* 9.8 Hz, 1H). ¹³C NMR (151 MHz, DMSO) δ 162.1, 140.7, 140.4, 132.0, 126.3, 123.9, 122.2, 118.4, 115.9.

### EXAMPLE 11

### 7-Ethylquinolin-2(1H)-one (IIIg, intermediate compound)

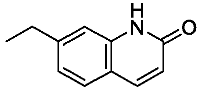

Title compound was prepared according to General Procedure 3 (Scheme 1). Yield: 73 % as a beige solid; mp 152-154 °C. ¹H NMR (600 MHz, DMSO) δ 11.60 (s, 1H), 7.80 (d, *J =* 9.48 Hz, 1H), 7.51 (d, *J* = 8.0 Hz, 1H), 7.08 (d, *J=* 1.6 Hz, 1H), 7.00 (dd, *J* = 1.7, 8.0 Hz, 1H), 6.37 (d, *J* = 9.4 Hz, 1H), 2.62 (q, *J =* 7.6 Hz, 2H), 1.16 (t, *J =* 7.6 Hz, 3H). ¹³C NMR (151 MHz, DMSO) δ 162.62, 147.15, 140.57, 139.64, 128.34, 122.58, 121.37, 117.76, 114.23, 28.88, 15.79. Elem. Anal. Calcd. for C₁₁H₁₁NO: C, 76.28; H, 6.40; N, 8.09. Found: C, 75.96; H, 6.38; N, 7.98.

### EXAMPLE 12

### The mixture of 7- methylquinolin-2(1H)-one (IIIh) and 5-methylquinolin-2(1H)-one (IIIi), (intermediate compounds)

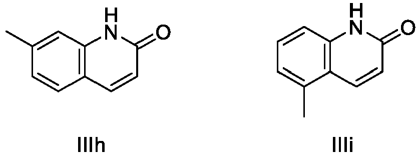

Title compounds were prepared according to General Procedure 3 (Scheme 1). The product was obtained as a mixture of two isomers 5- and 7-methylquinolin-2-on in a ratio 1:4. Total yield: 68 % as a beige solid. 7-Methylquinolin-2-one (IIIh): ¹H NMR (600 MHz, DMSO) δ 11.61 (s, 1H), 7.79 (d, *J* = 9.5 Hz, 1H), 7.48 (d, *J =* 8.0 Hz, 1H), 7.07-7.03 (m, 1H), 6.96-6.92 (m, 1H), 6.37 (d, *J =* 9.5 Hz, 1H), 2.32 (s, 3H). 5-Methylquinolin-2-one (IIIi): ¹H NMR (600 MHz, DMSO) δ 11.66 (s, 1H), 7.98 (dd, *J =* 9.7, 0.7 Hz, 1H), 7.32 (dd, *J =* 8.3, 7.3 Hz, 1H), 7.12 (d, *J =* 8.3 Hz, 1H), 6.97-6.96 (m, 1H), 6.46 (d, *J =* 9.7 Hz, 1H), 2.32 (s, 3H).

### General procedure 4: Cyclization to heterocycle of general formula III

1M solution of sodium *bis*(trimethylsilyl)amide in THF (16.9 mL, 16.9 mmol) was added dropwise at 0 °C under inert atmosphere to a solution of substituted aniline (11.2 mmol) and methyl 3,3-dimethoxypropanoate (2.0 g/1.9 mL, 13.5 mmol) in dry THF (25 mL). The reaction mixture was stirred at rt for 12 hours and then a solution of citric acid (20 %, 50 mL) was added carefully at cooling. The mixture was extracted with CH₂Cl₂ (4 × 50 mL). The organic layer was then washed with water (2 × 50 mL) and brine (1 × 50 mL). The organic layer was dried over anhydrous sodium sulfate and concentrated under reduced pressure. The crude product was carefully dissolved in sulfuric acid (10 mL) at cooling and the reaction mixture was stirred at rt for 4 hours. Then it was poured into ice water (150 mL), stirred at rt for 30 min and then extracted with EtOAc (3 × 40 mL). The organic layer was then washed with water (2 × 50 mL) and brine (1 × 30 mL), dried over anhydrous sodium sulfate, and concentrated under reduced pressure. The product was suspended in small amount of diethyl ether and filtered off.

### EXAMPLE 13

### 7-Fluoroquinolin-2(1H)-one (IIIj, intermediate compound)

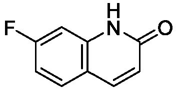

Title compounds were prepared according to General Procedure 4 (Scheme 1). Yield: 75 % as a light yellow solid; mp 274-276 °C (decomp.). ¹H NMR (600 MHz, DMSO) δ 11.76 (s, 1H), 7.85 (d, *J =* 9.6 Hz, 1H), 7.68-7.66 (m, 1H), 7.00-6.96 (m, 2H), 6.41 (d, *J =* 9.6 Hz, 1H). ¹³C NMR (151 MHz, DMSO) δ 163.57 (d, *J* = 246.7 Hz), 162.48, 140.96 (d, *J* = 12.3 Hz), 140.33, 130.90 (d, *J =* 10.6 Hz), 121.45 (d, *J* = 3.0 Hz), 116.68, 110.40 (d, *J =* 23.3 Hz), 101.56 (d, *J* = 25.5 Hz).

### EXAMPLE 14

### The mixture of 7-(trifluoromethyl)quinolin-2(1H)-one (IIIk) and 5-(trifluoromethyl)quinolin-2(1H)-one (IIII), (intermediate compounds)

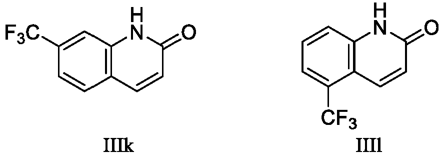

Title compounds were prepared according to General Procedure 4 (Scheme 1). The product was obtained as a mixture of two isomers 7-(trifluoromethyl)quinolin-2(1*H*)-one and 5-(trifluoromethyl)quinolin-2(1*H*)-one in a ratio 3:2. Total yield 94 % as a light yellow solid. 7-(Trifluoromethyl)quinolin-2(1*H*)-one (IIIk):. ¹H NMR (500 MHz, DMSO) δ 12.05 (1H), 8.00 (d, *J* = 9.6 Hz, 1H), 7.87 (d, J = 8.3 Hz, 1H), 7.61-7.58 (m, 1H), 7.45 (dd, *J* = 8.3 Hz, *J* = 1.8 Hz, 1H), 6.65 (d, *J =* 9.6 Hz, 1H). ¹³C NMR (125 MHz, DMSO) δ 161.9, 139.7, 138.9, 130.2 *(q, J= 31.8* Hz), 129.5, 124.1 (q, *J* = 272.8 Hz), 124.8, 121.9, 117.9 (q, *J* = 3.6 Hz), 112.1 (q, *J* = 4.6 Hz) 5-(Trifluoromethyl)quinolin-2(1H)-one (IIII): ¹H NMR (500 MHz, DMSO) δ 12.05 (1H) 7.96-7.92 (m, 1H), 7.65 (t, *J =* 7.8 Hz, 1H), 7.61-7.58 (m, 1H), 7.55 (d, *J* = 7.4 Hz, 1H), 6.70 (d, *J =* 9.9 Hz, 1H). ¹³C NMR (125 MHz, DMSO) δ 161.2, 140.4, 135.0, 130.2, 125.3 (q, *J =* 30.2 Hz), 124.7, 124.2 (q, *J =* 273.8 Hz), 120.5, 119.9 (q, *J =* 5.5 Hz), 114.9 (q, *J=* 1.5 Hz)

### EXAMPLE 15

### 5,7-Dimethylquinolin-2(1H)-one (IIIm, intermediate compound)

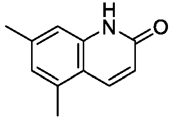

Title compound was prepared according to General Procedure 4 (Scheme 1). Yield: 27 % as a yellow solid; mp 256-258 °C. ¹H NMR (600 MHz, DMSO) δ 11.59 (s, 1H), 7.92 (dd, *J* = 0.7, 9.8 Hz, 1H), 6.90 (d, *J=* 0.8 Hz, 1H), 6.80 (d, *J =* 0.8 Hz, 1H), 6.37 (d, *J* = 9.7 Hz, 1H), 2.41 (s, 3H), 2.27 (s, 3H). ¹³C NMR (151 MHz, DMSO) δ 162.42, 140.70, 140.02, 137.39, 135.63, 125.04, 120.71, 116.23, 113.70, 21.79, 18.67. Elem. Anal. Calcd. for C₁₁H₁₁NO: C, 76.28; H, 6.40; N, 8.09. Found: C, 75.98; H, 6.15; N, 7.99.

### EXAMPLE 16

### The mixture of 7-iodoquinolin-2(1H)-one (IIIn) and 5-iodoquinolin-2(1H)-one (IIIo), (intermediate compounds)

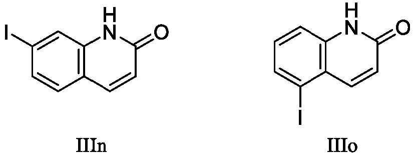

Title compounds were prepared according to General Procedure 4 (Scheme 1). The product was suspended in small amount of diethyl ether, filtered off and obtained as a mixture of two isomers 7-iodoquinolin-2(1*H*)-one and 5-iodoquinolin-2(1*H*)-one in a ratio 3:2. Total yield: 76% as a brownish solid. 7-Iodoquinolin-2(1H)-one (IIIn): ¹H NMR (600 MHz, DMSO) δ 11.69 (s, 1H), 7.86-7.84 (m, 1H), 7.63 (d, *J* = 1.6 Hz, 1H), 7.45 (dd, *J* = 8.2, 1.6 Hz, 1H), 7.40 (d, *J* = 8.2 Hz, 1H), 6.47 (d, *J =* 9.6 Hz, 1H). 5-Iodoquinolin-2(1*H*)-one (IIIo): ¹H NMR (600 MHz, DMSO) δ 11.85 (s, 1H), 7.84-7.82 (m, 1H), 7.68 (dd, *J =* 7.6, 1.0 Hz, 1H), 7.32-7.25 (m, 1H), 7.19 (dd, *J =* 8.3, 7.6 Hz, 1H), 6.55 (d, *J =* 9.8 Hz, 1H).

### EXAMPLE 17

### Synthesis of 7-nitroquinolin-2(1H)-one (IIIp, intermediate compound)

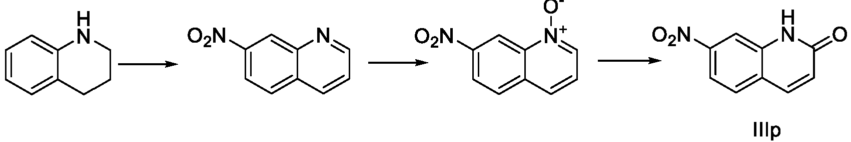

1,2,3,4-Tetrahydroquinoline (6.5 g, 0.049 mol) was added dropwise to a 96 % H₂SO₄ (40 mL) cooled in ice bath followed by addition of KNO₃ (4.94 g, 0.049 mol) in several portions. The reaction mixture was warmed to rt and stirred for 2 hours. The reaction mixture was poured into ice water (250 mL), stirred for 30 min and then extracted with EtOAc (3 × 150 mL). The organic phase was additionally washed with water (2 × 150 mL) and brine (1 × 150 mL), dried over anhydrous sodium sulfate and concentrated. The yield of 7-nitro-1,2,3,4-tetrahydroquinoline was 90 % as a brown oil. The obtained product was used in the next step without purification.

Diethyl azodicarboxylate (40 % in toluene, 33.6 mL, 0.074 mol) was added dropwise to a solution of 7-nitro-1,2,3,4-tetrahydroquinoline (6 g, 0.034 mol) in CH₂Cl₂ (150 mL). The reaction mixture was heated to 50 °C for 15 hours and then concentrated. The product was purified by column chromatography (mobile phase: hexane/EtOAc, 7:1). The yield of 7-nitroquinoline: 49 % as a beige solid; mp 128-129 °C. ¹H NMR (600 MHz, CDCl₃) δ 9.07 (dd, *J* = 4.1, 1.7 Hz, 1H), 9.00 (d, *J* = 2.4 Hz, 1H), 8.31 (dd, *J* = 8.9, 2.3 Hz, 1H), 8.26 (dd, *J* = 8.4, 1.1 Hz, 1H), 7.97 (d, *J =* 8.9 Hz, 1H), 7.58 (dd, *J =* 8.4, 4.2 Hz, 1H). ¹³C NMR (151 MHz, CDCl₃) δ 152.77, 148.20, 147.25, 136.02, 131.47, 129.56, 125.94, 124.02, 120.20.

MCPBA (77 %, 4.63 g, 21.0 mmol) was added in small portions to a solution of 7-nitroquinoline (2.6 g, 14.93 mmol) in CH₂Cl₂ (100 mL). The reaction mixture was stirred at rt for 48 hours. The reaction mixture was washed with NaHCO₃ (2 × 50 mL), water (2 × 75 ml) and brine (1 × 50 mL). The organic phase was dried over anhydrous sodium sulfate and concentrated. The yield of 7-nitroquinoline *N*-oxide was 93 % as a yellow solid; mp 173-174 °C. ¹H NMR (600 MHz, CDCl₃) δ 9.58 (d, *J* = 2.3 Hz, 1H), 8.58 (d, *J* = 6.0 Hz, 1H), 8.39 (dd, *J* = 8.9, 2.2 Hz, 1H), 8.04 (d, *J* = 8.9 Hz, 1H), 7.79 (d, *J =* 8.5 Hz, 1H), 7.49 (dd, *J* = 8.5*,* 6.0 Hz, 1H). ¹³C NMR (151 MHz, CDCl₃) δ 148.56, 141.37, 136.93, 133.56, 130.24, 124.74, 124.56, 122.57, 117.09. Elem. Anal. Calcd. for C₉H₆N₂O₃: C, 56.85; H, 3.18; N, 14.73. Found: C, 57.03; H, 3.34; N, 14.67.

Trifluoroacetanhydride (24.36 g/16.13 mL, 0.116 mol) was added dropwise to a suspension of 7-nitroquinoline *N*-oxide (2.2 g, 0.0116 mol) in DMF (15 mL) under inert atmosphere and cooling. The reaction mixture was stirred at rt overnight and turned into solution. The reaction mixture was poured into cooled saturated solution of Na₂CO₃ (150 mL). The precipitation was filtered off, washed with water and EtOAc (15 mL). The yield of 7-nitroquinolin-2(1*H*)-one was 64 % as a yellowish solid; mp 338-340 °C. ¹H NMR (600 MHz, DMSO) δ 12.07 (s, 1*H*), 8.08 (s, 1H), 8.00 (d, *J =* 9.6 Hz, 1H), 7.93-7.86 (m, 2H), 6.68 (d, *J =* 9.6 Hz, 1H). ¹³C NMR (151 MHz, DMSO) δ 162.16, 148.39, 139.74, 139.40, 130.01, 126.21, 124.17, 116.45, 110.77. Elem. Anal. Calcd. for C₉H₆N₂O₃: C, 56.85; H, 3.18; N, 14.73. Found: C, 56.69; H, 3.2; N, 14.54.

### EXAMPLE 18

### Synthesis of 5-nitroquinolin-2(1H)-one (IIIq, intermediate compound)

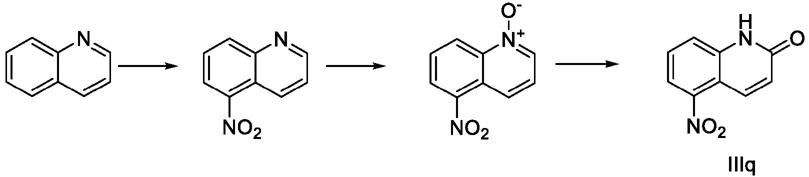

Quinoline (6.5 g/5.95 mL, 0.05 mol) was added dropwise to cooled H₂SO₄ (30 mL) followed by slow addition of 65 % HNO₃ (14.6 g/10 mL, 0.15 mol). The reaction mixture was stirred at rt for 3 hours and then slowly poured into saturated solution of Na₂CO₃ (500 mL). The precipitation was filtered off and washed with water. The filtrate cake was dissolved in EtOAc (400 mL) and washed with saturated solution of NaHCO₃ (1 × 150 mL), water (2 × 200 mL) and brine (1 × 200 mL). The organic phase was dried over anhydrous sodium sulfate and concentrated. The mixture of 5- and 8-nitroquinolines was separated by column chromatography (mobile phase: hexane/EtOAc, 7:1). Yield of 5-Nitroquinoline was 19 % as a beige solid; mp 63-64 °C. ¹H NMR (600 MHz, CDCl₃) δ 9.02 (dd, *J* = 4.2, 1.6 Hz, 1H), 8.98 (ddd, *J =* 8.8, 1.6, 0.9 Hz, 1H), 8.41 (dt, *J =* 8.4, 1.1 Hz, 1H), 8.36 (dd, *J =* 7.7, 1.2 Hz, 1H), 7.79 (dd, *J =* 8.4, 7.7 Hz, 1H), 7.63 (dd, *J =* 8.8, 4.2 Hz, 1H). ¹³C NMR (151 MHz, CDCl₃) δ 151.73, 148.36, 145.66, 136.71, 132.02, 127.58, 124.75, 124.03, 121.31.

MCPBA (77 %, 4.63 g, 21.0 mmol) was added in small portions to a solution of 5-nitroquinoline (2.6 g, 14.93 mmol) in CH₂Cl₂ (100 mL). The reaction mixture was stirred at rt for 48 hours. The reaction mixture was washed with NaHCO₃ (2 × 50 mL), water (2 × 75 ml) and brine (1 × 50 mL). The organic phase was dried over anhydrous sodium sulfate and concentrated. Yield of 5-nitroquinoline N-oxide was 90% as a yellow solid; mp 160-161 °C. ¹H NMR (600 MHz, DMSO) δ 8.89 (dt, *J =* 8.8, 1.1 Hz, 1H), 8.70 (dd, *J =* 6.1, 0.9 Hz, 1H), 8.49 (dd, *J =* 7.7, 1.2 Hz, 1H), 8.22 (dt, *J =* 9.0, 0.9 Hz, 1H), 7.93 (dd, *J =* 8.8, 7.7 Hz, 1H), 7.65 (dd, *J* = 9.0, 6.1 Hz, 1H). ¹³C NMR (151 MHz, DMSO) δ 146.50, 142.15, 136.72, 129.50, 127.18, 125.97, 125.41, 123.66, 120.25.

Trifluoroacetanhydride (24.36 g/16.13 mL, 0.116 mol) was added dropwise to a suspension of 5-nitroquinoline *N*-oxide (2.2 g, 0.0116 mol) in DMF (15 mL) under inert atmosphere and cooling. The reaction mixture was stirred at rt overnight and turned into solution. The reaction mixture was poured into cooled saturated solution of Na₂CO₃ (150 mL). The precipitation was filtered off, washed with water and EtOAc (15 mL). Yield of 5-nitroquinolin-2(1*H*)-one was 87 % as a beige solid; mp 310-312 °C (with decomposition). ¹H NMR (600 MHz, DMSO) δ 12.20 (s, 1H), 8.20 (d, *J =* 10.0 Hz, 1H), 7.82 (dd, *J* = 7.8, 1.2 Hz, 1H), 7.66 (t, *J =* 8.1 Hz, 1H), 7.61 (d, *J* = 8.2 Hz, 1H), 6.71 (d, *J* = 10.1 Hz, 1H). ¹³C NMR (151 MHz, DMSO) δ 161.37, 146.82, 140.79, 134.73, 130.63, 125.88, 121.55, 119.02, 112.03. Elem. Anal. Calcd. for C₉H₆N₂O₃: C, 56.85; H, 3.18; N, 14.73. Found: C, 56.58; H, 3.02; N, 14.84.

### General procedure 5: Synthesis of 2-chloroquinolines of general formula IV

Substituted quinolin-2(1*H*)-one III (1.3 mmol) was dissolved in POCl₃ (10 mL) and DMF (catalytic amount) was added. The reaction mixture was stirred for 3 hours at 110 °C and for 12 hours at rt and then carefully poured into ice (100 mL). The precipitated product was filtered off, washed with water to neutral pH and dried over P₂O₅.

### General procedure 6: Synthesis of 2-chloroquinolines of general formula IV

POCl₃ (5.1 g/3.1 mL, 33.2 mmol) was added to a solution of substituted quinolin-2(1*H*)-one III (16.6 mmol) in DMF (30 mL) and the reaction mixture was heated to 80-85 °C for 4 hours. After cooling, the reaction mixture was poured into saturated solution of NaHCO₃ (450 mL) and extracted with EtOAc (3 × 200 mL). The organic layer was additionally washed with water (2 × 150 mL) and brine (1 × 100 mL), dried over anhydrous sodium sulfate and concentrated. The product was purified by column chromatography (mobile phase: hexane/EtOAc).

### EXAMPLE 19

### 2,7-Dichloroquinoline (IVa, intermediate compound)

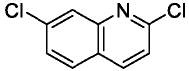

Title compound was prepared according to General Procedure 5 (Scheme 1). The product was obtained in the mixture with 2,5-dichloroquinoline and was separated by column chromatography (mobile phase: hexane/EtOAc, 7:1). The yield: 67 % as a white solid; mp 120-121 °C. ¹H NMR (600 MHz, DMSO) δ 8.45 (d, *J* = 8.6 Hz, 1H), 8.06 (d, *J =* 8.7 Hz, 1H), 7.99 (d, *J* = 2.1 Hz, 1H), 7.66 (dd, *J =* 2.1, 8.7 Hz, 1H), 7.59 (d, *J =* 8.6 Hz, 1H). ¹³C NMR (151 MHz, DMSO) δ 151.7, 148.1, 140.6, 136.1, 130.6, 128.5, 127.2, 125.9, 123.4. Elem. Anal. Calcd. for C₉H₅Cl₂N: C, 54.58; H, 2.54; N, 7.07. Found: C, 54.44; H, 2.37; N, 7.04.

### EXAMPLE 20

### 2,5-Dichloroquinoline (IVb, intermediate compound)

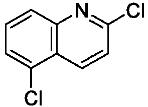

Title compound was prepared according to General Procedure 5 (Scheme 1). The product was obtained in the mixture with 2,7-dichloroquinoline, and was separated by column chromatography (mobile phase: hexane/EtOAc, 7:1).The yield: 31 % as a white solid; mp 72-74 °C. ¹H NMR (600 MHz, DMSO) δ 8.54 (dd, *J* = 0.8, 8.8 Hz, 1H), 7.93-7.91 (m, 1H), 7.80-7.76 (m, 2H), 7.69 (d, *J* = 8.8 Hz, 1H). ¹³C NMR (151 MHz, DMSO) δ 151.5, 148.4, 136.8, 131.8, 130.9, 128.13, 128.11, 125.1, 124.3. Elem. Anal. Calcd. for C₉H₅Cl₂N: C, 54.58; H, 2.54; N, 7.07. Found: C, 54.78; H, 2.58; N, 7.07.

### EXAMPLE 21

### 2,6-Dichloroquinoline (IVc, intermediate compound)

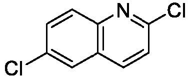

Title compound was prepared according to General Procedure 5 (Scheme 1). The product was purified by column chromatography (mobile phase: hexane/EtOAc, 1:1). The yield: 66 % as a white solid; mp 153-155 °C. ¹H NMR (600 MHz, DMSO) δ 8.38 (d, *J =* 8.6 Hz, 1H), 8.14 (d, *J =* 2.4 Hz, 1H), 7.93 (d, *J =* 9.0 Hz, 1H), 7.79 (dd, *J =* 2.5, 9.0 Hz, 1H), 7.61 (d, *J =* 8.7 Hz, 1H). ¹³C NMR (151 MHz, DMSO) δ 150.88, 146.17, 139.83, 132.11, 131.90, 130.46, 128.09, 127.43, 124.03. Elem. Anal. Calcd. for C₉H₅Cl₂N: C, 54.58; H, 2.54; N, 7.07. Found: C, 54.24; H, 2.44; N, 7.03.

### EXAMPLE 22

### 2,5,7-Trichloroquinoline (IVd, intermediate compound)

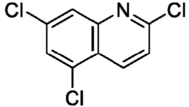

Title compound was prepared according to General Procedure 5 (Scheme 1). The product was suspended with diethyl ether, filtered off and dried over P₂O₅. The yield: 67 % as a white solid; mp 129-130 °C. ¹H NMR (600 MHz, DMSO) δ 8.52 (d, *J =* 8.9 Hz, 1H), 8.01 (d, *J =* 2.0 Hz, 1H), 7.94 (d, *J= 2.0* Hz, 1H), 7.71 (d, *J* = 8.9 Hz, 1H). ¹³C NMR (151 MHz, DMSO) δ 152.73, 148.28, 136.88, 135.75, 132.38, 128.29, 127.08, 124.65, 124.01. Elem. Anal. Calcd. for C₉H₄Cl₃N: C, 46.50; H, 1.73; N, 6.02. Found: C, 46.32; H, 1.92; N, 5.83.

### EXAMPLE 23

### 7-Bromo-2-chloroquinoline (IVe, intermediate compound)

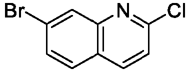

Title compound was prepared according to General Procedure 5 (Scheme 1). The product was obtained in the mixture with 5-bromo-2-chloroquinoline and was separated by column chromatography (mobile phase: hexane/EtOAc, 7:1). Yield: 41 % as a white solid; mp 115-116 °C. ¹H NMR (600 MHz, DMSO) δ 8.44 (d, *J* = 8.7 Hz, 1H), 8.14 (d, *J* = 1.9 Hz, 1H), 7.97 (d, *J =* 8.7 Hz, 1H), 7.77 (dd, *J =* 2.00, 8.7 Hz, 1H), 7.60 (d, *J =* 8.6 Hz, 1H). ¹³C NMR (151 MHz, DMSO) δ 151.6, 148.2, 140.6, 130.9, 130.6, 130.4, 126.2, 124.8, 123.5. Elem. Anal. Calcd. for C₉H₅BrClN: C, 44.58; H, 2.08; N, 5.78. Found: C, 44.83; H, 1.95; N, 5.74.

### EXAMPLE 24

### 5-Bromo-2-chloroquinoline (IVf, intermediate compound)

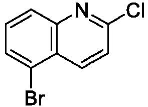

Title compound was prepared according to General Procedure 5 (Scheme 1). The product was obtained in the mixture with 7-bromo-2-chloroquinoline and was separated by column chromatography (mobile phase: hexane/EtOAc, 7:1). The yield: 16 % as a white solid; mp 77-78 °C. ¹H NMR (600 MHz, DMSO) δ 8.49 (dd, *J* = 0.8, 8.9 Hz, 1H), 7.98-7.95 (m, 2H), 7.73-7.69 (m, 2H). ¹³C NMR (151 MHz, DMSO) δ 151.5, 148.4, 139.3, 132.3, 131.7, 128.8, 126.5, 124.6, 121.6. Elem. Anal. Calcd. for C₉H₅BrClN: C, 44.58; H, 2.08; N, 5.78. Found: C, 44.47; H, 1.91; N, 5.75.

### EXAMPLE 25

### 2-Chloro-7-fluoroquinoline (IVg, intermediate compound)

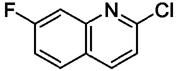

Title compound was prepared according to General Procedure 5 (Scheme 1). After completion, the reaction mixture was extracted with EtOAc (2 × 50 mL). The organic phase was washed with water (2 × 50 mL) and brine (1 × 50 mL), dried over anhydrous sodium sulfate and concentrated. The product was suspended with diethyl ether (25 mL), filtered off and dried over P₂O₅ to give product in high purity. The yield: 90 % as a beige solid; mp 98-99 °C. ¹H NMR (600 MHz, DMSO) δ 8.44 (dd, *J* = 0.7, 8.6 Hz, 1H), 8.10 (dd, *J* = 6.3, 9.0 Hz, 1H), 7.69 (dd, *J* = 2.6*,* 10.3 Hz, 1H), 7.57-7.53 (m, 2H). ¹³C NMR (151 MHz, DMSO) δ 163.66 (d, *J* = 249.3 Hz), 151.66, 148.69 (d, *J =* 13.3 Hz), 140.56, 131.42 (d, *J =* 10.4 Hz), 124.58, 122.36 (d, *J* = 2.7 Hz), 117.97 (d, *J =* 25.2 Hz), 112.26 (d, *J =* 21.3 Hz).

### EXAMPLE 26

### 2-Chloro-7-(trifluoromethyl)quinoline (IVh, intermediate compound)

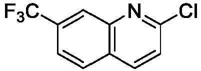

Title compound was prepared according to General Procedure 5 (Scheme 1). The product was obtained in the mixture with 2-chloro-5-(trifluoromethyl)quinoline and was separated by column chromatography (mobile phase: hexane/EtOAc, 8:1). The yield: 55 % as a white solid; mp 78-80 °C. ¹H NMR (600 MHz, DMSO) δ 8.54 (d, *J =* 8.3 Hz, 1H), 8.24 (d, *J =* 8.7 Hz, 1H), 7.88-7.86 (m, 2H), 7.72 (d, *J =* 8.7 Hz, 1H). ¹³C NMR (151 MHz, DMSO) δ 152.17, 146.61, 140.62, 131.28 (q, *J* = 32.3 Hz), 130.70, 129.14, 125.93 (q, *J =* 4.4 Hz), 125.19, 124.29 (q, *J=* 273.1 Hz), 123.06 (d, *J* = 3.1 Hz).

### EXAMPLE 27

### 2-Chloro-5-(trifluoromethyl)quinoline (IVi, intermediate compound)

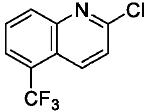

Title compound was prepared according to General Procedure 5 (Scheme 1). The product was obtained in the mixture with 2-chloro-7-(trifluoromethyl)quinoline and was separated by column chromatography (mobile phase: hexane/EtOAc, 8:1). The yield 28 % as a white solid; mp 41-43 °C. ¹H NMR (600 MHz, DMSO) δ 8.45 (dd, *J =* 1.1, 9.0 Hz, 1H), 8.22 (d, *J =* 8.5 Hz, 1H), 8.07 (d, *J* = 7.3 Hz, 1H), 7.92 (t, *J =* 8.0 Hz, 1H), 7.75 (d, *J* = 9.0 Hz, 1H). ¹³C NMR (151 MHz, DMSO) δ 151.51, 148.08, 136.02, 133.85, 130.52, 126.70 (q, *J =* 5.7 Hz), 125.43 (q, *J* = 31.1 Hz), 124.93, 124.38 (d, *J =* 273.1 Hz), 122.82.

### EXAMPLE 28

### 2-Chloro-7-ethylquinoline (IVj, intermediate compound)

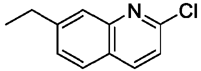

Title compound was prepared according to General Procedure 5 (Scheme 1). After completion, the reaction mixture was extracted with EtOAc (2 × 50 mL) and washed with water (2 × 50 mL) and brine (1 × 50 mL); the organic phase was dried over anhydrous sodium sulfate and concentrated. The product was suspended with diethyl ether (25 mL), filtered off and dried over P₂O₅ and obtained in high purity. The yield: 92 % as a brown solid; mp 148-149 °C. ¹H NMR (600 MHz, DMSO) δ 8.35 (d, *J* = 7.0 Hz, 1H), 7.91 (d, *J* = 7.0 Hz, 1H) 7.72-7.70 (m, 1H), 7.50 (dd, *J=* 7.0, 1.4 Hz, 1H), 7.47 (d, *J* = 7.0 Hz, 1H), 2.78 (q, *J* = 6.3 Hz, 2H), 1.24 (d, *J =* 6.3 Hz, 3H). ¹³C NMR (151 MHz, DMSO) δ 149.9, 147.7, 147.3, 139.8, 128.4, 128.0, 125.7, 125.20, 121.7, 28.5, 15.3. Elem. Anal. Calcd. for C₁₁H₁₀ClN: C, 68.94; H, 5.26; N, 7.31. Found: C, 68.74; H, 5.02; N, 7.18.

### EXAMPLE 29

### 2-Chloro-5,7-dimethylquinoline (IVk, intermediate compound)

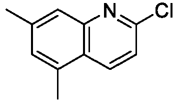

Title compound was prepared according to General Procedure 5 (Scheme 1). Yield: 57 % as a beige solid; mp 56-58°C. ¹H NMR (600 MHz, DMSO) δ 8.40 (d, *J =* 8.8 Hz, 1H), 7.53 (s, 1H), 7.46 (d, *J =* 8.7 Hz, 1H), 7.29 (s, 1H), 2.58 (s, 3H), 2.43 (s, 3H). ¹³C NMR (151 MHz, DMSO) δ 149.99, 148.58, 141.31, 137.07, 135.43, 130.27, 125.54, 124.65, 121.50, 21.85, 18.69. Elem. Anal. Calcd. for C₁₁H₁₀ClN: C, 68.94; H, 5.26; N, 7.31. Found: C, 69.05; H, 5.58; N, 7.19.

### EXAMPLE 30

### The mixture of 2-chloro-7-methylquinoline (IVl) a 2-chloro-5-methylquinoline (IVm), (intermediate compounds)

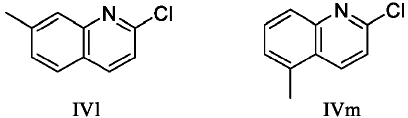

Title compounds were prepared according to General Procedure 5 (Scheme 1). The product was suspended in diethyl ether and filtered off. Total yield: 73 % as a beige solid as a mixture of 2,5- and 2,7-disubstituted quinolines in ratio 1:3. 2-Chloro-7-methylquinoline (IVl): ¹H NMR (600 MHz, DMSO) δ 8.34 (d, *J =* 8.6 Hz, 1H), 7.89 (d, *J =* 8.3 Hz, 1H), 7.70 (s, 1H), 7.46 (d, *J* = 8.6 Hz, 2H), 2.48 (s, 3H). 2-Chloro-5-methylquinoline (IVm): ¹H NMR (600 MHz, DMSO) δ 8.47 (d, *J =* 8.7 Hz, 1H), 7.75 (d, *J =* 8.4 Hz, 1H), 7.66 (dd, *J =* 8.5, 7.0 Hz, 1H), 7.55 (d, *J* = 8.7 Hz, 1H), 7.46-7.44 (m, 1H), 2.63 (s, 3H).

### EXAMPLE 31

### 2-Chloro-7-iodoquinoline (IVn, intermediate compound)

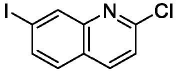

Title compound was prepared according to General Procedure 6 (Scheme 1). The product was obtained in the mixture with 2-chloro-5-iodoquinoline and was separated by column chromatography (mobile phase: hexane/EtOAc, 25:1). Yield: 31 % as a white solid; mp 113-114 °C. ¹H NMR (600 MHz, CDCl₃) δ 8.43 (s, 1H), 8.04 (d, *J =* 8.4 Hz, 1H), 7.80 (dd, *J =* 8.7, 1.7 Hz, 1H), 7.51 (d, *J =* 8.6 Hz, 1H), 7.38 (d, *J =* 8.7 Hz, 1H). ¹³C NMR (151 MHz, CDCl₃) δ 151.51, 148.43, 138.83, 137.71, 135.92, 128.66, 125.90, 123.07, 96.92. Elem. Anal. Calcd. for C₉H₅ClIN: C, 37.34; H, 1.74; N, 4.84. Found: C, 37.12; H, 1.69; N, 4.91.

### EXAMPLE 32

### 2-Chloro-5-iodoquinoline (IVo, intermediate compound)

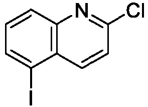

Title compound was prepared according to General Procedure 6 (Scheme 1). The product was obtained in the mixture with 2-chloro-7-iodoquinoline and was separated by column chromatography (mobile phase: hexane/EtOAc, 25:1). Yield: 11 % as a yellow solid; mp 85-87°C. ¹H NMR (600 MHz, CDCl₃) δ 8.32 (d, *J =* 8.7 Hz, 1H), 8.10 (d, *J =* 7.4 Hz, 1H), 8.00 (d, *J* = 8.4 Hz, 1H), 7.44 - 7.41 (m, 2H). ¹³C NMR (151 MHz, CDCl₃) δ 151.85, 148.11, 143.37, 138.23, 131.58, 129.68, 129.15, 124.05, 97.87. Elem. Anal. Calcd. for C₉H₅ClIN: C, 37.34; H, 1.74; N, 4.84. Found: C, 37.05; H, 1.92; N, 4.55.

### EXAMPLE 33

### 2-Chloro-7-nitroquinoline (IVp, intermediate compound)

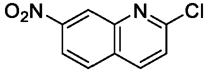

Title compound was prepared according to General Procedure 6 (Scheme 1). The reaction mixture was poured into ice water and the precipitation was filtered off. The product was obtained in high purity and used in the next reaction without additional purification. Yield: 82 % as a beige solid; mp 142-143 °C. ¹H NMR (600 MHz, DMSO) δ 8.67 (d, *J =* 2.3 Hz, 1H), 8.60 (d, *J* = 8.7 Hz, 1H), 8.33 (dd, *J* = 8.9, 2.3 Hz, 1H), 8.27 (d, *J* = 9.0 Hz, 1H), 7.79 (d, *J =* 8.6 Hz, 1H). ¹³C NMR (151 MHz, DMSO) δ 152.95, 149.01, 146.52, 140.64, 130.93, 130.65, 126.20, 124.04, 121.10. Elem. Anal. Calcd. for C₉H₅ClN₂O₂: C, 51.82; H, 2.42; N, 13.43. Found: C, 51.78; H, 2.27; N, 13.36.

### EXAMPLE 34

### 2-Chloro-5-nitroquinoline (IVq, intermediate compound)

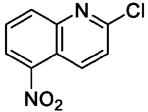

Title compound was prepared according to General Procedure 6 (Scheme 1). The reaction mixture was poured into ice water and the precipitation was filtered off. The product was obtained in high purity and used in the next reaction without additional purification. The yield: 95 % as a white solid; mp 129-130 °C (lit mp (Pedron et al., 2018) 134 °C). ¹H NMR (600 MHz, DMSO) δ 8.83 (d, *J* = 9.1 Hz, 1H), 8.48 - 8.35 (m, 1H), 8.31 (d, *J* = 8.2 Hz, 1H), 7.96 (t, *J* = 8.1 Hz, 1H), 7.82 (d, *J* = 9.1 Hz, 1H). ¹³C NMR (151 MHz, DMSO) δ 151.94, 147.77, 145.87, 136.09, 135.39, 130.37, 125.85, 125.75, 119.81. Elem. Anal. Calcd. for C₉H₅ClN₂O₂: C, 51.82; H, 2.42; N, 13.46. Found: C, 51.57; H, 2.18; N, 13.63.

### General Procedure 7: Synthesis of compounds of general formula I

The mixture of (R)-2-(4-hydroxyphenoxy)propanoic acid (0.14 g, 0.75 mmol) and potassium carbonate (0.21 g, 1.5 mmol) was stirred in DMF (7 ml) at rt for 30 min and then was heated to 75 °C for 2 hours. Substituted-2-chloroquinoline of general formula IV (0.5 mmol) was added and reaction mixture was heated to 145 °C for 2-10 hours. After reaction completion, the reaction mixture was poured into ice water (50 mL), acidified to pH 3-4 and extracted with EtOAc (3 × 30 mL). The organic layer was additionally washed with water (2 × 20 mL) and brine (1 × 20 mL), dried over anhydrous sodium sulfate and concentrated. The product was purified by column chromatography or suspended with diethyl ether and filtered off.

### General Procedure 8: Synthesis of compounds of general formula I

The mixture of (R)-2-(4-hydroxyphenoxy)propanoic acid (0.18 g, 1 mmol) and sodium hydroxide (0.09 g, 2.2 mmol) in mixture of DMSO (1 mL) and water (0.5 mL) was heated to 70 °C for 30 min. Then, a solution of substituted 2-chloroquinoline of general formula IV (1 mmol) in DMSO (2 mL) was added and the reaction mixture was heated to 100 °C for 1-12 hours. After reaction completion, the reaction mixture was poured into ice water (50 mL), acidified to pH 3-4 and extracted with EtOAc (3 × 30 mL). The organic layer was additionally washed with water (2 × 20 mL) and brine (1 × 20 mL), dried over anhydrous sodium sulfate and concentrated. The products were purified by column chromatography (mobile phase: Hex/EtOAc/CH₃COOH, 30:10:1).

### EXAMPLE 35

### (R)-2-(4-((7-Chloroquinolin-2-yl)oxy)phenoxy)propanoic acid

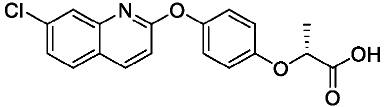

Title compound was prepared according to General Procedure 7 (Scheme 1). The reaction mixture was heated for 8 hours. The product was purified by column chromatography (mobile phase: hexane/EtOAc/CH₃COOH, 60:40: 1). Yield: 58 % as a white solid; mp 157-159 °C. ¹H NMR (600 MHz, DMSO) δ 13.02 (s, 1H), 8.38 (d, *J* = 8.9 Hz, 1H), 7.93 (d, *J =* 8.7 Hz, 1H), 7.64 (d, *J* = 2.2 Hz, 1H), 7.46 (dd, *J* = 2.1, 8.6 Hz, 1H), 7.21 (d, *J* = 8.8 Hz, 1H), 7.15-7.12 (m, 2H), 6.92-6.89 (m, 2H), 4.80 (q, *J* = 6.8 Hz, 1H), 1.49 (d, *J* = 6.8 Hz, 3H). ¹³C NMR (151 MHz, DMSO) δ 173.7, 163.0, 155.1, 147.2, 146.8, 140.9, 135.2, 130.2, 126.3, 125.9, 124.6, 123.2, 116.1, 113.8, 72.4, 18.9. Elem. Anal. Calcd. for C₁₈H₁₄ClNO₄: C, 62.89; H, 4.11; N, 4.07. Found: C, 62.69; H, 4.31; N, 3.95.

### EXAMPLE 36

### (R)-2-(4-((6-Chloroquinolin-2-yl)oxy)phenoxy)propanoic acid

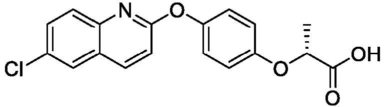

Title compound was prepared according to General Procedure 7 (Scheme 1). The reaction mixture was heated for 10 hours. The product was purified by column chromatography (mobile phase: hexane/EtOAc/CH₃COOH, 60:40:1). Yield: 41 % as a white solid; mp 110-112 °C. ¹H NMR (600 MHz, DMSO) δ 8.33 (d, *J =* 8.7 Hz, 1H), 8.03 (d, *J =* 2.2 Hz, 1H), 7.62-7.58 (m, 2H), 7.25 (d, *J =* 8.9 Hz, 1H), 7.14-7.12 (m, 2H), 6.91-6.88 (m, 2H), 4.80 (q, *J* = 6.8 Hz, 1H), 1.49 (d, *J* = 6.8 Hz, 3H).¹³C NMR (151 MHz, DMSO) δ 173.71, 162.56, 155.12, 147.21, 144.73, 140.20, 130.81, 129.52, 129.43, 127.09, 126.76, 123.20, 116.09, 114.52, 72.44, 18.90. Elem. Anal. Calcd. for C₁₈H₁₄ClNO₄: C, 62.89; H, 4.11; N, 4.07. Found: C, 62.51; H, 4.05; N, 4.06.

### EXAMPLE 37

### (R)-2-(4-((5-Chloroquinolin-2-yl)oxy)phenoxy)propanoic acid

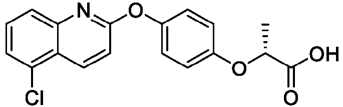

Title compound was prepared according to General Procedure 7 (Scheme 1). The Reaction mixture was heated for 8 hours. The product was purified by column chromatography (mobile phase: hexane/EtOAc/CH₃COOH, 60:40: 1). Yield: 43 % as a white solid; mp 152-154 °C. ¹H NMR (600 MHz, DMSO) δ 13.01 (s, 1H), 8.52 (d, *J* = 9.1 Hz, 1H), 7.59 (s, 3H), 7.33 (d, *J* = 9.1 Hz, 1H), 7.16-7.14 (m, 2H), 6.92-6.90 (m, 2H), 4.81 (q, *J* = 6.8 Hz, 1H), 1.49 (d, *J* = 6.8 Hz, 3H). ¹³C NMR (151 MHz, DMSO) δ 173.68, 162.79, 155.18, 147.24, 147.17, 137.08, 130.86, 130.79, 127.21, 125.64, 123.51, 123.19, 116.12, 114.76, 72.41, 18.89. Elem. Anal. Calcd. for C₁₈H₁₄ClNO₄: C, 62.89; H, 4.11; N, 4.07. Found: C, 62.88; H, 4.05; N, 3.95.

### EXAMPLE 38

### (R)-2-(4-((5,7-Dichloroquinolin-2-yl)oxy)phenoxy)propanoic acid

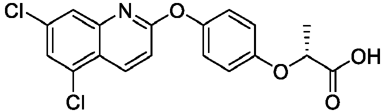

Title compound was prepared according to General Procedure 7 (Scheme 1). Reaction mixture was heated for 4 hours. The products were purified by column chromatography (mobile phase: hexane/EtOAc/CH₃COOH, 60:40:1). Yield: 56 % as a white solid; mp 135-137 °C. ¹H NMR (500 MHz, DMSO) δ 12.98 (s, 1H), 8.53 (dd, *J =* 9.1, 0.8 Hz, 1H), 7.76 (dd, *J =* 2.0, 0.5 Hz, 1H), 7.69 (dd, *J* = 2.0, 0.8 Hz, 1H), 7.38 (d, *J* = 9.1 Hz, 1H), 7.19 (d, *J =* 9.1 Hz, 2H), 6.94 (d, *J* = 9.0 Hz, 2H), 4.84 (q, *J* = 6.7 Hz, 1H), 1.53 (d, *J* = 6.8 Hz, 3H). ¹³C NMR (126 MHz, DMSO) δ 173.61, 163.52, 155.21, 147.27, 146.85, 137.07, 134.80, 132.02, 126.07, 125.67, 123.08, 122.25, 116.03, 115.04, 72.29, 18.82. Elem. Anal. Calcd. for C₁₈H₁₃Cl₂NO₄: C, 57.16; H, 3.46; N, 3.70. Found: C, 57.15; H, 3.36; N, 3.68.

### EXAMPLE 39

### (R)-2-(4-((7-Bromoquinolin-2-yl)oxy)phenoxy)propanoic acid

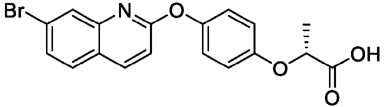

Title compound was prepared according to General Procedure 7 (Scheme 1). The reaction mixture was heated for 10 hours. The product was purified by column chromatography (mobile phase: hexane/EtOAc, 1:1). Yield: 49 % as a white solid; mp 164-166 °C. ¹H NMR (600 MHz, DMSO) δ 13.01 (s, 1H), 8.37 (d, *J =* 8.2 Hz, 1H), 7.86 (d, *J =* 8.6 Hz, 1H), 7.79 (d, *J =* 2.0 Hz, 1H), 7.58 (dd, *J =* 2.0, 8.6 Hz, 1H), 7.23 (d, *J =* 8.9 Hz, 1 H), 7.15-7.12 (m, 2H), 6.92-6.88 (m, 2H), 4.80 (q, *J =* 6.8 Hz, 1H), 1.49 (d, *J =* 6.8 Hz, 3H). ¹³C NMR (151 MHz, DMSO) δ 173.7, 162.9, 155.1, 147.2, 147.0, 140.9, 130.2, 129.5, 128.4, 124.8, 123.9, 123.2, 116.1, 113.9, 72.4, 18.9. Elem. Anal. Calcd. for C₁₈H₁₄BrNO₄: C, 55.69; H, 3.64; N, 3.61. Found: C, 55.68; H, 3.56; N 3.49.

### EXAMPLE 40

### (R)-2-(4-((5-Bromoquinolin-2-yl)oxy)phenoxy)propanoic acid

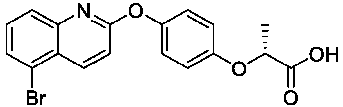

Title compound was prepared according to General Procedure 7 (Scheme 1). The reaction mixture was heated for 10 hours. The product was purified by column chromatography (mobile phase: hexane/EtOAc/CH₃COOH, 60:40:1). Yield: 44 % as a white solid; mp 161-163 °C. ¹H NMR (500 MHz, DMSO) δ 13.09 (s, 1H), 8.49 (dd, *J* = 0.8, 9.1 Hz, 1H), 7.80 (dd, *J* = 1.1, 7.6 Hz, 1H), 7.66 (d, J = 8.4 Hz, 1H), 7.57-7.53 (m, 1H), 7.36 (d, *J* = 9.0 Hz, 1H), 7.20-7.17 (m, 2H), 6.96-6.92 (m, 2H), 4.84 (q, *J* = 6.8 Hz, 1H), 1.53 (d, *J* = 6.8 Hz, 3H). ¹³C NMR (126 MHz, DMSO) δ 173.6, 162.7, 155.1, 147.1, 147.1, 139.5, 131.3, 129.2, 127.8, 124.8, 123.1, 121.4, 116.0, 114.9, 72.3, 18.8. Elem. Anal. Calcd. for C₁₈H₁₄BrNO₄: C, 55.69; H, 3.64; N, 3.61. Found: C, 55.44; H, 3.44; N, 3.53.

### EXAMPLE 41

### (R)-2-(4-((7-Fluoroquinolin-2-yl)oxy)phenoxy)propanoic acid

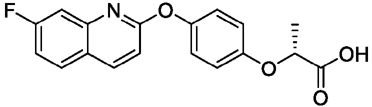

Title compound was prepared according to General Procedure 8 (Scheme 1). The reaction mixture was heated for 8 hours. The product was purified by column chromatography (mobile phase: hexane/EtOAc/CH₃COOH, 60:40:1). Yield: 35 % as a white solid; mp 129-130 °C. ¹H NMR (600 MHz, DMSO) δ 13.02 (s, 1H), 8.37 (dd, *J* = 0.7, 8.9 Hz, 1H), 7.97 (dd, *J* = 6.3, 8.8 Hz, 1H), 7.37-7.32 (m, 2H), 7.17-7.12 (m, 3H), 6.92-6.89 (m, 2H), 4.80 (q, *J* = 6.8 Hz, 1H), 1.49 (d, *J* = 6.8 Hz, 3H). ¹³C NMR (151 MHz, DMSO). δ ¹³C NMR (151 MHz, DMSO) δ 173.71, 163.48 (d, *J* = 246.4 Hz), 163.13, 155.11, 147.45 (d, *J* = 13.2 Hz), 147.27, 140.90, 130.80 (d, *J* = 10.4 Hz), 123.20, 123.09, 116.10, 115.01 (d, *J* = 24.6 Hz), 112.64 (d, *J* = 2.7 Hz), 111.56 (d, *J* = 20.9 Hz), 72.41, 18.89.

### EXAMPLE 42

### (R)-2-(4-((7-(Trifluoromethyl)quinolin-2-yl)oxy)phenoxy)propanoic acid

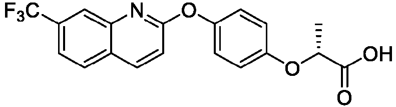

Title compound was prepared according to General Procedure 8 (Scheme 1). The reaction mixture was heated for 8 hours. The product was purified by column chromatography (mobile phase: hexane/EtOAc/CH₃COOH, 65:35: 1). Yield: 47 % as a white solid; mp 122-123 °C. ¹H NMR (500 MHz, DMSO) δ 13.03 (s, 1H), 8.51 (d, *J* = 8.9 Hz, 1H), 8.17 (d, *J* = 8.4 Hz, 1H), 7.91 (s, 1H), 7.73 (dd, *J* = 1.9, 8.5 Hz, 1H), 7.41 (d, *J* = 8.9 Hz, 1H), 7.21-7.18 (m, 2H), 6.96-6.93 (m, 2H), 4.84 (q, *J* = 6.7 Hz, 1H), 1.53 (d, *J* = 6.7 Hz, 3H). ¹³C NMR (151 MHz, DMSO) δ 173.68, 163.28, 155.22, 147.07, 145.34, 140.92, 130.59 (d, *J =* 31.8 Hz), 130.17, 127.91, 124.73 (d, *J* = 4.4 Hz), 124.53 (d, *J* = 272.4 Hz), 123.20, 120.71 (d, *J* = 3.8 Hz), 116.12, 115.96, 72.44, 18.88.

### EXAMPLE 43

### (R)-2-(4-((5-(Trifluoromethyl)quinolin-2-yl)oxy)phenoxy)propanoic acid

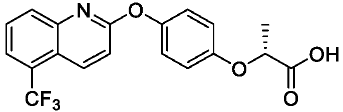

Title compound was prepared according to General Procedure 8 (Scheme 1). The reaction mixture was heated for 6 hours. The product was purified by column chromatography (mobile phase: hexane/EtOAc/CH₃COOH, 65:35: 1). Yield: 54 % as a white solid; mp 150-151 °C. ¹H NMR (600 MHz, DMSO) δ 12.98 (s, 1H), 8.44 (dd, *J* = 2.1, 9.3 Hz, 1H), 7.88 (dd, *J =* 7.9, 14.6 Hz, 2H), 7.73 (t, *J* = 7.9 Hz, 1H), 7.40 (d, *J* = 9.2 Hz, 1H), 7.18-7.15 (m, 2H), 6.93-6.90 (m, 2H), 4.81 (q, *J =* 6.8 Hz, 1H), 1.50 (d, *J =* 6.7 Hz, 3H). ¹³C NMR (151 MHz, DMSO) δ 173.68, 162.56, 155.24, 147.04, 146.93, 136.35, 132.94, 129.60, 125.24 (q, *J* = 30.0 Hz), 124.71 (q, *J =* 273.5 Hz), 124.00 (d, *J =* 5.8 Hz), 123.19, 121.33, 116.14, 115.39, 72.41, 18.87.

### EXAMPLE 44

### (R)-2-(4-((7-Ethylquinolin-2-yl)oxy)phenoxy)propanoic acid

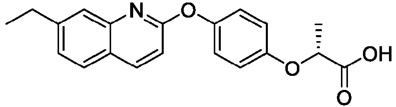

Title compound was prepared according to General Procedure 8 (Scheme 1). The reaction mixture was heated for 6 hours. The product was purified by column chromatography (mobile phase: hexane/EtOAc/CH₃COOH, 30:10:1). Yield: 27 % as a light yellow solid; mp 135-137 °C. ¹H NMR (500 MHz, DMSO) δ 12.68 (s, 1H), 8.49 (d, *J =* 8.69 Hz, 1H), 7.82 (d, *J =* 8.2 Hz, 1H), 7.44 (s, 1H), 7.33 (dd, *J =* 1.7, 8.2 Hz, 1H), 7.17-7.13 (m, 3H), 6.95-6.91 (m, 2H), 4.83 (q, *J* = 6.8 Hz, 1H), 2.73 (q, *J* = 7.6 Hz, 2H), 1.53 (d, *J* = 6.8 Hz, 3H), 1.21 (t, *J* = 7.6 Hz, 3H). ¹³C NMR (126 MHz, DMSO) δ 173.63, 162.20, 154.87, 147.44, 146.62, 146.39, 140.42, 128.00, 126.15, 125.44, 124.04, 123.14, 115.96, 112.25, 72.35, 28.72, 18.83, 15.77. Elem. Anal. Calcd. for C₁₈H₁₄ClNO₄: C, 62.89; H, 4.11; N, 4.07. Found: C, 62.88; H, 4.05; N, 3.95.

### EXAMPLE 45

### (R)-2-(4-((5,7-Dimethylquinolin-2-yl)oxy)phenoxy)propanoic acid

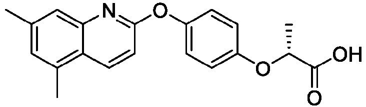

Title compound was prepared according to General Procedure 8 (Scheme 1). The reaction mixture was heated for 10 hours. The product was purified by column chromatography (mobile phase: hexane/EtOAc/CH₃COOH, 60:40:1). Yield: 22 % as a yellow solid; mp 119-120 °C. ¹H NMR (600 MHz, DMSO) δ 12.95 (s, 1H), 8.35 (dd, *J* = 8.9 Hz, 1H), 7.25 (s, 1H), 7.11-7.08 (m, 4H), 6.90-6.88 (m, 2H), 4.79 (q, *J =* 6.8 Hz, 1H), 2.55 (s, 3H), 2.34 (s, 3H), 1.49 (d, *J =* 6.7 Hz, 3H). ¹³C NMR (151 MHz, DMSO) δ 173.74, 162.00, 154.92, 147.59, 146.91, 140.13, 137.30, 134.89, 127.96, 125.11, 123.12, 116.03, 111.61, 72.47, 21.67, 18.91, 18.81. Elem. Anal. Calcd. for C₂₀H₁₉NO₄: C, 71.20; H, 5.68; N, 4.15. Found: C, 70.92; H, 5.35; N, 4.01.

### EXAMPLE 46

### (R)-2-(4-((7-methylquinolin-2-yl)oxy)phenoxy)propanoic acid

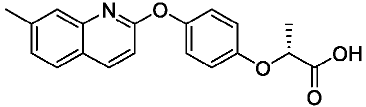

Title compound was prepared according to General Procedure 8 (Scheme 1). The reaction mixture was heated at 100 °C for 12 hours. The product was purified by column chromatography (mobile phase: hexane/EtOAc/CH₃COOH, 30:10:1). Yield: 48 %. ¹H NMR (500 MHz, DMSO) δ 13.0 (s, 1H), 8.30 (d, *J* = 8.8 Hz, 1H), 7.80 (d, *J* = 8.2 Hz, 1H), 7.44 (s, 1H), 7.29 (dd, *J =* 8.2 Hz, 1.7 Hz, 1H), 7.19 - 7.07 (m, 3H), 6.97-6.89 (m, 2H), 4.83 (q, *J =* 6.8 Hz, 1H), 2.42 (s, 3H), 1.53 (d, *J =* 6.8 Hz, 3H). ¹³C NMR (125 MHz, DMSO) δ 173.4, 161.9, 154.6, 147.1, 146.0, 140.2, 140.1, 127.6, 126.9, 126.5, 123.5, 122.8, 115.7, 111.9, 72.1, 21.4, 18.6.

### EXAMPLE 47

### (R)-2-(4-((5-methylquinolin-2-yl)oxy)phenoxy)propanoic acid

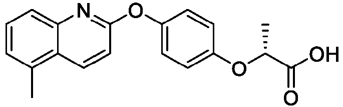

Title compound was prepared according to General Procedure 8 (Scheme 1). The reaction mixture was heated at 100 °C for 12 hours. The product was purified by column chromatography (mobile phase: hexane/EtOAc/CH₃COOH, 30:10:1). Yield: 10 %. ¹H NMR (500 MHz, DMSO) δ 13.0 (s, 1H), 8.46 (d, *J* = 9.0 Hz, 1H), 7.51 (dd, *J* = 8.4 Hz, 6.8 Hz, 1H), 7.46 (d, *J =* 8.4 Hz, 1H), 7.29 (d, *J =* 8.4 Hz, 1H), 7.21 (d, *J =* 9.0 Hz, 1H), 7.19 - 7.07 (m, 2H), 6.97 - 6.89 (m, 2H), 4.83 (q, *J =* 6.8 Hz, 1H), 2.63 (s, 3H), 1.53 (d, *J =* 6.8 Hz, 3H). ¹³C NMR (125 MHz, DMSO) δ 173.4, 161.5, 154.6, 147.2, 146.2, 137.2, 135.0, 129.9, 125.6, 125.5, 124.7, 122.8, 115.7, 112.3, 72.1, 18.6, 18.6.

### EXAMPLE 48

### (R)-2-(4-((7-iodoquinolin-2-yl)oxy)phenoxy)propanoic acid

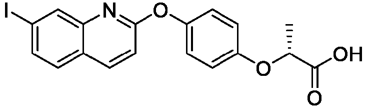

Title compound was prepared according to General Procedure 8 (Scheme 1). The product was purified by column chromatography (mobile phase: hexane/EtOAc/CH₃COOH, 50:10:2). Yield: 71 % as a white solid; mp 150-152 °C. ¹H NMR (600 MHz, DMSO) δ 8.34 (dd, *J* = 8.9, 0.8 Hz, 1H), 7.97 (dd, *J* = 1.6, 0.8 Hz, 1H), 7.72 (dd, *J* = 8.5*,* 1.7 Hz, 1H), 7.68 (d, *J =* 8.4 Hz, 1H), 7.21 (d, *J =* 8.8 Hz, 1H), 7.13 (d, *J =* 9.0 Hz, 2H), 6.90 (d, *J =* 9.0 Hz, 2H), 4.80 (q, *J = 6.8* Hz, 1H), 1.49 (d, *J =* 6.8 Hz, 3H). ¹³C NMR (151 MHz, DMSO) δ 173.71, 162.58, 155.13, 147.19, 147.05, 140.98, 135.81, 133.77, 129.92, 125.02, 123.16, 116.08, 114.00, 97.42, 72.48, 18.90. Elem. Anal. Calcd. for C₁₈H₁₄INO₄: C, 49.68; H, 3.24; N, 3.22. Found: C, 49.82; H, 3.44; N, 3.02.

### EXAMPLE 49

### (R)-2-(4-((5-iodoquinolin-2-yl)oxy)phenoxy)propanoic acid

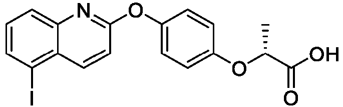

Title compound was prepared according to General Procedure 8 (Scheme 1). The product was purified by column chromatography (mobile phase: hexane/EtOAc/CH₃COOH, 50:10:2). Yield: 65 % as a white solid; mp 172-174 °C. ¹H NMR (600 MHz, DMSO) δ 8.32 (d, *J* = 9.2 Hz, 1H), 8.01 (dd, *J =* 7.4, 1.2 Hz, 1H), 7.65 - 7.55 (m, 1H), 7.35 (dd, *J =* 8.5, 7.4 Hz, 1H), 7.28 (d, *J* = 9.1 Hz, 1H), 7.14 (d, *J* = 9.0 Hz, 2H), 6.91 (d, *J* = 9.0 Hz, 2H), 4.81 (q, *J* = 6.9 Hz, 1H), 1.49 (d, *J =* 6.8 Hz, 3H). ¹³C NMR (151 MHz, DMSO) δ 173.69, 162.84, 155.15, 147.28, 146.62, 144.41, 136.37, 131.87, 128.63, 127.63, 123.14, 116.12, 115.27, 99.05, 72.42, 18.90. Elem. Anal. Calcd. for C₁₈H₁₄INO₄: C, 49.68; H, 3.24; N, 3.22. Found: C, 49.78; H, 3.16; N, 3.1.

### EXAMPLE 50

### (R)-2-(4-((7-nitroquinolin-2-yl)oxy)phenoxy)propanoic acid

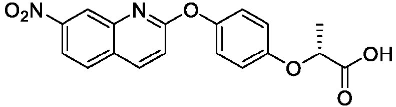

Title compound was prepared according to General Procedure 8 (Scheme 1). Yield: 83 % as a white solid; mp 168-169 °C. ¹H NMR (600 MHz, DMSO) δ 13.01 (s, 1H), 8.53 (dd, *J =* 9.0, 0.8 Hz, 1H), 8.30 (d, *J =* 0.6 Hz, 1H), 8.16 (d, *J =* 1.5 Hz, 2H), 7.44 (d, *J =* 8.9 Hz, 1H), 7.18 (d, *J* = 9.0 Hz, 2H), 6.93 (d, *J* = 9.0 Hz, 2H), 4.82 (q, *J* = 6.8 Hz, 1H), 1.50 (d, *J* = 6.8 Hz, 3H). ¹³C NMR (151 MHz, DMSO) δ 173.67, 163.74, 155.32, 148.60, 146.90, 145.31, 140.91, 130.36, 129.62, 123.21, 122.78, 118.75, 117.01, 116.15, 72.46, 18.88. Elem. Anal. Calcd. for C₁₈H₁₄N₂O₆: C, 61.02; H, 3.98; N, 7.91. Found: C, 59.82; H, 4.16; N, 8.08.

### EXAMPLE 51

### (R)-2-(4-((5-nitroquinolin-2-yl)oxy)phenoxy)propanoic acid

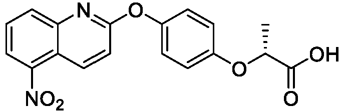

Title compound was prepared according to General Procedure 8 (Scheme 1). The reaction mixture was heated at 100 °C for 1 hour. Yield: 42 % as a white solid; mp 155-157 °C. ¹H NMR (600 MHz, DMSO) δ 13.03 (s, 1H), 8.78 (dd, *J* = 9.3, 0.8 Hz, 1H), 8.22 (dd, *J* = 7.7, 1.1 Hz, 1H), 7.98 (d, *J* = 8.4 Hz, 1H), 7.77 (dd, *J =* 8.4, 7.8 Hz, 1H), 7.47 (d, *J* = 9.4 Hz, 1H), 7.17 (d, *J* = 9.0 Hz, 2H), 6.92 (d, *J =* 9.0 Hz, 2H), 4.81 (q, *J =* 6.8 Hz, 1H), 1.50 (d, *J =* 6.8 Hz, 3H). ¹³C NMR (151 MHz, DMSO) δ 173.67, 162.78, 155.31, 146.90, 146.82, 146.13, 136.21, 134.32, 129.49, 123.21, 123.04, 118.13, 116.53, 116.16, 72.41, 18.88. Elem. Anal. Calcd. for C₁₈H₁₄N₂O₆: C, 61.02; H, 3.98; N, 7.91. Found: C, 61.31; H, 3.72; N, 7.98.

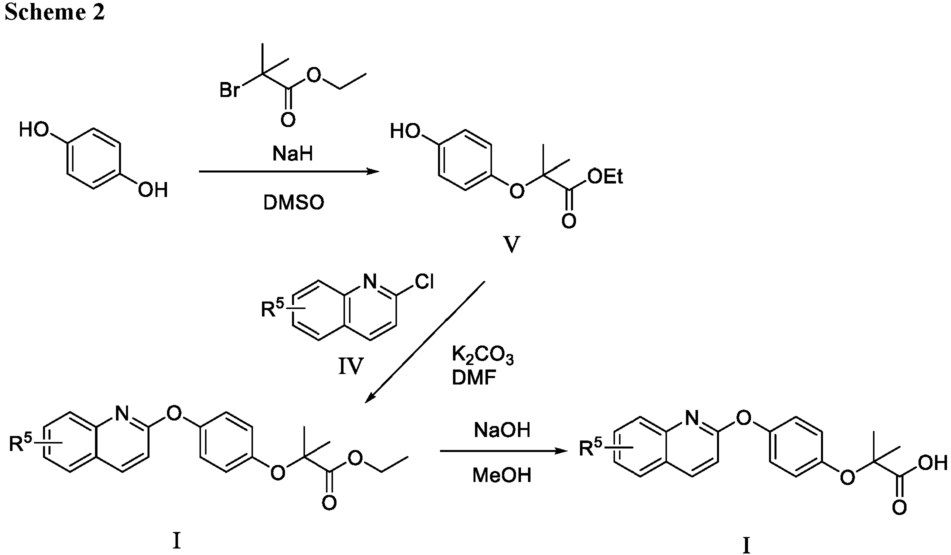

### EXAMPLE 52

### Ethyl 2-(4-hydroxyphenoxy)-2-methylpropanoate (V, intermediate compound)

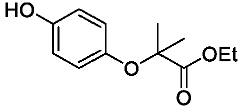

Title compound was prepared according to Scheme 2. A solution of hydroquinone (1.0 g, 9.0 mmol) and sodium hydride (60% dispersion in mineral oil) (0.44 g, 10.8 mmol) in DMSO (10 mL) was heated to 80 °C for 2 hours. After cooling, ethyl 2-bromo-2-methylpropanoate (1.77 g/1.4 mL, 9.0 mmol) was added dropwise and the reaction mixture was stirred at rt for 4 hours. Then it was poured into water (70 mL), acidified to pH 2 and extracted with EtOAc (3 × 50 mL). The organic layer was washed with water (2 × 50 mL) and brine (1 × 50 mL), dried over anhydrous sodium sulfate, and concentrated under reduced pressure. The product was purified by column chromatography (mobile phase: hexane/EtOAc, 4:1). Ethyl 2-(4-hydroxyphenoxy)-2-methylpropanoate was obtained in 32 % the yield as a white solid; mp 82-83 °C (lit mp (Giampietro et al., 2019b) 85-86 °C). ¹H NMR (500 MHz, DMSO) δ 9.09 (s, 1H), 6.68 - 6.66 (m, 2H), 6.64 - 6.62 (m, 2H), 4.14 (q, *J* = 7.1 Hz, 2H), 1.41 (s, 6H), 1.19 (t, *J* = 7.1 Hz, 3H). ¹³C NMR (126 MHz, DMSO) δ 173.46, 153.09, 147.25, 121.85, 115.58, 79.29, 60.94, 25.09, 14.10. Elem. Anal. Calcd. for C₁₂H₁₆O₄: C, 64.27; H, 7.19. Found: C, 64.12; H, 7.28.

### General procedure 9: Synthesis of esters of general formula I

A mixture of ethyl 2-(4-hydroxyphenoxy)-2-methylpropanoate V (0.17 g, 1.2 mmol) and potassium carbonate (0.28 g, 1.2 mmol) in DMF (10 mL) was stirred at rt for 30 min and then was heated to 75 °C for 2 hours. Substituted-2-chloroquinoline of formula IV (0.4 mmol) was added and the reaction mixture was heated to 145 °C for 12 hours. After cooling, reaction mixture was poured into ice water (70 mL), acidified to pH 2 and extracted with EtOAc (3 × 30 mL). The organic layer was washed with water (2 × 20 mL) and brine (1 × 20 mL), dried over anhydrous sodium sulfate, and concentrated under reduced pressure. The product was purified by column chromatography (mobile phase: hexane/EtOAc, 10:1).

### General procedure 10: Synthesis of compounds of general formula I

0.1 M Aqueous solution NaOH (24 mL) was added dropwise to a solution of ethyl 2-(4-((substituted-quinolin-2-yl)oxy)phenoxy)-2-methylpropanoate of formula VI (1.6 mmol) in MeOH (10 mL) and refluxed for 3 hours. After cooling, reaction mixture was concentrated under reduced pressure. The residue was dissolved in water (50 mL), acidified to pH 2 and extracted with EtOAc. The organic layer was washed with water (2 × 20 mL) and brine (1 × 20 mL), dried over anhydrous sodium sulfate, and concentrated under reduced pressure. The product was suspended with diethyl ether (15 mL) and filtered off.

### EXAMPLE 53

### Ethyl 2-(4-((7-chloroquinolin-2-yl)oxy)phenoxy)-2-methylpropanoate

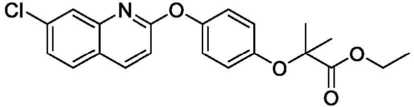

Title compound was prepared according to General Procedure 9 (Scheme 2). Yield: 59 % as a colorless oil. ¹H NMR (600 MHz, CDCl₃) δ 8.05 (dd, *J =* 0.8, 8.9 Hz, 1H), 7.75 (dt, *J =* 0.6, 2.1 Hz, 1H), 7.66 (d, *J =* 8.6 Hz, 1H), 7.35 (dd, *J =* 2.1, 8.6 Hz, 1H), 7.11-7.09 (m, 2H), 7.03 (d, *J =* 8.8 Hz, 1H), 6.92-6.89 (m, 2H), 4.25 (q, *J* = 7.1 Hz, 2H), 1.62 (s, 6H), 1.28 (t, *J* = 7.1 Hz, 3H). ¹³C NMR (151 MHz, CDCl₃) δ 174.32, 162.63, 152.49, 148.31, 147.08, 139.46, 135.76, 128.51, 127.07, 125.73, 124.03, 122.17, 120.53, 112.86, 79.65, 61.53, 25.51, 14.20.

### EXAMPLE 54

### Ethyl 2-(4-((7-bromoquinolin-2-yl)oxy)phenoxy)-2-methylpropanoate

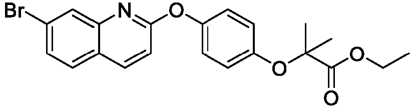

Title compound was prepared according to General Procedure 9 (Scheme 2). Yield: 40 % as a colorless oil. ¹H NMR (500 MHz, CDCl₃) δ 8.07 (d, *J* = 8.8 Hz, 1H), 7.95 (d, *J* = 1.9 Hz, 1H), 7.61 (d, *J* = 8.6 Hz, 1H), 7.50 (dd, *J =* 1.9, 8.6 Hz, 1H), 7.14-7.11 (m, 2H), 7.07 (d, *J =* 8.8 Hz, 1H), 6.95-6.92 (m, 2H), 4.28 (q, *J =* 7.1 Hz, 2H), 1.64 (s, 6H), 1.30 (t, *J =* 7.1 Hz, 3H). ¹³C NMR (126 MHz, CDCl₃) δ 174.20, 162.42, 152.39, 148.17, 147.15, 139.43, 130.22, 128.47, 128.16, 124.18, 123.89, 122.07, 120.42, 112.98, 79.54, 61.43, 25.41, 14.11.

### EXAMPLE 55

### 2-(4-((7-Chloroquinolin-2-yl)oxy)phenoxy)-2-methylpropanoic acid

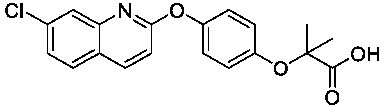

Title compound was prepared according to General Procedure 10 (Scheme 2). Yield: 82 % as a light beige solid; mp 189-191 °C. ¹H NMR (600 MHz, DMSO) δ 8.39 (dd, *J =* 0.8, 8.9 Hz, 1H), 7.94 (d, *J =* 8.6 Hz, 1H), 7.64 (d, *J* = 2.1 Hz, 1H), 7.47 (dd, *J* = 2.1, 8.6 Hz, 1H), 7.21 (d, *J* = 8.8 Hz, 1H), 7.14-7.11 (m, 2H), 6.88-6.86 (m, 2H), 1.50 (s, 6H). ¹³C NMR (151 MHz, DMSO) δ 175.51, 162.90, 152.85, 147.81, 146.77, 140.95, 135.20, 130.20, 126.30, 125.90, 124.58, 122.86, 120.11, 113.78, 79.24, 25.62. Elem. Anal. Calcd. for C₁₉H₁₆ClNO₄: C, 65.37; H, 5.22; N, 3.63. Found: C, 65.45; H, 5.31; N, 3.41.

### EXAMPLE 56

### 2-(4-((7-Bromoquinolin-2-yl)oxy)phenoxy)-2-methylpropanoic acid

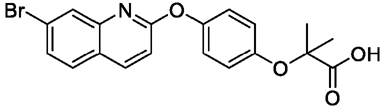

Title compound was prepared according to General Procedure 10 (Scheme 2). Yield: 78 % as a light beige solid; mp 144-146 °C. ¹H NMR (500 MHz, DMSO) δ 13.06 (s, 1H), 8.41 (d, *J* = 9.00 Hz, 1H), 7.90 (d, *J =* 8.6 Hz, 1H), 7.83 (d, *J* = 2.0 Hz, 1H), 7.62 (dd, *J* = 2.0, 8.6 Hz, 1H), 7.26 (d, *J* = 8.8 Hz, 1H), 7.18-7.15 (m, 2H), 6.92-6.89 (m, 2H), 1.54 (s, 6H). ¹³C NMR (126 MHz, DMSO) δ 175.11, 161.90, 151.47, 147.51, 146.27, 141.15, 135.47, 130.08, 126.31, 125.10, 124.78, 122.26, 120.11, 113.92, 79.46, 26.11. Elem. Anal. Calcd. for C₁₉H₁₆BrNO₄: C, 56.73; H, 4.01; N 3.48. Found: C, 56.68; H, 3.98; N, 3.25.

### EXAMPLE 57

### Synthesis of 3-chloroquinoxalin-6-amine (VIIa, intermediate compound)

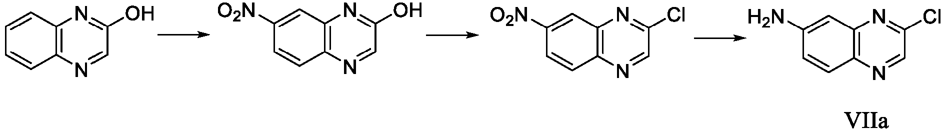

A solution of 65 % nitric acid (3.3 g/2.4 mL, 34 mmol) in acetic acid (10 mL) was added dropwise to solution of quinoxalin-2-ol (5.0 g, 34 mmol) in acetic acid (40 mL). The reaction mixture was stirred at rt for 18 hours. The formed precipitation was filtered off, the filtrate cake was washed with water (80 mL) and dried over P₂O₅. 7-Nitroquinoxalin-2-ol was obtained in 83 % yield as a light yellow solid; mp 277-279 °C. ¹H NMR (600 MHz, DMSO) δ 12.69 (s, 1H), 8.31 (s, 1H), 8.06 (d, *J* = 2.5 Hz, 1H), 8.03 (dd, *J* = 2.5, 8.8 Hz, 1H), 7.97 (d, *J* = 8.8 Hz, 1H). ¹³C NMR (151 MHz, DMSO) δ 156.07, 155.08, 148.14, 135.84, 132.88, 130.76, 118.00, 111.74. Elem. Anal. Calcd. for C₈H₅N₃O₃: C, 50.27; H, 2.64; N 21.98. Found: C, 50.57; H 2.61; N 21.83.

7-Nitroquinoxalin-2-ol (4.9 g, 25 mmol) was dissolved in POCl₃ (40 mL) and DMF (catalytic amount) was added. The reaction mixture was stirred for 3 hours at 110 °C and after cooling poured into ice. The formed precipitation was filtered off, the filtrate cake was washed with water (100 mL) and dried over P₂O₅. 2-Chloro-7-nitroquinoxaline: Yield: 94 % as a light yellow solid; mp 184-186 °C. ¹H NMR (600 MHz, DMSO) δ 9.17 (s, 1H), 8.82 (d, *J* = 2.5 Hz, 1H), 8.54 (dd, *J* = 2.5, 9.1 Hz, 1H), 8.35 (d, *J* = 9.1 Hz, 1H). ¹³C NMR (151 MHz, DMSO) δ 149.79, 149.45, 148.90, 143.45, 140.83, 131.55, 124.71, 124.49. Elem. Anal. Calcd. for C₈H₄ClN₃O₂: C, 45.85; H, 1.92; N 20.05. Found: C, 45.54; H, 1.89; N 19.86.

To a solution of 2-chloro-7-nitroquinoxaline (4.43 g, 21.1 mmol) in EtOAc (70 mL) was added stannous chloride dihydrate (16.7 g, 74 mmol) and the reaction mixture was refluxed for 2 hours. After cooling to rt, 50 % aqueous solution NaOH (50 mL) was added to the reaction mixture dropwise at 0 °C and the reaction mixture filtered on a pad of silica gel, then eluted with hot acetone. The filtrate was concentrated and product was purified by column chromatography (mobile phase: hexane/EtOAc 2.5:1) to give 3-chloroquinoxalin-6-amine VIIa as a yellow solid in 57 % yield; mp 206-208 °C. ¹H NMR (600 MHz, DMSO) δ 8.38 (s, 1H), 7.72 (d, *J* = 9.0 Hz, 1H), 7.21 (dd, *J* = 2.5, 9.0 Hz, 1H), 6.77 (d, *J =* 2.4 Hz, 1H), 6.30 (s, 2H). ¹³C NMR (151 MHz, DMSO) δ 152.55, 147.08, 144.84, 138.45, 135.25, 130.20, 123.01, 104.33. Elem. Anal. Calcd. for C₈H₆ClN₃: C, 53.50; H, 3.37; N 23.40. Found: C, 53.43; H, 3.34; N 23.20.

### EXAMPLE 58

### 2-Chloro-7-iodoquinoxaline (VIIb, intermediate compound)

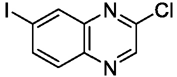

3-Chloroquinoxalin-6-amine VIIa (0.5 g, 2.8 mmol) was added to a solution of *p-*toluenesulfonic acid monohydrate (1.6 g, 8.4 mmol) in MeCN (20 mL) and the reaction mixture was cooled to 10 °C. The solution of sodium nitrite (0.39 g, 5.6 mmol) and potassium iodide (1.16 g, 7.0 mmol) in water (10 mL) was added dropwise. The reaction mixture was stirred at 10 °C for 10 min, and then 2 hours at rt. The reaction mixture was diluted with water (70 mL) and 1M NaHCO₃ was dropped to achieve pH = 10, then 2M Na₂S₂O₃ (6 mL) was added. The reaction mixture was extracted with EtOAc (3 × 50 mL). The organic layer was then washed with water (1 × 30 mL) and brine (1 × 30 mL), dried over anhydrous sodium sulfate and concentrated. The product was purified by column chromatography (mobile phase: hexane/CHCl₃ 1:1) to give 2-chloro-7-iodoquinoxaline in 54 % yield as a white solid; mp 146-147 °C. ¹H NMR (500 MHz, DMSO-*D₆*) δ 9.01 (s, 1H), 8.47 (d, *J* = 1.9 Hz, 1H), 8.16 (dd, *J* = 1.9, 8.7 Hz, 1H), 7.90 (d, *J* = 8.7 Hz, 1H). ¹³C NMR (126 MHz, DMSO-*D₆*) δ 147.62, 146.15, 142.09, 139.86, 139.38, 136.69, 130.61, 98.96. Elem. Anal. Calcd. for C₈H₄ClIN₂: C, 33.08; H, 1.39; N 9.64. Found: C, 33.34; H, 1.58; N 9.37.

### EXAMPLE 59

### N-(3-chloroquinoxalin-6-yl)acetamide (VIIc, intermediate compound)

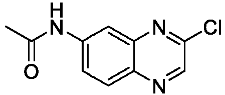

A solution of acetyl chloride (0.21 g/0.19 mL, 2.6 mmol) in CHCl₃ (5 mL) was added dropwise to a solution of 3-chloroquinoxalin-6-amine VIIa (0.24 g, 1.3 mmol) and sodium carbonate (0.43 g, 3.9 mmol) in CHCl₃ (20 mL). The mixture was heated to 50 °C for 4 hours. After cooling, reaction mixture was concentrated under reduced pressure. The product was suspended with diethyl ether (15 mL), filtered off, and dried over P₂O₅. *N*-(3-Chloroquinoxalin-6-yl)acetamide: yield: 80 % as a light beige solid; mp 204-205 °C. ¹H NMR (600 MHz, DMSO-*D₆*) 10.50 (s, 1H), 8.78 (s, 1H), 8.36 (d, *J =* 2.3 Hz, 1H), 8.02 (d, *J =* 9.0 Hz, 1H), 7.88 (dd, *J =* 2.3, 9.1 Hz, 1H), 2.11 (s, 3H). ¹³C NMR (151 MHz, DMSO-*D₆*) δ 169.87, 147.72, 143.62, 142.83, 142.29, 137.77, 129.98, 124.33, 114.54, 24.78. Elem. Anal. Calcd. for C₁₀H₈ClN₃O: C, 54.19; H, 3.64; N 18.96. Found: C, 54.4; H, 3.48; N 18.78.

### EXAMPLE 60

### 2,7-dichloroquinoxaline (VIId, intermediate compound)

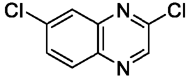

A mixture of glyoxylic acid (3,87 g, 42 mmol) and 4-chlorophenyl-1,2-diamine (3 g, 21 mmol) in DMF (25 mL) was heated to 70 °C for 16 hours. The reaction mixture was poured into ice water (150 mL) and the precipitation was filtered off. The filtrate cake was washed with EtOAc (10 mL). The product is a mixture of 7-chloroquinoxalin-2(1*H*)-on and 6-chloroquinoxalin-2(1*H*)-on in ratio 1:2. The mixture was used in the next step without purification. Total yield: 3 g (79 %). 7-Chloroquinoxalin-2(1*H*)-on: ¹H NMR (600 MHz, DMSO-*D₆*) 12.43 (s, 1H), 8.12 (s, 1H), 7.73 (d, *J* = 8.6 Hz, 1H), 7.32-7.22 (m, 2H). 6-Chloroquinoxalin-2(1*H*)-on [29]: ¹H NMR (600 MHz, DMSO) 12.48 (s, 1H), δ 8.16 (s, 1H), 7.78 (d, *J =* 2.4 Hz, 1H), 7.55 (dd, *J =* 8.7, 2.4 Hz, 1H), 7.32-7.22 (m, 1H). ¹³C NMR (151 MHz, DMSO) δ 155.18, 153.62, 152.54, 135.38, 133.51, 133.07, 131.40, 131.31, 131.16, 130.99, 128.32, 127.40, 123.88, 117.89, 115.49.

Phosphoryl chloride (5.1 g/3.1 mL, 33.2 mmol) was added to a solution of 7-chloroquinoxalin-2(1*H*)-on and 6-chloroquinoxalin-2(1*H*)-on (3 g, 16.6 mmol) in DMF (30 mL) and the reaction mixture was heated to 80-85 °C for 4 hours. After cooling, the reaction mixture was poured into saturated solution of NaHCO₃ (450 mL) and extracted with EtOAc (3 × 200 mL). The organic layer was additionally washed with water (2 × 150 mL) and brine (1 × 100 mL), dried over anhydrous sodium sulfate and concentrated. The products were purified by column chromatography (mobile phase: hexane/EtOAc, 25:1). 2,7-Dichloroquinoxaline: Yield: 16 % as a white solid; mp 134-136 °C. ¹H NMR (600 MHz, DMSO) δ 8.97 (s, 1H), 8.13-8.09 (m, 2H), 7.88 (dd, *J =* 8.9, 2.3 Hz, 1H). ¹³C NMR (151 MHz, DMSO) δ 148.54, 146.41, 142.12, 139.73, 136.52, 131.79, 131.32, 127.54. Elem. Anal. Calcd. for C₈H₄Cl₂N₂: C, 48.28; H, 2.03; N, 14.07. Found: C, 48.44; H, 2.15; N, 14.23.

### EXAMPLE 61

### 2,6-Dichloroquinoxaline (VIIe, intermediate compound)

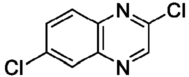

Title compound was prepared according to method described in Example 60 and isolated as a second product by column chromatography (mobile phase: hexane/EtOAc, 25:1). Yield: 33 % as a white solid; mp 157-158 °C (lit mp [30] 156-158 °C). ¹H NMR (600 MHz, DMSO-*D₆*) δ 8.99 (s, 1H), 8.19 (d, *J =* 2.4 Hz, 1H), 8.02 (d, *J =* 8.9 Hz, 1H), 7.91 (dd, *J* = 8.9, 2.3 Hz, 1H). ¹³C NMR (151 MHz, DMSO-*D₆*) δ 147.81, 147.15, 141.36, 140.47, 135.51, 132.65, 130.53, 128.37. Elem. Anal. Calcd. for C₈H₄Cl₂N₂: C, 48.28; H, 2.03; N, 14.07. Found: C, 48.63; H, 2.21; N, 14.19.

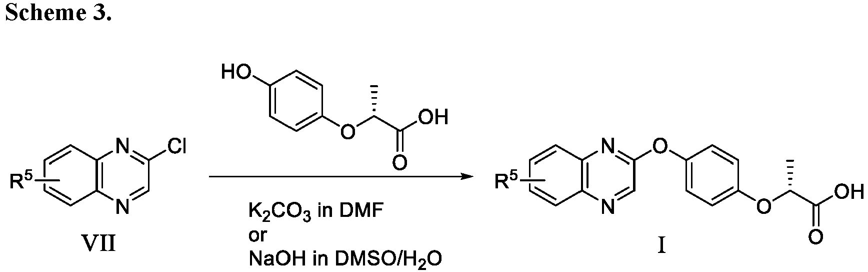

### General Procedure 11: Synthesis of compounds of general formula I

The mixture of (*R*)-2-(4-hydroxyphenoxy)propanoic acid (0.14 g, 0.75 mmol) and potassium carbonate (0.21 g, 1.5 mmol) was stirred in DMF (7 ml) at rt for 30 min and then was heated to 75 °C for 2 hours. Substituted-chloroquinoxalines of general formula VII (0.5 mmol) was added and reaction mixture was heated to 145 °C for 2-10 hours. After reaction completion, the reaction mixture was poured into ice water (50 mL), acidified to pH 3-4 and extracted with EtOAc (3 × 30 mL). The organic layer was additionally washed with water (2 × 20 mL) and brine (1 × 20 mL), dried over anhydrous sodium sulfate and concentrated. The product was purified by column chromatography or suspended with diethyl ether and filtered off.

### General Procedure 12: Synthesis of compounds of general formula I

The mixture of (R)-2-(4-hydroxyphenoxy)propanoic acid (0.18 g, 1 mmol) and sodium hydroxide (0.09 g, 2.2 mmol) in mixture of DMSO (1 mL) and water (0.5 mL) was heated to 70 °C for 30 min. Then, a solution of Substituted-chloroquinoxalines of general formula VII (1 mmol) in DMSO (2 mL) was added and the reaction mixture was heated to 100 °C for 1-12 hours. After reaction completion, the reaction mixture was poured into ice water (50 mL), acidified to pH 3-4 and extracted with EtOAc (3 × 30 mL). The organic layer was additionally washed with water (2 × 20 mL) and brine (1 × 20 mL), dried over anhydrous sodium sulfate and concentrated. The products were purified by column chromatography (mobile phase: Hex/EtOAc/CH₃COOH, 30:10:1).

### EXAMPLE 62

### (R)-2-(4-(Quinoxalin-2-yl)oxy)phenoxy)propanoic acid

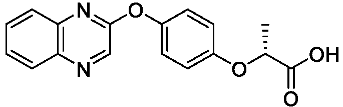

Title compound was prepared according to General Procedure 11 (Scheme 3). The reaction mixture was heated for 2 hours. The product suspended with diethyl ether and filtered off. Yield: 67 % as a white solid; mp 162-163 °C. ¹H NMR (500 MHz, DMSO) δ 13.07 (s, 1H), 8.83 (s, 1H), 8.05 (d, *J* = 7.8 Hz, 1H), 7.72 (d, *J* = 3.7 Hz, 2H), 7.70-7.66 (m, 1H), 7.26-7.23 (m, 2H), 6.98-6.95 (m, 2H), 4.86 (q, *J* = 6.7 Hz, 1H), 1.53 (d, *J* = 6.8 Hz, 3H). ¹³C NMR (126 MHz, DMSO-*D₆*) δ 173.29, 157.29, 155.07, 146.21, 139.91, 139.44, 139.18, 130.82, 128.83, 127.71, 127.35, 122.79, 115.81, 72.01, 18.52. Elem. Anal. Calcd. for C₁₇H₁₄N₂O₄: C, 65.80; H, 4.55; N, 9.03. Found: C, 65.52; H, 4.69; N, 8.86.

2-Chloroquinoxaline used for the synthesis of (*R*)-2-(4-(quinoxalin-2-yl)oxy)phenoxy) propanoic acid is commercially available.

### EXAMPLE 63

### (R)-2-(4-((7-Chloroquinoxalin-2-yl)oxy)phenoxy)propanoic acid

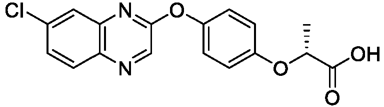

Title compound was prepared according to General Procedure 11 (Scheme 3). The reaction mixture was heated for 6 hours. The product was purified by column chromatography (mobile phase: hexane/EtOAc/CH₃COOH, 60:40: 1). The yield: 75 % as a white solid; mp 203-204 °C. ¹H NMR (600 MHz, DMSO) δ 13.04 (s, 1H), 8.80 (s, 1H), 8.03 (d, *J* = 8.8 Hz, 1H), 7.77 (d, *J* = 2.3 Hz, 1H), 7.66 (dd, *J* = 2.3, 8.8 Hz, 1H), 7.23-7.20 (m, 2H), 6.94-6.90 (m, 2H), 4.82 (q, *J* = 6.8 Hz, 1H), 1.49 (d, *J* = 6.8 Hz, 3H). ¹³C NMR (151 MHz, DMSO) δ 173.63, 158.23, 155.55, 146.39, 140.88, 140.55, 138.16, 135.60, 130.89, 128.57, 126.60, 123.09, 116.20, 72.38, 18.87. Elem. Anal. Calcd. for C₁₇H₁₃ClN₂O₄: C, 59.23; H, 3.80; N, 8.13. Found: C, 59.15; H, 3.82; N, 8.11.

### EXAMPLE 64

### (R)-2-(4-((6-Chloroquinoxalin-2-yl)oxy)phenoxy)propanoic acid

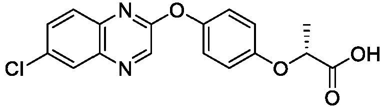

Title compound was prepared according to General Procedure 12 (Scheme 3). The reaction mixture was heated for 2 hours. The product was suspended with diethyl ether and filtered off. Yield: 69 % as a beige solid; mp 130-131 °C. ¹H NMR (600 MHz, DMSO) δ 13.02 (s, 1H), 8.84 (s, 1H), 8.09 (dd, *J=* 1.9, 1.0 Hz, 1H), 7.71-7.70 (m, 2H), 7.21 (d, *J* = 9.0 Hz, 2H), 6.93 (d, *J* = 9.0 Hz, 2H), 4.82 (q, *J* = 6.8 Hz, 1H), 1.50 (d, *J* = 6.8 Hz, 3H). ¹³C NMR (151 MHz, DMSO) δ 173.78, 153.42, 150.37, 142.31, 140.19, 137.51, 134.18, 130.70, 128.75, 128.44, 127.72, 120.84, 115.69, 72.37, 18.87. Elem. Anal. Calcd. for C₁₇H₁₃ClN₂O₄: C, 59.23; H, 3.80; N, 8.13. Found: C, 59.45; H, 3.63; N, 8.1.

### EXAMPLE 65

### (R)-2-(4-((7-Bromoquinoxalin-2-yl)oxy)phenoxy)propanoic acid

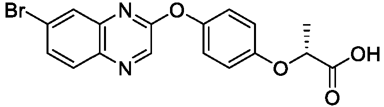

Title compound was prepared according to General Procedure 11 (Scheme 3). The reaction mixture was heated for 6 hours. The product was purified by column chromatography (mobile phase: hexane/EtOAc 1:1). Yield: 48 % as a white solid; mp 194-196 °C. ¹H NMR (500 MHz, DMSO) δ 8.85 (s, 1H), 7.98 (d, *J=* 8.8 Hz, 1H), 7.94 (d, *J=* 2.2 Hz, 1H), 7.81 (dd, *J=* 2.2, 8.8 Hz, 1H), 7.26-7.23 (m, 2H), 6.97-6.94 (m, 2H), 4.84 (q, *J =* 6.7 Hz, 1H), 1.52 (d, *J=* 6.7 Hz, 3H). ¹³C NMR (126 MHz, DMSO) δ 173.61, 158.05, 155.50, 146.26, 140.95, 140.71, 138.30, 131.13, 130.90, 129.69, 124.15, 122.98, 116.10, 72.40, 18.83. Elem. Anal. Calcd. for C₁₇H₁₃BrN₂O₄: C, 52.46; H, 3.37; N, 7.20. Found: C, 52.18; H, 3.18; N, 7.04.

7-Bromo-2-chloroquinoxaline used for the synthesis of (*R*)-2-(4-(7-bromoquinoxalin-2-yloxy)phenoxy)propanoic acid is commercially available.

### EXAMPLE 66

### (R)-2-(4-((7-Methylquinoxalin-2-yl)oxy)phenoxy)propanoic acid

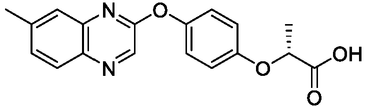

Title compound was prepared according to General Procedure 11 (Scheme 3). The reaction mixture was heated for 6 hours. The product was purified by column chromatography (mobile phase: hexane/EtOAc 1: 1). Yield: 62 % as a light beige solid; mp 138-140 °C. ¹H NMR (600 MHz, DMSO) δ 13.02 (s, 1H), 8.59 (s, 1H), 7.89 (d, *J* = 9.1 Hz, 1H), 7.25 (dd, *J* = 2.8, 9.1 Hz, 1H), 7.20-7.18 (m, 2H), 7.06 (d, *J* = 2.8 Hz, 1H), 6.93-6.91 (m, 2H), 4.82 (q, *J* = 6.8 Hz, 1H), 3.82 (s, 3H), 1.50 (d, *J* = 6.8 Hz, 3H). ¹³C NMR (151 MHz, DMSO) δ 173.68, 161.50, 158.15, 155.42, 146.67, 141.79, 136.71, 135.25, 130.17, 123.21, 120.07, 116.16, 106.68, 72.37, 56.42, 18.90. Elem. Anal. Calcd. for C₁₈H₁₆N₂O₅: C, 63.53; H, 4.74; N, 8.23. Found: C, 63.35; H, 4.63, N 7.95.

2-Chloro-7-methylquinoxaline used for the synthesis of (*R*)-2-(4-(7-methylquinoxalin-2-yloxy)phenoxy)propanoic acid is commercially available.

### EXAMPLE 67

### (R)-2-(4-((6,7-Dimethoxyquinoxalin-2-yl)oxy)phenoxy)propanoic acid

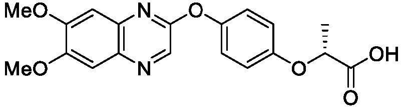

Title compound was prepared according to General Procedure 11 (Scheme 3). The reaction mixture was heated for 10 hours. The product was purified by column chromatography (mobile phase: hexane/EtOAc/CH₃COOH, 20:20:1). Yield: 31 % as a light beige solid; mp 136-138 °C. ¹H NMR (600 MHz, DMSO) δ 8.53 (s, 1H), 7.36 (s, 1H), 7.15-7.12 (m, 2H), 7.05 (s, 1H), 6.90-6.88 (m, 2H), 4.74 (q, *J* = 6.7 Hz, 1H), 3.88 (s, 3H), 3.83 (s, 3H), 1.47 (d, *J* = 6.7 Hz, 3H). ¹³C NMR (151 MHz, DMSO) δ 173.96, 157.07, 155.43, 153.29, 150.79, 146.90, 136.42, 135.91, 135.82, 123.02, 116.10, 107.62, 106.39, 72.80, 56.60, 56.40, 19.01. Elem. Anal. Calcd. for C₁₉H₁₈N₂O₆: C, 61.62; H, 4.90; N 7.56. Found: C, 61.33; H, 4.88; N, 7.25.

2-Chloro-6,7-dimethoxy-quinoxaline used for the synthesis of (R)-2-(4-(6,7-dimethoxyquinoxalin-2-yloxy)phenoxy)propanoic acid is commercially available.

### EXAMPLE 68

### (R)-2-(4-((7-Iodoquinoxalin-2-yl)oxy)phenoxy)propanoic acid

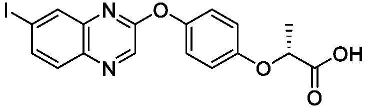

Title compound was prepared according to General Procedure 11 (Scheme 3). The reaction mixture was heated for 6 hours. The product was purified by column chromatography (mobile phase: hexane/EtOAc/CH₃COOH, 10:10:1). Yield: 57 % as a light beige solid; mp 170-172 °C. ¹H NMR (500 MHz, DMSO) δ 8.81 (s, 1H), 8.10 (d, *J* = 1.9 Hz, 1H), 7.92 (dd, *J* = 1.9, 8.7 Hz, 1H), 7.78 (d, *J* = 8.7 Hz, 1H), 7.21-7.17 (m, 2H), 6.93-6.90 (m, 2H), 4.67 (q, *J* = 6.7 Hz, 1H), 1.47 (d, *J* = 6.8 Hz, 3H). ¹³C NMR (126 MHz, DMSO) δ 173.84, 157.52, 155.70, 145.55, 140.61, 140.48, 138.30, 136.22, 135.69, 130.33, 122.46, 115.67, 97.53, 73.25, 18.82. Elem. Anal. Calcd. for C₁₇H₁₃IN₂O₄: C, 46.81; H, 3.00; N, 6.42. Found: C, 46.48; H, 2.81; N, 6.63.

### EXAMPLE 69

### (R)-2-(4-((7-Acetamidoquinoxalin-2-yl)oxy)phenoxy)propanoic acid

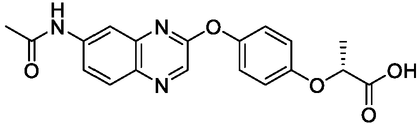

Title compound was prepared according to General Procedure 11 (Scheme 3). The reaction mixture was heated for 6 hours. The product was purified by column chromatography (mobile phase: hexane/EtOAc/CH₃COOH, 5:5:1). Yield: 67 % as a light beige solid; mp 243-244 °C. ¹H NMR (600 MHz, DMSO) δ 10.50 (s, 1H), 8.57 (s, 1H), 8.08 (d, *J* = 2.3 Hz, 1H), 7.89 (d, *J* = 8.9 Hz, 1H), 7.65 (dd, *J* = 2.3, 9.1 Hz, 1H), 7.09-7.08 (m, 2H), 6.86-6.85 (m, 2H), 4.39 (q, *J* = 6.9 Hz, 1H), 2.05 (s, 3H), 1.40 (d, *J* = 6.7 Hz, 3H). ¹³C NMR (151 MHz, DMSO) δ 175.35, 169.61, 158.18, 156.62, 145.58, 141.63, 140.77, 137.73, 136.04, 129.39, 122.63, 120.92, 115.97, 114.39, 75.41, 24.70, 19.60. Elem. Anal. Calcd. for C₁₉H₁₇N₃O₅: C, 62.12; H, 4.66; N, 11.44. Found: C, 61.89; H, 4.32; N, 11.51.

### EXAMPLE 70

### (R)-2-(4-((7-Methoxyquinoxalin-2-yl)oxy)phenoxy)propanoic acid

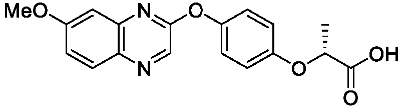

Title compound was prepared according to General Procedure 11 (Scheme 3). The reaction mixture was heated for 6 hours. The product was purified by column chromatography (mobile phase: hexane/EtOAc, 1:1). Yield: 62 % as a light beige solid; mp 138-140 °C. ¹H NMR (600 MHz, DMSO) δ 13.02 (s, 1H), 8.59 (s, 1H), 7.89 (d, *J* = 9.1 Hz, 1H), 7.25 (dd, *J* = 2.8, 9.1 Hz, 1H), 7.20-7.18 (m, 2H), 7.06 (d, *J* = 2.8 Hz, 1H), 6.93-6.91 (m, 2H), 4.82 (q, *J* = 6.8 Hz, 1H), 3.82 (s, 3H), 1.50 (d, *J* = 6.8 Hz, 3H). ¹³C NMR (151 MHz, DMSO) δ 173.68, 161.50, 158.15, 155.42, 146.67, 141.79, 136.71, 135.25, 130.17, 123.21, 120.07, 116.16, 106.68, 72.37, 56.42, 18.90. Elem. Anal. Calcd. for C₁₈H₁₆N₂O₅: C, 63.53; H, 4.74; N, 8.23. Found: C, 63.35; H, 4.63, N 7.95.

2-Chloro-7-methoxyquinoxaline used for the synthesis of (*R*)-2-(4-((7-methoxyquinoxalin-2-yl)oxy)phenoxy)propanoic acid is commercially available.

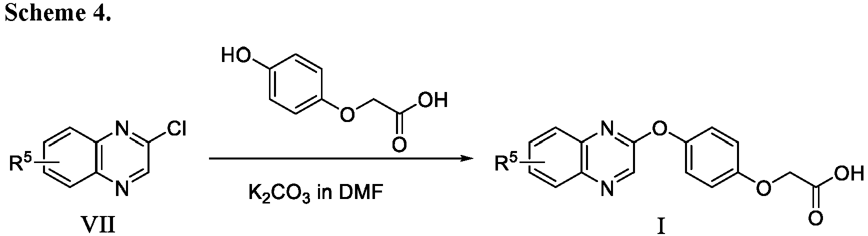

### General Procedure 13: Synthesis of compounds of general formula I

The mixture of -2-(4-hydroxyphenoxy)acetic acid (0.75 mmol) and potassium carbonate (0.21 g, 1.5 mmol) was stirred in DMF (7 ml) at rt for 30 min and then was heated to 75 °C for 2 hours. Substituted-chloroquinoxalines of general formula VII (0.5 mmol) was added and reaction mixture was heated to 145 °Cfor 2-10 hours. After reaction completion, the reaction mixture was poured into ice water (50 mL), acidified to pH 3-4 and extracted with EtOAc (3 × 30 mL). The organic layer was additionally washed with water (2 × 20 mL) and brine (1 × 20 mL), dried over anhydrous sodium sulfate and concentrated. The product was purified by column chromatography or suspended with diethyl ether and filtered off.

### EXAMPLE 71

### 2-(4-(Quinoxalin-2-yloxy)phenoxy)acetic acid

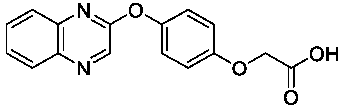

Title compound was prepared according to General Procedure 13 (Scheme 4). The reaction mixture was heated for 2 hours. The product was suspended with diethyl ether and filtered off. The yield: 64 % as a white solid; mp 185-187 °C. ¹H NMR (500 MHz, DMSO-*D₆*) δ 8.81 (s, 1H), 8.05 (d, *J* = 7.6 Hz, 1H), 7.74-7.66 (m, 3H), 7.26-7.22 (m, 2H), 7.02-6.99 (m, 2H), 4.71 (s, 2H). ¹³C NMR (126 MHz, DMSO-*D₆*) δ 170.56, 157.44, 155.47, 146.40, 140.01, 139.55, 139.27, 131.00, 128.94, 127.88, 127.45, 122.91, 115.66, 65.10. Elem. Anal. Calcd. for C₁₆H₁₂N₂O₄: C, 64.86; H, 4.08; N, 9.46. Found: C, 64.78; H, 3.91; N, 9.5.

### EXAMPLE 72

### 2-(4-((7-Iodoquinoxalin-2-yl)oxy)phenoxy)acetic acid

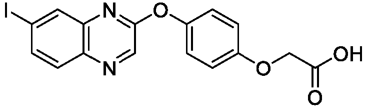

Title compound was prepared according to General Procedure 13 (Scheme 4). The reaction mixture was heated for 6 hours. The product was purified by column chromatography (mobile phase: hexane/EtOAc/CH₃COOH, 10:10:1). Yield: 62 % as a white solid; mp 177-178 °C. ¹H NMR (500 MHz, DMSO) δ 8.83 (s, 1H), 8.10 (d, *J =* 1.8 Hz, 1H), 7.94 (dd, *J =* 1.9, 8.6 Hz, 1H), 7.80 (d, *J* = 8.7 Hz, 1H), 7.26-7.23 (m, 2H), 7.01-6.98 (m, 2H), 4.69 (s, 2H). ¹³C NMR (126 MHz, DMSO) δ 170.45, 157.51, 155.53, 146.05, 140.65, 140.48, 138.36, 136.30, 135.69, 130.38, 122.66, 115.53, 97.59, 65.19. Elem. Anal. Calcd. for C₁₆H₁₁IN₂O₄: C, 45.52; H, 2.63; N, 6.64. Found: C, 45.37; H, 2.49; N, 6.5.

### EXAMPLE 73

### 2-(4-((7-Acetamidoquinoxalin-2-yl)oxy)phenoxy)acetic acid

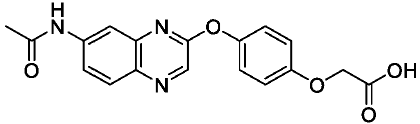

Title compound was prepared according to General Procedure 13 (Scheme 4). The reaction mixture was heated for 6 hours. The product was purified by column chromatography (mobile phase: hexane/EtOAc/CH₃COOH, 5:5: 1). The yield: 67 % as a light pink solid; mp 216-218 °C. ¹H NMR (500 MHz, DMSO) δ 10.39 (s, 1H), 8.66 (s, 1H), 8.14 (d, *J* = 2.3 Hz, 1H), 7.96 (d, *J* = 9.0 Hz, 1H), 7.67 (dd, *J* = 2.2, 9.0 Hz, 1H), 7.25-7.22 (m, 2H), 7.02-6.99 (m, 2H), 4.72 (s, 2H), 2.10 (s, 3H). ¹³C NMR (126 MHz, DMSO) δ 170.42, 169.30, 162.55, 157.77, 155.33, 146.29, 141.22, 140.40, 137.52, 135.80, 129.18, 122.81, 120.61, 115.56, 114.06, 65.06, 24.42.Elem. Anal. Calcd. for C₁₈H₁₅N₃O₅: C, 61.19; H, 4.28; N, 11.89. Found: C, 61.35; H, 4.33; N, 11.76.

### EXAMPLE 74

### 2-(4-(Pyrazin-2-yloxy)phenoxy)acetic acid

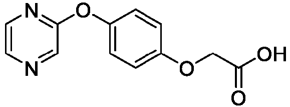

Title compound was prepared according to General Procedure 13 (Scheme 4). 2-Chloropyrazine was used instead of substituted-chloroquinoxaline of general formula VII. The reaction mixture was heated for 6 hours. The product was purified by column chromatography (mobile phase: hexane/EtOAc/CH₃COOH, 10:10:1). Yield: 78 % as a light beige solid; mp 194-196 °C. ¹H NMR (500 MHz, DMSO) δ 8.49 (d, *J* = 1.4 Hz, 1H), 8.33 (d, *J* = 2.7 Hz, 1H), 8.17 (dd, *J* = 1.4, 2.7 Hz, 1H), 7.13-7.09 (m, 2H), 6.95-6.92 (m, 2H), 4.59 (s, 2H). ¹³C NMR (126 MHz, DMSO) δ 170.64, 160.21, 155.51, 146.39, 141.31, 138.73, 135.51, 122.53, 115.54, 65.65. Elem. Anal. Calcd. for C₁₂H₁₀N₂O₄: C, 58.54; H, 4.09; N, 11.38. Found: C, 58.19; H, 4.23; N, 11.2.

### EXAMPLE 75

### (R)-2-(4-(Pyrazin-2-yloxy)phenoxy)propanoic acid

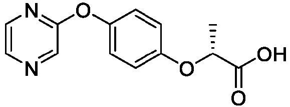

Title compound was prepared according to General Procedure 11 (Scheme 3). 2-Chloropyrazine was used instead of substituted-chloroquinoxaline of general formula VII. The reaction mixture was heated for 6 hours. The product was purified by column chromatography (mobile phase: hexane/EtOAc/CH₃COOH, 10:10:1). Yield: 76 % as a light beige solid; mp 131-133 °C. ¹H NMR (500 MHz, DMSO) δ 13.03 (s, 1H), 8.49 (d, *J* = 1.4 Hz, 1H), 8.33 (d, *J* = 2.8 Hz, 1H), 8.17 (dd, *J* = 1.4, 2.8 Hz, 1H), 7.13-7.10 (m, 2H),6.93-6.90 (m, 2H), 4.81 (q, *J* = 6.7 Hz, 1H), 1.50 (d, *J* = 6.8 Hz, 3H). ¹³C NMR (126 MHz, DMSO) δ 173.29, 160.14, 154.96, 146.50, 141.28, 138.75, 135.52, 122.59, 115.88, 72.16, 18.50. Elem. Anal. Calcd. for C₁₃H₁₂N₂O₄: C, 60.00; H, 4.65; N, 10.76. Found: C, 59.82; H, 4.53; N, 10.59.

### EXAMPLE 76

### Ethyl (R)-2-(4-((5-chloroquinolin-2-yl)oxy)phenoxy)propanoate

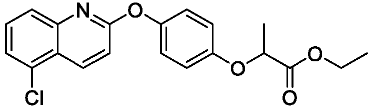

Sulfuric acid (3 drops) was added to solution of (*R*)-2-(4-((5-chloroquinolin-2-yl)oxy)phenoxy)propanoic acid (**EXAMPLE 37**) (0.16 mmol, 55 mg) in ethanol (5 mL) and reaction mixture was stirred at rt for 2 hours. Upon completion, the reaction mixture was concentrated under reduced pressure. The product was purified by column chromatography (mobile phase: hexane/EtOAc, 7:1). Yield: 32 % as a colorless oil. ¹H NMR (600 MHz, DMSO) δ 8.51 (d, *J* = 9.1 Hz, 1H), 7.61-7.56 (m, 3H), 7.33 (d, *J* = 9.1 Hz, 1H), 7.16-7.14 (m, 2H), 6.94-6.91 (m, 2H), 4.93 (q, *J* = 6.7 Hz, 1H), 4.17-4.09 (m, 2H), 1.50 (d, *J* = 6.7 Hz, 3H), 1.16 (t, *J* = 7.1 Hz, 3H). ¹³C NMR (151 MHz, DMSO) δ 172.07, 162.76, 154.99, 147.37, 147.22, 137.07, 130.86, 130.80, 128.13, 125.64, 123.51, 123.24, 116.33, 114.74, 72.61, 61.34, 18.83, 14.53. Elem. Anal. Calcd. for C₂₀H₁₈ClNO₄: C, 64.61; H, 4.88; N, 3.77; Found: C, 64.45; H, 4.92; N, 3.63.

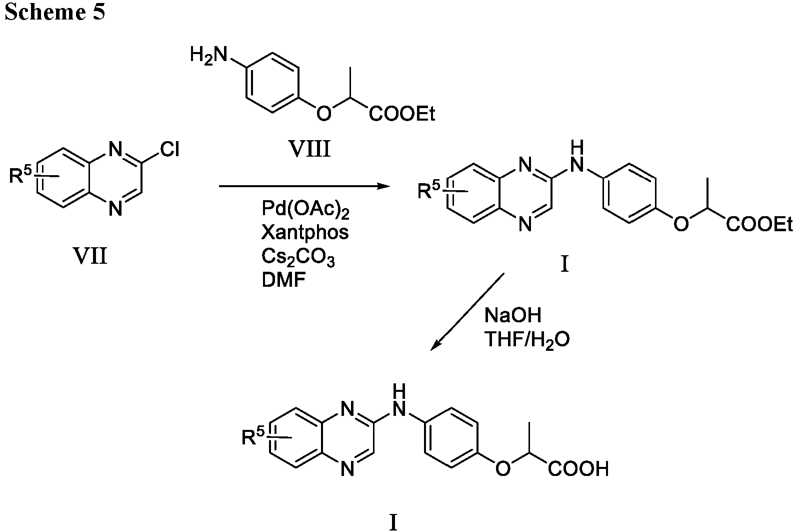

### General procedure 14: Synthesis of ethyl esters of general formula I

Substituted 2-chloroquinoxaline of general formula VII (0.2 g, 1 mmol), ethyl 2-(4-aminophenoxy)propanoate VIII (0.21 g, 1 mmol), xantphos (29 mg, 0.05 mmol), Pd(OAc)₂ (0.011 g, 0.05 mmol) and Cs₂CO₃ (0.39 g, 1.2 mmol) in DMF (7 mL) was heated to 85 °C for 20 hours under inert atmosphere. After cooling, the reaction mixture was diluted with EtOAc (100 mL) and filtered through silica gel. Silica gel layer was additionally washed with EtOAc (150 mL). The filtrate was concentrated, and product was purified by column chromatography (mobile phase: hexane/EtOAc, 3:1).

### General procedure 15: Synthesis of final compounds of general formula I

A solution of compound of general formula IX (0.09 g, 0.242 mmol) and 0.1 M sodium hydroxide (4.84 mL) in THF (2 mL) was stirred at rt for 3 hours. The reaction mixture was diluted with water (15 mL) and washed with EtOAc (1 × 10 mL). The aqueous layer was acidified with 1 M HCl to pH 4 and extracted with EtOAc (2 ×15 mL). The organic layer was washed with water (1 × 10 mL) and brine (1 × 10 mL), dried over anhydrous sodium sulfate, and concentrated to give product of general formula I in high purity.

### EXAMPLE 77

### Ethyl 2-(4-aminophenoxy)propanoate (VIII, intermediate compound)

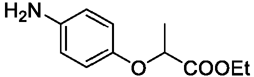

A mixture of 4-hydroxyaniline (2 g, 18.33 mmol) and BOC₂O (4 g, 18.33 mmol) in DMF (10 mL) was stirred at rt for 2 hours. The reaction mixture was concentrated, the residue was dissolved in EtOAc (100 mL) and washed with 5 % NaHCO₃ (2 × 75 mL), water (2 × 75 mL) and brine (1 × 75 mL). The organic layer was dried over anhydrous sodium sulfate and concentrated to give tert-butyl (4-hydroxyphenyl)carbamate as a white solid in 93 % yield in high purity; mp 137-138 °C. ¹H NMR (500 MHz, DMSO) δ 9.01 (s, 1H), 8.97 (d, *J* = 9.8 Hz, 1H), 7.20 (d, *J* = 8.3 Hz, 2H), 6.6 9 - 6.56 (m, 2H), 1.44 (s, 9H). ¹³C NMR (126 MHz, DMSO) δ 153.46, 152.96, 131.47, 120.45, 115.46, 78.87, 28.65.

A mixture of tert-butyl (4-hydroxyphenyl)carbamate (2.5 g, 12.06 mmol), ethyl 2-bromopropionate (14.46 mmol) and potassium carbonate (2.5 g, 18.09 mmol) in DMF (25 mL) was heated to 110 °C for 4 hours. The reaction mixture was concentrated, the residue was dissolved in EtOAc (100 mL) and washed with water (3 × 75 mL) and brine (1 × 75 mL). The organic layer was dried over anhydrous sodium sulfate and concentrated. The product was purified by column chromatography (mobile phase: hexane/EtOAc, 7:1). Yield: 91 % as a white solid; mp 77-78 °C. ¹H NMR (500 MHz, acetone) δ 8.22 (s, 1H), 7.48 - 7.42 (m, 2H), 6.86 - 6.81 (m, 2H), 4.79 (q, *J* = 6.8 Hz, 1H), 4.18 (q, *J* = 7.1 Hz, 2H), 1.54 (d, *J* = 6.7 Hz, 3H), 1.48 (s, 9H), 1.23 (t, *J* = 7.1 Hz, 3H). ¹³C NMR (126 MHz, acetone) δ 171.61, 153.27, 153.00, 133.62, 119.58, 115.42, 78.79, 72.75, 60.54, 27.67, 17.94, 13.54.

Trifluoroacetic acid (2 mL) was added dropwise to a solution of ethyl 2-(4-((tert-butoxycarbonyl)amino)phenoxy)propanoate (0.58 g, 1.86 mmol) in CH₂Cl₂ (20 mL) at cooling in ice bath. The reaction mixture was stirred for 3 hours at rt and then was poured into saturated solution of NaHCO₃ (100 mL). The organic layer was separated, washed with water (2 × 70 mL) and brine (1 × 70 mL), dried over anhydrous sodium sulfate and concentrated to give product in high purity. Yield of VIII: 70 % as a brownish oil. ¹H NMR (500 MHz, DMSO) δ 6.64 - 6.57 (m, 2H), 6.50 - 6.45 (m, 2H), 4.68 (s, 2H), 4.64 (q, *J* = 6.9 Hz, 1H), 4.16 - 3.97 (m, 2H), 1.42 (dd, *J* = 6.8, 0.5 Hz, 3H), 1.16 (td, *J* = 7.1, 0.5 Hz, 3H). ¹³C NMR (126 MHz, DMSO) δ 172.19, 148.62, 143.31, 116.57, 114.92, 73.06, 60.62, 18.54, 14.16.

### EXAMPLE 78

### Ethyl 2-(4-((7-chloroquinoxalin-2-yl)amino)phenoxy)propanoate

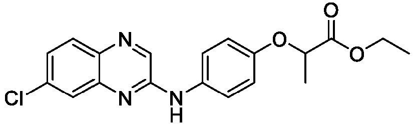

Title compound was prepared according to General Procedure 14 (Scheme 5). Yield: 36 % as a yellow solid; mp 130-132 °C. ¹H NMR (500 MHz, DMSO) δ 9.99 (s, 1H), 8.50 (d, *J* = 0.9 Hz, 1H), 7.86 (d, *J* = 9.1 Hz, 2H), 7.84 - 7.80 (m, 1H), 7.75 - 7.62 (m, 1H), 7.46 - 7.38 (m, 1H), 6.93 - 6.85 (m, 2H), 4.97 - 4.79 (m, 1H), 4.17 - 4.13 (m, 2H), 1.51 (dd, *J* = 6.7, 1.0 Hz, 3H), 1.21 - 1.17 (m, 3H). ¹³C NMR (126 MHz, DMSO) δ 171.84, 152.88, 150.22, 141.83, 141.35, 135.48, 134.38, 133.99, 130.23, 125.21, 124.95, 120.55, 115.48, 72.19, 60.93, 18.51, 14.24. Elem. Anal. Calcd. for C₁₉H₁₃ClN₃O₃: C, 61.38; H, 4.88; N, 11.30. Found: C, 61.09; H, 4.58; N, 11.47.

### EXAMPLE 79

### Ethyl 2-(4-((6-chloroquinoxalin-2-yl)amino)phenoxy)propanoate

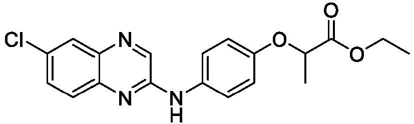

Title compound was prepared according to General Procedure 14 (Scheme 5). Yield: 53 % as a yellow solid; mp 141-143 °C. ¹H NMR (600 MHz, DMSO-*D*₆) δ 9.87 (s, 1H), 8.48 (s, 1H), 7.84 - 7.79 (m, 3H), 7.64 (d, *J* = 8.8 Hz, 1H), 7.58 (dd, *J* = 8.8, 2.4 Hz, 1H), 6.88 (d, *J* = 9.1 Hz, 2H), 4.87 (q, *J* = 6.8 Hz, 1H), 4.13 - 4.09 (m, 2H), 1.47 (d, *J* = 6.7 Hz, 3H), 1.15 (t, *J* = 7.1 Hz, 3H). ¹³C NMR (151 MHz, DMSO-*D*₆) δ 172.16, 153.19, 150.35, 142.32, 140.15, 137.53, 134.44, 130.72, 128.78, 128.43, 127.73, 120.80, 115.91, 72.61, 61.25, 18.83, 14.54. Elem. Anal. Calcd. for C₁₉H₁₈ClN₃O₃: C, 61.38; H, 4.88; N, 11.30. Found: C, 61.58; H, 4.61; N, 11.2.

### EXAMPLE 80

### 2-(4-((7-chloroquinoxalin-2-yl)amino)phenoxy)propanoic acid

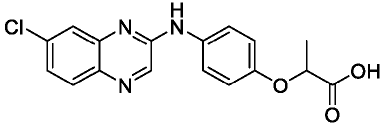

Title compound was prepared according to General Procedure 15 (Scheme 5). Yield: 90 % as a brownish solid; mp 237-240 °C (with decomp.). ¹H NMR (500 MHz, DMSO-*D*₆) δ 12.96 (s, 1H), 9.96 (s, 1H), 8.49 (s, 1H), 7.86 (d, *J* = 8.6 Hz, 2H), 7.82 (d, *J* = 8.7 Hz, 1H), 7.72 (d, *J* = 2.3 Hz, 1H), 7.42 (dd, *J* = 8.6, 2.3 Hz, 1H), 6.90 (d, *J* = 8.5 Hz, 2H), 4.80 (q, *J* = 6.7 Hz, 1H), 1.51 (d, *J* = 6.7 Hz, 3H). ¹³C NMR (126 MHz, DMSO-*D*₆) δ 173.50, 153.14, 150.24, 141.86, 141.34, 135.47, 134.39, 133.72, 130.22, 125.23, 124.92, 120.59, 115.28, 72.00, 18.56. Elem. Anal. Calcd. for C₁₇H₁₄ClN₃O₃: C, 59.40; H, 4.11; N, 12.22. Found: C, 59.25; H, 4.39; N, 12.51.

### EXAMPLE 81

### 2-(4-((6-chloroquinoxalin-2-yl)amino)phenoxy)propanoic acid

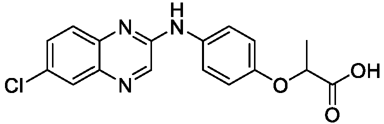

Title compound was prepared according to General Procedure 15 (Scheme 5). Yield: 94 % as a yellow solid; mp 253-254 °C. ¹H NMR (600 MHz, DMSO) δ 12.90 (s, 1H), 9.87 (s, 1H), 8.48 (s, 1H), 7.82 (d, *J* = 2.5 Hz, 1H), 7.81 (d, *J* = 9.0 Hz, 2H), 7.65 (d, *J* = 8.8 Hz, 1H), 7.57 (dd, *J* = 8.9, 2.4 Hz, 1H), 6.87 (d, *J* = 9.0 Hz, 2H), 4.76 (q, *J* = 6.7 Hz, 1H), 1.47 (d, *J* = 6.8 Hz, 3H). ¹³C NMR (151 MHz, DMSO) δ 173.63, 157.92, 155.53, 146.42, 141.62, 139.83, 138.61, 132.13, 131.51, 129.48, 128.02, 123.12, 116.21, 72.39, 18.87. Elem. Anal. Calcd. for C₁₇H₁₄ClN₃O₃: C, 59.40; H, 4.11; N, 12.22. Found: C, 59.63; H, 4.37; N, 11.95.

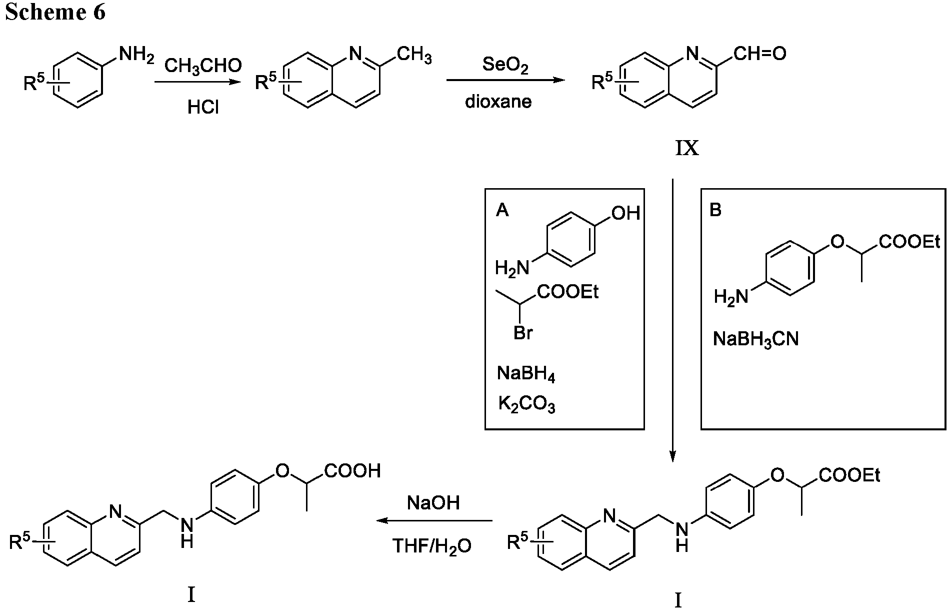

### General procedure 16: Synthesis of substituted quinoline-2-carbaldehyde of general formula IX

Acetaldehyde (2.2 g/ 2.8 mL, 0.05 mol) was dropped to a suspension of substituted aniline (0.012 mol) in HCl (10 mL) at 0 °C. The reaction mixture was stirred for 30 min at cooling and then was heated to 60 °C for 3 hours. After cooling to rt, the reaction mixture was diluted with water (150 mL) and the aqueous solution was separated by decantation. Powder NaHCO₃ was added to the aqueous solution to achieve pH 7-8. Aqueous phase was extracted with EtOAc (4 × 70 mL). The organic layer then was washed with water (2 × 50 mL) and brine (1 × 50 mL), dried over anhydrous sodium sulfate and concentrated. Resulting substituted 2-methylquinoline was purified by column chromatography (mobile phase: hexane/EtOAc, 15:1).

A mixture of substituted 2-methylquinoline (4.72 mmol) and selenium dioxide (0.78 g, 7.08 mmol) in dioxane (50 mL) was refluxed for 1 hour. The reaction mixture was filtered off, the filtrate was concentrated, and product was purified by column chromatography (mobile phase: hexane/EtOAc, 20:1)

### EXAMPLE 82

### 5-Chloroquinoline-2-carbaldehyde (IXa, intermediate compound)

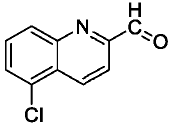

Title compound was prepared according to General Procedure 16 (Scheme 6). Yield: 59 % as a beige solid; mp 109-110 °C. ¹H NMR (600 MHz, DMSO) δ 10.10 (d, *J* = 0.9 Hz, 1H), 8.70 (d, *J* = 8.7 Hz, 1H), 8.17 (dt, *J* = 8.4, 1.0 Hz, 1H), 8.05 (d, *J* = 8.7 Hz, 1H), 7.92 (dd, *J* = 7.5, 1.1 Hz, 1H), 7.85 (dd, *J* = 8.4, 7.5 Hz, 1H). ¹³C NMR (151 MHz, DMSO) δ 193.85, 153.41, 148.39, 134.91, 131.51, 130.96, 130.02, 129.9, 127.68, 118.93 Elem. Anal. Calcd. for C₁₀H₆ClNO: C, 62.68; H, 3.16; N, 7.31. Found: C, 62.34; H, 3.35; N, 7.17.

### EXAMPLE 83

### 5,7-Dichloroquinoline-2-carbaldehyde (IXb, intermediate compound)

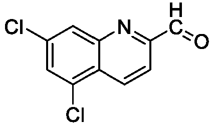

Title compound was prepared according to General Procedure 16 (Scheme 6). Yield: 80 % as a beige solid; mp 141-143 °C. ¹H NMR (500 MHz, DMSO) δ 10.12 (s, 1H), 8.75 (d, *J* = 8.7 Hz, 1H), 8.32 - 8.24 (m, 1H), 8.13 - 8.07 (m, 2H). ¹³C NMR (126 MHz, DMSO) δ 193.08, 153.80, 147.89, 135.05, 134.81, 131.97, 129.53, 128.25, 126.11, 118.86. Elem. Anal. Calcd. for C₁₀H₅Cl₂NO: C, 53.13; H, 2.23; N, 6.20. Found: C, 52.85; H, 2.1; N, 6.37.

### EXAMPLE 84

### 2-(4-(((5,7-Dichloroquinolin-2-yl)methyl)amino)phenoxy)propanoic acid

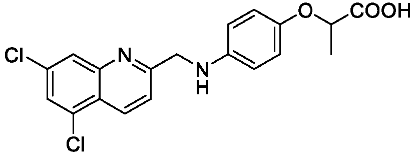

Title compound was prepared according to Scheme 6. A mixture of 5,7-dichloro-2-methylquinoline (0.21 g, 0.93 mmol) and 4-hydroxyaniline (0.13 g, 1.19 mmol) in CH₂Cl₂ (20 mL) was stirred at rt overnight. The reaction mixture was concentrated, and product was purified by column chromatography (mobile phase: hexane/EtOAc, 7:1). Resulting 4-(((5,7-dichloroquinolin-2-yl)methylene)amino)phenol (0.1 g, 0.315 mmol) was dissolved in acetone (15 mL) and potassium carbonate (0.06 g, 0.434 mmol) was added followed by addition of ethyl bromopropionate (0.074 g/53µL, 0.41 mmol). The reaction mixture was refluxed overnight. After cooling, it was concentrated, and the residue was dissolved in EtOAc (30 mL) and washed with water (2 × 30 mL) and brine (1 × 30 mL). The organic layer was dried over anhydrous sodium sulfate and concentrated. Ethyl 2-(4-(((5,7-dichloroquinolin-2-yl)methylene)amino)phenoxy)propanoate was obtained in 85% yield. It was dissolved in CH₃OH (10 mL) and sodium borohydride (0.03 g, 0.79 mmol) was added. The reaction mixture was stirred at rt for 15 min and then concentrated. The residue was dissolved in EtOAc (20 mL) and washed with water (2 × 20 mL) and brine (1 × 20 mL). The product is a mixture of methyl and ethyl esters of 2-(4-(((5,7-dichloroquinolin-2-yl)methyl)amino)phenoxy)propanoate. Total yield: 85 % as a yellow oil. A solution of the mixture of methyl and ethyl esters of 2-(4-(((5,7-dichloroquinolin-2-yl)methyl)amino)phenoxy)propanoate (75 mg) and 0.1 M NaOH (3.5 mL) in THF (5 mL) was stirred at rt for 3 hours. The reaction mixture was acidified to pH 5 and extracted with EtOAc (3 × 20 mL). The organic layer was washed with water (1 × 20 mL) and brine (1 × 20 mL) and concentrated. 2-(4-(((5,7-dichloroquinolin-2-yl)methyl)amino)phenoxy)propanoic acid was obtained in 50 % yield. ¹H NMR (500 MHz, DMSO) δ 12.71 (s, 1H), 8.49 (dd, *J* = 8.7*,* 0.9 Hz, 1H), 8.06 (dd, *J* = 2.1, 0.8 Hz, 1H), 7.87 (d, *J* = 2.0 Hz, 1H), 7.70 (d, *J* = 8.8 Hz, 1H), 6.67 - 6.60 (m, 2H), 6.56 - 6.45 (m, 3H), 4.54 (q, *J* = 6.7 Hz, 1H), 4.50 (s, 2H), 1.40 (d, *J* = 6.8 Hz, 3H). ¹³C NMR (126 MHz, DMSO) δ 173.77, 164.26, 149.23, 147.91, 143.08, 133.88, 133.19, 131.79, 127.12, 126.68, 123.65, 121.45, 116.32, 113.32, 72.52, 49.84, 18.59.

### EXAMPLE 85

### Ethyl 2-(4-(((5-chloroquinolin-2-yl)methyl)amino)phenoxy)propanoate

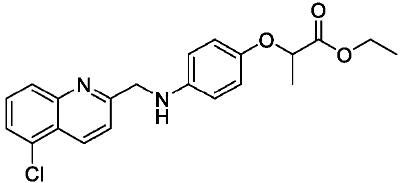

Title compound was prepared according to Scheme 6. To a solution of ethyl 2-(4-aminophenoxy)-2-propanoate VIII (0.22 g, 1.04 mmol) and acetic acid (63 mg/60 µL, 1.04 mmol) in CH₃OH (10 mL) was added of 5-chloroquinoline-2-carbaldehyde (0.2 g, 1.04 mmol), the reaction mixture was stirred for 5 min and then a solution of sodium cyanoborohydride (0.13 g, 2.08 mmol) in CH₃OH (5 mL) was added. The reaction mixture was stirred at rt for 1 hour and then concentrated under reduced pressure. The residue was dissolved in EtOAc (50 mL) and washed with water (2 × 40 mL). The organic layer was separated, dried over anhydrous sodium sulfate, and concentrated. Product was purified by column chromatography (mobile phase: hexane/EtOAc, 3:1). Yield: 38 % as a yellow oil. ¹H NMR (600 MHz, DMSO) δ 8.47 (dd, *J* = 8.7, 0.9 Hz, 1H), 8.00 - 7.94 (m, 1H), 7.73 - 7.68 (m, 2H), 7.65 (d, *J* = 8.7 Hz, 1H), 6.61 (d, *J* = 9.0 Hz, 2H), 6.49 (d, *J* = 9.0 Hz, 2H), 6.22 (s, 1H), 4.60 (q, *J* = 6.8 Hz, 1H), 4.48 (s, 2H), 4.04 (qd, *J* = 7.1, 1.2 Hz, 2H), 1.37 (d, *J* = 6.8 Hz, 3H), 1.08 (t, *J* = 7.1 Hz, 3H). ¹³C NMR (151 MHz, DMSO) δ 172.46, 162.95, 149.36, 148.35, 143.72, 133.31, 130.71, 130.29, 128.61, 126.83, 125.15, 121.49, 116.99, 113.72, 73.26, 61.01, 50.23, 18.88, 14.48.

### BIOLOGICAL METHODS

Method for assessing biological activities:

### Cellular models

Human hepatocellular carcinoma HepG2 cell line was purchased from the European Collection of Cell Cultures (ECACC, Salisbury, UK). Primary human hepatocytes (donor JEL) have been purchased from BioIV, Royston, UK.

### Reporter gene assay to determine interactions with PPARα, PPARγ, PPARβ/δ, LXRα and FXR

In HepG2 cells, luciferase gene reporter assays have been performed with the transiently transfected reporter gene luciferase construct with the UAS yeast binding site interacting with GAL4 protein (pGL5-UAS 9x, Promega). DNA constructs encoding human LBD domains of human PPARα, PPARγ, of LXRα (pGAL4-PPARα, pGAL4-PPARγ, pGAL4-LXRα) have been cotransfected with the reporter construct. For FXR, FXRE-luc construct with FXR response elements upstream of Firefly luciferase has been used together with an expression construct for human FXR for HepG2 cells transfection. HepG2 cells were then treated with compounds of the invention for 24 h. Dose-response curves with concentrations from 0.01 up to 30 µM of tested compounds have been analyzed using a plate reader luminometr to determine relative expression for nuclear receptors activation to either control (vehicle-treated samples) or to prototype NRs ligands (n=3) (Table 1).

### Cytotoxicity

Cell viability in HepG2 cells after 48 h treatment with test compounds at a concentration from 1 up to 30 µM was determined using the modified formazan-based MTT assay (CellTiter 96^{®}AQᵤₑₒᵤₛOne *Solution Cell Proliferation Assay (Cat. No. G3582, Promega,* Madison, WI, USA). Briefly, the cells were treated with the test compounds or vehicle alone (DMSO) for 48h. At the end of the treatment, 20µl of MTS reagent was added directly to each culture wells and further cultivated for 1.5 h. Finally, the absorbance of converted formazan was recorded at 490nm by plate reader (BioTec Synergy 2. Winooski. VT. USA). Vehicle (DMSO) and TritonX100 (0.9%; v/v) controls of cell viability was set to be 100% and 0%, respectively. In cytotoxic control, TritonX-100 (0.9% v/v) was added to cells 45 minutes before the addition of MTS reagent.

### Animal experiments

Six-week-old male C57BL/6J mice have been purchased from AnimaLab (Prague) and were housed in individually ventilated cages in 12 h light/dark cycle (23 ± 1 °C, 55 ± 10 % humidity).

In the pilot study, compounds of the invention **37** and **35** were applied by *per os* probe (gavage) to male mice of age 15 weeks (n=4 per group) three times (3 x 30 mg/kg b.w.) in 1%PEG300/methylcellulose solution (compounds of EXAMPLE **37**or **35**). Livers were sampled 24 hours after application into liquid nitrogen for total RNA insolation and for qRT-PCR analysis (Quantstudio 7 HT Fast Real-Time PCR System).

In the proof-of-concept study, after a 1-week of adaptation period, male C57BL/6J mice were randomly divided into three groups: (i) mice fed a chow diet (control; PicoLab RD 20, LabDiet) and tap water (*n*=4) or (ii) mice fed a high-fat diet (HFD) consisting of Western diet (AIN-76A WD, TestDiet, 20 % fat) and glucose (18.1 g/L) with fructose (24 g/L) provided in water. At week 21 on HFD diet, mice were randomly assigned into two experimental groups (10 and 11 mice per group): (ii) group on HFD, (iii) group on HFD administered with 10 mg/kg compound of EXAMPLE **37** as a salt (hydrochloride) (HFD +comp. **37**). Compound of EXAMPLE **37**or vehicle (1% methylcellulose) were administered once daily by oral gavage for another 3 weeks together with diets.

The *i.p.* Glucose tolerance test has been performed seven days before the termination of the study. For the purpose, 1.5 g per BW of glucose dissolved in saline has been applied and blood was sampled in four intervals up to 120 minutes form tail vein. A standard glucometer was used to measure glycemia.

For biochemistry analyses, plasma liver enzyme activities were analyzed in plasma by a commercial Preventive Care Profile Plus test with Vetscan 2 device (Abaxis). Level of cholesterol and triglycerides in plasma and liver were measured by commercial kits (Erba Lachema s.r.o., Brno, Czech Republic).

Animals were sacrificed by anesthesia overdose. Plasma samples were obtained from the whole blood by centrifugation at 2,000 × g for 5 min at 4°C. The liver, gallbladder and abdominal fat were excised, weighted and immediately frozen in liquid nitrogen. All samples were stored at -80°C until analysis.

All animal experiments were performed in compliance with the EU Directive 2010/63/EU and all animals received care according to the guidelines set by the Animal-welfare Body of the Charles University (Prague, Czech Republic). Project was approved by the Animal-welfare Body of Ministry of Education, Youth and Sports (approval No. MSMT-6649/2021-2).

### RT-qPCR experiments

Expressions of NR target genes mRNAs in livers of mice or in 3D cultured (spheroids) primary human hepatocytes have been performed using TaqMan probes-based RT-qPCR after isolation of total RNA by Trizol^{®} (ThermoFischer Scientific) reagent and reverse transcription synthesis of cDNA in Quant Studio cycler. Real-time qPCR TaqMan probes have been purchased prom ThermoFisher scientific (Cat. No. 4351372 and 433118). Delta-delta method and Gapdh or TATA-box binding protein (TBP) housekeeping genes were used for relative quantification. Data are expressed as fold up-/down-regulation compared to vehicle-treated samples/mice.

### Statistical analysis

All statistical analyses were made in GraphPad Prism 8 statistical software (San Diego, USA). Data are presented as means with standard deviations. The statistical significance was determined using either Student's t test or one-way ANOVA with Dunnett's. Values of p < 0.05 were considered statistically significant.

### Results

In the tested library of compounds of formula I, we found several agonists and inverse agonist/antagonist for tested human nuclear receptors PPARα, PPARγ, FXR, LXRα or TGR5 in luciferase reporter assays. These activities are depicted in Fig. 1-3 or summarized in Table 1, when we compared activation of these NRs with compounds of invention with known agonists/inverse agonist of the receptors.

Most of these novel compounds do not significantly affect viability of the HepG2 cell line (bellow 80%) after 48h-treatment as determined with Cell Titer 96^{®} AQᵤₑₒᵤₛ One Solution Cell Proliferation Assay (MTS).

In a pilot animal study, we applied compounds of EXAMPLEs **35** and **37** to C57BL/6J male animals in three doses (3x30 mg/kg). Subsequent RT-qPCR analyses showed that key target genes of FXR, LXRα and PPARs are significantly regulated in mice after application. Importantly, critical genes involved in fatty acid oxidation (Cpt1, Hadha, Hadhb, Ucp2, Ppara) or lipogenesis (Acaca, Scd1, Fasn, Srebf1) have been significantly up-regulated or suppressed (Fig. 4). Pck1 gene encoding for phosphoenolpyruvate carboxykinase 1 has been downregulated by both compounds of invention.

In the follow-up proof-of-concept study with the compound of EXAMPLE 37 in mice fed with high fat diet, we observed that the compound significantly decreases plasma triglyceride (TG) levels and improves ALT and AST plasma levels but increases ALP plasma levels in wild-type C57BL/6J mice fed with high fat diet (20%) with fructose in water. The compound of EXAMPLE **37** also significantly decreased body weight (BW) in HFD-treated mice after three weeks of treatment and significantly decreased glucose plasma levels in *i.p.* Glucose tolerance test (IPGTT, 1.5 g/kg BW) in the study after 15 minutes indicating improved glucose tolerance and utilization.

Similarly as in the pilot study, the compound of EXAMPLE **37** upregulated key genes of fatty acid oxidation but down-regulated key genes of gluconeogenesis. These data altogether suggest that the invented compound of EXAMPLE **37** decrease liver fatty acid synthesis but stimulates fatty acid oxidation indicating potential amelioration of hepatocyte triglycerides storage and liver steatosis. Suppression of Pck1 and G6pc genes may improve glycemia, glucose tolerance and mitigate insulin resistance in metabolic syndrome (Fig. 5,6).

Finally, we corroborated the effects of invented compounds of EXAMPLES **37** and **35** in 3D spheroids of primary human hepatocytes. We observed that compound of EXAMPLE **35** has augmented effect on some critical genes involved in fatty acid oxidation in comparison with fenofibrate, the prototype PPARα activating drug. The compound of EXAMPLE **35** displays a combine effect of obeticholic acid (the FXR ligand) and of TFCA (an inverse agonist of LXRα) with suitable upregulation of genes involved in fatty acid catabolism.

This invention discloses compounds of formula I, and compositions comprising these compounds, first-in-class class agonists or partial agonists or combined or dual agonists of FXR or PPARs receptors that are at the same time antagonists/inverse agonists of LXRα. In addition, some novel compounds of formula I, and compositions comprising these compounds, are agonists or partial agonists of GPBAR1 (TGR5) receptor. Thus, derivatives of formula I could be used for therapy of metabolic diseases or can bed for the further modification to tune multitarget synergistic activities against FXR, LXRα, PPARs and TGR5 receptors.

The present invention relates to a compound of general Formula I :

### Industrial Applicability

The compounds of the present invention are industrially manufacturable and usable for the treatment of many diseases which are mediated by the action, or by loss of action, of PPARα, PPARγ or FXR receptors or its endogenous ligands, or excessive stimulation of LXRα, such as dyslipidemias, atherosclerosis, cholestasis, lipid metabolic disorders, liver diseases or related pathologies.

### References cited

Alabaster CT, Bell AS, Campbell SF, Ellis P, Henderson CG, Roberts DA, Ruddock KS, Samuels GMR and Stefaniak MH (1988) 2(1H)-Quinolinones with cardiac stimulant activity. 1. Synthesis and biological activities of (six-membered heteroaryl)-substituted derivatives. Journal of Medicinal Chemistry 31:2048-2056.
Bunay J, Fouache A, Trousson A, de Joussineau C, Bouchareb E, Zhu Z, Kocer A, Morel L, Baron S and Lobaccaro JA (2021) Screening for liver X receptor modulators: Where are we and for what use? Br J Pharmacol 178:3277-3293.
Cariello M, Piccinin E and Moschetta A (2021) Transcriptional Regulation of Metabolic Pathways via Lipid-Sensing Nuclear Receptors PPARs, FXR, and LXR in NASH. Cell Mol Gastroenterol Hepatol 11:1519-1539.
Dibba P, Li AA, Perumpail BJ, John N, Sallam S, Shah ND, Kwong W, Cholankeril G, Kim D and Ahmed A (2018) Emerging Therapeutic Targets and Experimental Drugs for the Treatment of NAFLD. Diseases 6**.**
Effenberger FH, W. (1969) Chemische Berichte 102:3260-3267.
Fruchart JC, Hermans MP, Fruchart-Najib J and Kodama T (2021) Selective Peroxisome Proliferator-Activated Receptor Alpha Modulators (SPPARMalpha) in the Metabolic Syndrome: Is Pemafibrate Light at the End of the Tunnel? Curr Atheroscler Rep 23:3.
Giampietro L, Ammazzalorso A, Amoroso R and De Filippis B (2019a) Development of Fibrates as Important Scaffolds in Medicinal Chemistry. ChemMedChem 14:1051-1066.
Giampietro L, Laghezza A, Cerchia C, Florio R, Recinella L, Capone F, Ammazzalorso A, Bruno I, De Filippis B, Fantacuzzi M, Ferrante C, Maccallini C, Tortorella P, Verginelli F, Brunetti L, Cama A, Amoroso R, Loiodice F and Lavecchia A (2019b) Novel Phenyldiazenyl Fibrate Analogues as PPAR α/γ/δ Pan-Agonists for the Amelioration of Metabolic Syndrome. ACS Medicinal Chemistry Letters 10:545-551.
Griffett K, Welch RD, Flaveny CA, Kolar GR, Neuschwander-Tetri BA and Burris TP (2015) The LXR inverse agonist SR9238 suppresses fibrosis in a model of non-alcoholic steatohepatitis. Mol Metab 4:353-357
Jones PH (2009) Fibrates, in Clinical Lipidology: A Companion to Braunwald's Heart Disease (Ballantyne CM ed) pp 315-322.
Ni M, Zhang B, Zhao J, Feng Q, Peng J, Hu Y and Zhao Y (2019) Biological mechanisms and related natural modulators of liver X receptor in nonalcoholic fatty liver disease. Biomed Pharmacother 113:109779
Parlati L, Regnier M, Guillou H and Postic C (2021) New targets for NAFLD. JHEP Rep 3:100346.
Pawlak M, Lefebvre P and Staels B (2015) Molecular mechanism of PPARalpha action and its impact on lipid metabolism, inflammation and fibrosis in non-alcoholic fatty liver disease. J Hepatol 62:720-733
Pedron J, Boudot C, Hutter S, Bourgeade-Delmas S, Stigliani J-L, Sournia-Saquet A, Moreau A, Boutet-Robinet E, Paloque L, Mothes E, Laget M, Vendier L, Pratviel G, Wyllie S, Fairlamb A, Azas N, Courtioux B, Valentin A and Verhaeghe P (2018) Novel 8-nitroquinolin-2(1H)-ones as NTR-bioactivated antikinetoplastid molecules: Synthesis, electrochemical and SAR study. European journal of medicinal chemistry 155:135-152.
Sumida Y, Yoneda M, Ogawa Y, Yoneda M, Okanoue T and Nakajima A (2020) Current and new pharmacotherapy options for non-alcoholic steatohepatitis. Expert Opin Pharmacother 21:953-967.
Todisco S, Santarsiero A, Convertini P, De Stefano G, Gilio M, Iacobazzi V and Infantino V (2022) PPAR Alpha as a Metabolic Modulator of the Liver: Role in the Pathogenesis of Nonalcoholic Steatohepatitis (NASH). Biology (Basel) 11.

## Claims

1. A compound of general formula (I) 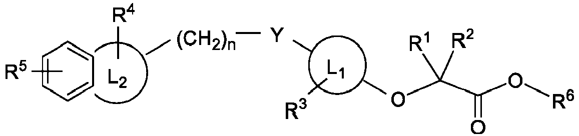 wherein
L₁ is either aryl or 6-membered heteroaryl, which optionally further includes a heteroatom selected from N, O, and S;
L₂ is aryl, 5 or 6-membered heteroaryl containing 1 or 2 heteroatoms selected from N, O and S; or C5-C6 cycloalkyl;
R¹ is H or independently selected from the group consisting of C1-C2 alkyl, C1-C2 haloalkyl, wherein each of these substituents may optionally be further substituted by one or more substituents selected from the group consisting of halogen, pseudohalogen, hydroxy, amino, or alkyloxy moieties;
R² is H or independently selected from the group consisting of C1-C4 alkyl, C1-C4 haloalkyl, wherein each of these substituents may optionally be further substituted by one or more substituents selected from the group consisting of halogen, pseudohalogen, hydroxy, amino, or alkyloxy moieties;
R³ is H or independently selected from the group consisting of halogen, pseudohalogen, C1-C2 alkyl, C1-C2 haloalkyl, hydroxy or amino moieties;
R⁴ is H or independently selected from the group consisting of halogen, pseudohalogen, C1-C2 alkyl, C1-C2 haloalkyl, methoxy C1-C4 alkyl, thio C1-C4 alkyl, halogen, pseudohalogen, wherein each of these substituents may optionally be further substituted by halogen, pseudohalogen, hydroxy, or amino moiety;
R⁵ is H or independently selected from the one or two groups consisting of C1-C4 alkyl, C1-C4 haloalkyl, methoxy C1-C4 alkyl, thio C1-C4 alkyl, halogen, CF3, pseudohalogen,
wherein each of these substituents may optionally be further substituted by halogen, pseudohalogen, hydroxy, nitro, or amino moiety;
R⁶ is H or independently selected from C1-C6 alkyl, C1-C4 haloalkyl, methoxy C1-C4 alkyl, thio C1-C4 alkyl, wherein each of these substituents may optionally be further substituted by halogen, pseudohalogen, hydroxy, nitro, or amino moiety;
Y is O, N, or S; and
n is 0, 1 or 2,
for use, as a compound dually acting on two nuclear receptors, in the prevention or treatment of disorders selected from hyperlipidemia, hypercholesterolemia, dyslipidemia, steatosis, steatohepatitis, cholestasis, non-alcoholic fatty liver disease, atherosclerosis, hepatectomy, aseptic inflammation, obesity and type 2 diabetes mellitus.

## Patentansprüche

1. Eine Verbindung der allgemeinen Formel (I) 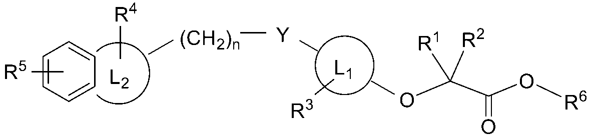 wobei
L ₁ ist entweder ein Arylrest oder ein sechsgliedriger Heteroarylrest, der optional zusätzlich ein Heteroatom aus N, O und S enthält;
L ₂ ist ein Aryl-, 5- oder 6-gliedriger Heteroaryl-Ring mit 1 oder 2 Heteroatomen, ausgewählt aus N, O und S; oder ein C5-C6- Cycloalkyl-Ring ;
R ¹ ist H oder unabhängig ausgewählt aus der Gruppe bestehend aus C1-C2-Alkyl, C1-C2 -Halogenalkyl , wobei jeder dieser Substituenten optional weiter durch einen oder mehrere Substituenten substituiert sein kann, die aus der Gruppe bestehend aus Halogen, Pseudohalogen , Hydroxy, Amino oder Alkyloxygruppen ausgewählt sind ;
R ² ist H oder unabhängig ausgewählt aus der Gruppe bestehend aus C1-C4-Alkyl, C1-C4 -Halogenalkyl , wobei jeder dieser Substituenten optional weiter durch einen oder mehrere Substituenten substituiert sein kann, die aus der Gruppe bestehend aus Halogen, Pseudohalogen , Hydroxy, Amino oder Alkyloxygruppen ausgewählt sind ;
R ³ ist H oder unabhängig ausgewählt aus der Gruppe bestehend aus Halogen, Pseudohalogen , C1-C2-Alkyl, C1 -C2- Halogenalkyl , Hydroxy oder Aminogruppen;
R ⁴ ist H oder unabhängig ausgewählt aus der Gruppe bestehend aus Halogen, Pseudohalogen , C1-C2-Alkyl, C1-C2- Halogenalkyl , Methoxy-C1-C4-Alkyl, Thio-C1-C4-Alkyl, Halogen, Pseudohalogen , wobei jeder dieser Substituenten optional weiter durch Halogen, Pseudohalogen , Hydroxy- oder Aminogruppe substituiert sein kann;
R ⁵ ist H oder unabhängig ausgewählt aus einer oder zwei Gruppen bestehend aus C1-C4-Alkyl, C1-C4- Halogenalkyl , Methoxy-C1-C4-Alkyl, Thio-C1-C4-Alkyl, Halogen, CF3, Pseudohalogen , wobei jeder dieser Substituenten optional weiter durch Halogen, Pseudohalogen , Hydroxy-, Nitro- oder Aminogruppe substituiert sein kann;
R ⁶ ist H oder unabhängig ausgewählt aus C1-C6-Alkyl, C1-C4- Halogenalkyl , Methoxy-C1-C4-Alkyl, Thio-C1-C4-Alkyl, wobei jeder dieser Substituenten optional weiter durch Halogen, Pseudohalogen , Hydroxy, Nitro oder Amino substituiert sein kann;
Y ist O, N oder S; und
n ist 0, 1 oder 2,
zur Verwendung als Verbindung, die dual auf zwei Kernrezeptoren wirkt, zur Vorbeugung oder Behandlung von Erkrankungen, die ausgewählt sind aus Hyperlipidämie, Hypercholesterinämie, Dyslipidämie, Steatose, Steatohepatitis, Cholestase, nichtalkoholischer Fettlebererkrankung, Arteriosklerose, Hepatektomie , aseptischer Entzündung, Fettleibigkeit und Typ-2-Diabetes mellitus.

## Revendications

1. Un composé de formule générale (I) 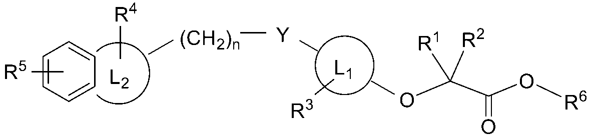 où
L ₁ est soit un aryle, soit un hétéroaryle à 6 chaînons, qui comprend éventuellement un hétéroatome choisi parmi N, O et S ;
L ₂ est un aryle, un hétéroaryle à 5 ou 6 chaînons contenant 1 ou 2 hétéroatomes choisis parmi N, O et S ; ou un cycloalkyle C5-C6 ;
R ¹ est H ou sélectionné indépendamment dans le groupe constitué de C1-C2 alkyle, C1-C2 haloalkyle , où chacun de ces substituants peut éventuellement être substitué par un ou plusieurs substituants sélectionnés dans le groupe constitué d'unités halogène, pseudohalogène , hydroxy, amino ou alkyloxy ;
R ² est H ou indépendamment sélectionné dans le groupe constitué de C1-C4 alkyle, C1-C4 haloalkyle , où chacun de ces substituants peut éventuellement être substitué par un ou plusieurs substituants sélectionnés dans le groupe constitué d'unités halogène, pseudohalogène , hydroxy, amino ou alkyloxy ;
R ³ est H ou sélectionné indépendamment dans le groupe constitué d'unités halogènes, pseudohalogènes , alkyles C1-C2, haloalkyles C1 -C2 , hydroxy ou amino ;
R ⁴ est H ou sélectionné indépendamment dans le groupe constitué d'halogène, de pseudohalogène , d'alkyle C1-C2, d'haloalkyle C1-C2 , de méthoxy alkyle C1-C4, de thio alkyle C1-C4, d'halogène, de pseudohalogène , dans lequel chacun de ces substituants peut éventuellement être substitué par un halogène, un pseudohalogène , un groupe hydroxy ou amino ;
R ⁵ est H ou sélectionné indépendamment parmi les un ou deux groupes constitués de C1-C4 alkyle, C1-C4 haloalkyle , méthoxy C1-C4 alkyle, thio C1-C4 alkyle, halogène, CF3, pseudohalogène , où chacun de ces substituants peut éventuellement être substitué par un halogène, un pseudohalogène , un groupe hydroxy, nitro ou amino ;
R ⁶ est H ou sélectionné indépendamment parmi les alkyles C1-C6, les haloalkyles C1-C4 , les alkyles méthoxy C1-C4, les alkyles thio C1-C4, chacun de ces substituants pouvant éventuellement être substitué par un halogène, un pseudohalogène , un groupe hydroxy, nitro ou amino ;
Y est O, N ou S ; et
n vaut 0, 1 ou 2,
pour être utilisé, en tant que composé agissant doublement sur deux récepteurs nucléaires, dans la prévention ou le traitement de troubles sélectionnés parmi l'hyperlipidémie, l'hypercholestérolémie, la dyslipidémie, la stéatose, la stéatohépatite, la cholestase, la stéatose hépatique non alcoolique, l'athérosclérose, l'hépatectomie , l'inflammation aseptique, l'obésité et le diabète sucré de type 2.
